# EUROPÄISCHE PATENTSCHRIFT

(11) **EP 1 711 494 B1**
(45) Veröffentlichungstag und Bekanntmachung des Hinweises auf die Patenterteilung: **09.06.2010**
(21) Anmeldenummer: 05701163.7
(22) Anmeldetag: 25.01.2005
(51) Int. Cl.: C07D 413/10, C07D 413/14, C07D 417/14, C07D 417/10, C07D 491/113, A61K 31/4245, A61P 3/04, A61P 3/10

(54) **ARYLSUBSTITUIERTE HETEROZYKLEN, VERFAHREN ZU IHRER HERSTELLUNG UND IHRE VERWENDUNG ALS ARZNEIMITTEL**
ARYL SUBSTITUTED HETEROCYCLES, METHOD FOR PRODUCTION AND USE THEREOF AS MEDICAMENTS
HETEROCYCLES ARYL-SUBSTITUES, PROCEDE POUR LEUR PRODUCTION ET LEUR UTILISATION COMME MEDICAMENTS

(30) Priorität: 25.01.2004 DE 102004003812
(43) Veröffentlichungstag der Anmeldung: 18.10.2006
(73) Patentinhaber: Sanofi-Aventis Deutschland GmbH, 65929 Frankfurt am Main (DE)
(72) Erfinder: SCHWINK, Lothar, 35260 Stadtallendorf (DE); STENGELIN, Siegfried, 65817 Eppstein (DE); BOEHME, Thomas, 65428 Rüsselsheim (DE); GOSSEL, Matthias, 65428 Hofheim (DE); HESSLER, Gerhard, 65719 Hofheim (DE); LENNIG, Petra, 65926 Frankfurt am Main (DE)
(86) Internationale Anmeldenummer: PCT/EP2005/000699
(87) Internationale Veröffentlichungsnummer: WO 2005/070925

(56) Entgegenhaltungen:
- WO-A-00/66578
- WO-A-00/78758
- WO-A-01/82930
- WO-A-97/26258
- WO-A-02/079200
- WO-A-03/087044
- WO-A-03/089418
- WO-A-03/097047
- WO-A-2004/072025
- WO-A-2004/111045

## Beschreibung

Die Erfindung betrifft arylsubstituierte Hetreozyklen sowie deren physiologisch verträgliche Salze und physiologisch funktionelle Derivate.

Es sind bereits den hier beschriebenen Heterozyklen in ihrer Gesamtstruktur ähnliche Verbindungen mit pharmakologischer Wirkung im Stand der Technik beschrieben.
WO 2004/111045 betrifft Pyrrolidinone als Faktor Xa-Inhibitoren.
WO 03/089418 betrifft Indansäurederivate und deren Verwendung zur Behandlung der Obesitas und Diabetes.
WO 00/66578 betrifft Verbindungen für die Behandlung von Obesitas, die als Neuropeptid -Y-Antagonisten wirken.
WO 97/26258 betrifft 3-(3-substituierte-1, 2, 4-Thiadiazol-5-yl)pyrazinderivate, die als Angiogenese-Inhibitoren wirken.
WO 01/82930 betrifft Pyrazol- und Pyrazolonderivate, die als Inhibitoren für bakterielle RNA-Polymerase wirksam sind.
WO 00/78758 betrifft Imidazolderivate, die Inhibitoren gegen die Produktion von Interleukin-4, Interleukin-5 von Typ 2 Helfer-T-Zellen darstellen.
WO 02/079200 betrifft heterozyklylsubstituierte Propanhydroxaminsäuren als Procollagen C-Proteinase Inhibitoren.

Verbindungen mit MCH-antagonistischer Wirkung zur zur Behandlung der Obesitas sind im Stand der Technik beschrieben. Beispielsweise die WO2003097047 beschreibt arylsubstituierte Oxadiazole, WO2003087044 beschreibt arylsubstituierte Carboxyamide, WO2004072025 beschreibt N-arylsubstituierte Aminopyrrolidine. Weitere Beispiele von Verbindungen mit MCH-antagonistischer Wirkung sind in der WO2001021577, WO2003035624, WO2002089729, WO2002006245, WO2002002744, WO2002057233, WO2003045313 und WO2002010146 beschrieben.

Der Erfindung lag die Aufgabe zugrunde, Verbindungen zur Verfügung zu stellen, die eine Gewichtsreduktion bei Säugetieren bewirken und die zur Prävention und Behandlung von Obesitas und Diabetes geeignet sind.
Überrschenderweise wurde eine Serie von Verbindungen gefunden, die die Aktivität von MCH-Rezeptoren modulieren. Insbesondere zeichnen sich die Verbindungen durch einen Antagonismus des MCH1R aus.

Die Erfindung betrifft daher Verbindungen der Formel I, 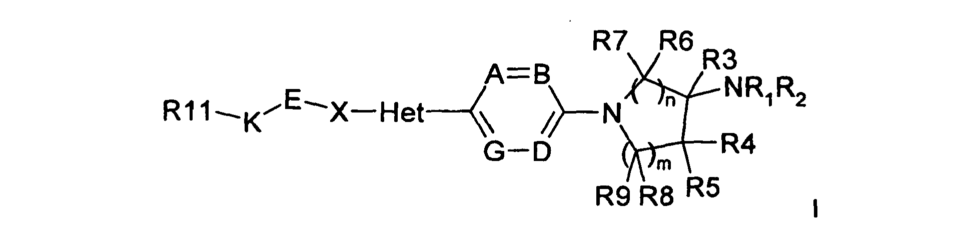 worin bedeuten
- R1, R2: unabhängig voneinander H, (C₁-C₈)-Alkyl, -(CR13R14)ₒ-R12, (C₁-C₄)- Alkoxy-(C₁-C₄)-alkyl, (C₃-C₈)-Alkenyl, (C₃-C₈)-Alkinyl, CO-(C₁-C₈)-Alkyl, -CO-(CH₂)ₒ -R12, CO(C(R15)(R16))_{q}N(R17)(R18), CO(C(R19)(R20))ₛO(R21); oder R1 und R2 bilden zusammen mit dem Stickstoffatom, an das sie gebunden sind, einen 4 bis 10-gliedrigen mono-, bi- oder spirocyclischen Ring welcher ausser dem Stickstoffatom 0 bis 4 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, Cl, Br, CF₃, NO₂, CN, (C₁-C₆)-Alkyl, O-(C₁-C₈)-Alkyl, (C₁-C₄)-Alkoxy-(C₁-C₄)-alkyl, Hydroxy-(C₁-C₄)-Alkyl, (C₀-C₈)-Alkylen-aryl, Oxo, CO(R22), CON(R23)(R24), Hydroxy, COO(R25), N(R26)CO(C₁-C₆)-Alkyl, N(R27)(R28) oder SO₂CH₃;
- o: 0, 1, 2, 3, 4, 5, 6;
- q, s: unabhängig voneinander 0, 1, 2, 3, 4;
- R15, R16, R17, R18, R19, R20, R21,:
- R22, R23, R24, R25, R26, R27, R28: unabhängig voneinander H, (C₁-C₆)-Alkyl;
- R17 und R18, R23 und R24, R27 und R28: unabhängig voneinander optional zusammen mit dem Stickstoffatom, an das sie gebunden sind, einen 5-6 gliedrigen Ring, welcher ausser dem Stickstoffatom noch 0-1 weitere Heteroatome aus der Gruppe NH, N-(C₁- C₆)-Alkyl, Sauerstoff und Schwefel beinhalten kann;
- R12: OH, O-(C₁-C₆)-Alkyl, CN, COO(R29), CON(R30)(R31), N(R32)(R33), 3- 12 gliedriger mono-, bi- oder spirocyclischer Ring, der ein oder mehrere Heteroatome aus der Gruppe N, O und S enthalten kann und der 3-12 gliedrige Ring weitere Substituenten wie F, Cl, Br, I, OH, CF₃, NO₂, CN, OCF₃, Oxo, O-(C₁-C₆)-Alkyl, (C₁-C₄)-Alkoxy-(C₁-C₄)-alkyl, S-(C₁-C6)- Alkyl, (C₁-C₆)-Alkyl, (C₂-C₆)-Alkenyl, (C₃-C₈)-Cycloalkyl, (C₂-C₆)-Alkinyl, O-(C₀-C₈)-Alkylen-aryl, (C₀-C₈)-Alkylen-aryl, N(R34)(R35), CO(C₁-C₆)-Alkyl, COO(R36) und S(O)ᵤ (R37) enthalten kann;
- u: 0, 1, 2;
- R34, R35: unabhängig voneinander H, (C₁-C₈)-Alkyl;
- R34 und R35: optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten und optional mit 1-2 Oxo-Gruppen substituiert sein kann;
- R36, R37: H, (C₁-C₈)-Alkyl;
- R13, R14: unabhängig voneinander H, (C₁-C₈)-Alkyl, Hydroxy-(C₁-C₄)-alkyl, OH, (C₁-C₄)-Alkoxy-(C₁-C₄)-alkyl;
- R29, R30, R31: unabhängig voneinander H, (C₁-C₈)-Alkyl, (C₂-C₆)-Alkenyl, (C₀-C₈)- Alkylen-aryl;
- R32, R33: unabhängig voneinander H, (C₁-C₆)-Alkyl
oder
- R32 und R33: optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten und optional mit 1-2 Oxo-Gruppen substituiert sein kann;
- R3: H, (C₁-C₆)-Alkyl;
- R4, R5: unabhängig voneinander H, (C₁-C₆)-Alkyl, OH, O-(C₁-C₆)-Alkyl, O-CO(C₁- C₆)-Alkyl, S-(C₁-C₆)-Alkyl;
- R6, R7, R8, R9: unabhängig voneinander H, (C₁-C₈)-Alkyl,
oder
- R6 und R7, R8 und R9: unabhängig voneinander optional Oxo;
- n, m: unabhängig voneinander 0, 1, 2;
- A, B, D, G: unabhängig voneinander N, C(R38) und die Gesamtzahl der Stickstoffatome in diesem Ring 0 oder 1 beträgt, wobei für den Fall, dass die Gesamtzahl der Stickstoffatome 1 ist, A oder B N bedeutet;
- R38: H, F, Cl, Br, I, OH, CF₃, NO₂, CN, OCF₃, O-(C₁-C₆)-Alkyl, O-(C₁-C₄)- Alkoxy-(C₁-C₄)-alkyl, S-(C₁-C₆)-Alkyl. (C₁-C₆)-Alkyl, (C₂-C₆)-Alkenyl, (C₃-C₈)-Cycloalkyl, O-(C₃-C₈)-Cycloalkyl, (C₃-C₈)-Cycloalkenyl, O-(C₃- C₈)-Cycloalkenyl, (C₂-C₆)-Alkinyl, (C₀-C₈)-Akylen-aryl, O-(C₀-C₈)- Alkylen-aryl, S-Aryl, N(R39)(R40), SO₂-CH₃, COOH, COO-(C₁-C₆)-Alkyl, CON(R41)(R42), N(R43)CO(R44), N(R45)SO₂(R46), CO(R47), - (CR48R49)ₓ-O(R50);
- R39, R40, R41, R42, R43, R45: unabhängig voneinander H, (C₁-C₈)-Alkyl;
oder
- R39 und R40, R41 und R42: unabhängig voneinander optional zusammen mit dem Stickstoffatom, an das sie gebunden sind, einen 5-6 gliedrigen Ring, welcher ausser dem Stickstoffatom noch 0-1 weitere Heteroatome aus der Gruppe NH, N-(C₁- C₆)-Alkyl, Sauerstoff und Schwefel beinhalten kann;
- R44, R46, R47: unabhängig voneinander H, (C₁-C₈)-Alkyl, Aryl;
- R48, R49: unabhängig voneinander H, (C₁-C₈)-Alkyl;
- R50: H, (C₁-C₆)-Alkyl;
- x: 1,2,3,4;
- Het: ausgewählt ist aus der Gruppe bestehend aus Oxadiazolen, Thiadiazolen, Triazolen, Thiophenen Furanen und Pyrrolen;
- X: eine Bindung, eine Gruppe der Formel -(CR51R52)_{y}-, worin eine oder mehrere Gruppen -(CR51R52)- durch Y ersetzt sein können, wobei sich ein chemisch sinnvoller Rest ergibt, C=C, C≡C;
- Y: O, S, N(R53), CO, SO, SO₂;
- R51, R52: unabhängig voneinander H, (C₁-C₄)-Alkyl, wobei R51 und R52 in den y Gruppen jeweils die gleichen oder verschiedene Bedeutungen aufweisen können;
- y: 1, 2, 3, 4, 5, 6;
- R53: H, (C₁-C₈)-Alkyl;
- E: 3-14 gliedrige bivalente carbo- oder heterocyclische Ringstruktur mit 0-4 Heteroatomen aus der Gruppe N, O und S, die optional Substituenten aus der Gruppe H, F, Cl, Br, I, OH, CF₃, NO₂, CN, OCF₃, Oxo, O-(C₁-C₆)- Alkyl, O-(C₁-C₄)-Alkoxy-(C₁-C₄)-alkyl, S-(C₁-C₆)-Akyl, (C₁-C₆)-Alkyl, (C₂-C₆)-Alkenyl, (C₃-C₈)-Cycloalkyl, O-(C₃-C₈)-Cycloalkyl, (C₂-C₆)- Alkinyl, (C₀-C₈)-Alkylen-aryl, O-(C₀-C₈)-Alkylen-aryl, S-Aryl, N(R54)(R55), SO₂-CH₃, COOH, COO-(C₁-C₆)-Alkyl, CON(R56)(R57), N(R58)CO(R59), N(R60)SO₂(R61), CO(R62) tragen und mono- oder bicyclisch sein kann;
- R54, R55, R56, R57, R58, R60: unabhängig voneinander H, (C₁-C₈)-Alkyl;
- R54 und R55, R56 und R57: unabhängig voneinander optional zusammen mit dem Stickstoffatom, an das sie gebunden sind, einen 5-6 gliedrigen Ring, welcher ausser dem Stickstoffatom noch 0-1 weitere Heteroatome aus der Gruppe NH, N-(C₁- C₆)-Alkyl, Sauerstoff und Schwefel beinhalten kann;
- R59, R61, R62: unabhängig voneinander H, (C₁-C₈)-Alkyl, Aryl;
- K: eine Bindung, eine Gruppe der Formel -(CR63R64)_{z}-, worin eine oder mehrere Gruppen -(CR63R64)- durch Z ersetzt sein können, wobei sich ein chemisch sinnvoller Rest ergibt, C≡C, C=C;
- Z: O, S, N(R65), CO, SO, SO₂;
- R63, R64: unabhängig voneinander H, (C₁-C₈)-Alkyl, Hydroxy, (C₁-C₆)-Alkoxy, wobei R63 und R64 in den z Gruppen jeweils die gleichen oder verschiedene Bedeutungen aufweisen können;
- z: 1, 2,3,4, 5, 6;
- R65: H, (C₁-C₈)-Alkyl;
- R11: H, (C₁-C₈)-Alkyl, (C₁-C₄)-Alkoxy-(C₁-C₄)-alkyl, (C₃-C₈)-Alkenyl, (C₃-C₈)- Alkinyl, ein 3 bis 10-gliedriger mono-, bi- oder spirocyclischer Ring, welcher 0 bis 4 Heteroatome beinhalten kann, ausgewählt aus der Gruppe Sauerstoff, Stickstoff und Schwefel, wobei das Ringsystem zusätzlich substituiert sein kann mit F, Cl, Br, CF₃, NO₂, CN, (C₁-C₆)-Alkyl, O-(C₁- C₈)-Alkyl, (C₁-C₄)-Alkoxy-(C₁-C₄)-alkyl, (C₀-C₈)-Alkylen-aryl, Oxo, CO(R66), CON(R67)(R68), Hydroxy, Hydroxy-(C₁-C₄)-alkyl, COO(R69), N(R70)CO(C₁-C₆)-Alkyl, N(R71)(R72) oder SO₂CH₃;
- R66, R67, R68, R69, R70, R71, R72: unabhängig voneinander H, (C₁-C₈)-Alkyl;
oder
- R67 und R68, R71 und R72: unabhängig voneinander optional zusammen mit dem Stickstoffatom, an das sie gebunden sind, einen 5-6 gliedrigen Ring, welcher ausser dem Stickstoffatom noch 0-1 weitere Heteroatome aus der Gruppe NH, N-(C₁- C₆)-Alkyl, Sauerstoff und Schwefel beinhalten kann; oder
deren N-Oxide sowie deren physiologisch verträgliche Salze.

Die Erfindung bezieht sich auf Verbindungen der Formel I, in Form ihrer Racemate, enantiomerenangereicherten Mischungen und reinen Enantiomere sowie auf ihre Diastereomere und Mischungen davon.

Die Alkyl-, Alkenyl- und Alkinylreste in den Substituenten R1, R2, R3, R4, R5, R6, R7, R8, R9, R10, R11, R12, R13, R14, R15, R16, R17, R18, R19, R20, R21, R22, R23, R24, R25, R26, R27, R28, R29, R30, R31, R32, R33, R34, R35, R36, R37, R38, R39, R40, R41, R42, R43, R44, R45, R46, R47, R48, R49, R50, R51, R52, R53, R54, R55, R56, R57, R58, R59, R60, R61, R62, R63, R64, R65, R66, R67, R68, R69, R70, R71, R72, R73, R74, R75, R76, R77, R78, R79, R80, R81, R82, R83 und R84 können sowohl geradkettig, verzweigt oder optional halogeniert sein.

Dies trifft auch zu, sofern die Akyl-, Alkenyl-und Alkinylreste Teil einer anderen Gruppe sind, zum Beispiel Teil einer Alkoxygruppe (wie (C₁-C₄)-Alkoxy-(C₁-C₄-alkyl)). Geeignete Halogene sind Fluor, Chlor, Brom und Iod, bevorzugt Fluor, Chlor und Brom, besonders bevorzugt Fluor.

Beispiele für Alkylgruppen sind: Methyl, Ethyl, Propyl, Butyl, Pentyl, Hexyl, Heptyl und Octyl. Dabei sind sowohl die n-Isomere dieser Reste als auch verzweigte Isomere wie Isopropyl, Isobutyl, Isopentyl, sec-Butyl, tert-Butyl, Neopentyl, 3,3-Dimethylbutyl usw. mit umfasst.

### Beispiele für

Cycloalkylreste sind: Cyclopropyl, Cyclobutyl, Cyclopentyl, Cyclohexyl, Cycloheptyl, Cyclooctyl, Cyclononyl und Cyclodecyl. Gegebenenfalls kann es sich auch um polycyclische Ringsysteme handeln, wie Decalinyl, Norbornanyl, Bomanyl oder Adamantanyl. Die Cycloalkylreste können unsubstituiert oder gegebenenfalls mit einem oder mehreren weiteren Resten substituiert sein.

Beispiele für Alkenyl- und Alkinylgruppen sind: Vinyl, 1-Propenyl, 2-Propenyl (Allyl), 2-Butenyl, 2-Methyl-2-propenyl, 3-Methyl-2-butenyl, Ethinyl, 1-Propinyl, 2-Propinyl (Propargyl), 1-Butinyl, 2-Butinyl oder 3-Butinyl.

Beispiele für Cycloalkenyl sind: Cyclopentenyl, Cyclohexenyl, Cycloheptenyl und Cyclooctenyl.

Die Alkenylreste und Cycloalkenylreste können ein bis drei konjugierte oder nicht konjugierte Doppelbindungen (also auch Alk-dienyl- sowie Alk-trienylreste), vorzugsweise eine Doppelbindung in einer geraden oder verzweigten Kette aufweisen. Für Alkinylreste gilt das gleiche für die Dreifachbindungen. Die Alkenyl- und Alkinylreste können unsubstituiert oder gegebenenfalls mit einem oder mehreren weiteren Resten substituiert sein.

Als Aryl werden in der vorliegenden Erfindung Reste bezeichnet, die von monocyclischen oder bicyclischen Aromaten abgeleitet sind, die keine Ringheteroatome enthalten. Sofern es sich nicht um monocyclische Systeme handelt, ist bei der Bezeichnung Aryl für den zweiten Ring auch die gesättigte Form (Perhydroform) oder die teilweise ungesättigte Form (beispielsweise die Dihydroform oder Tetrahydroform), sofern die jeweiligen Formen bekannt und stabil sind, möglich. Die Bezeichnung Aryl umfasst in der vorliegenden Erfindung beispielsweise auch bicyclische Reste, in denen sowohl beide Ringe aromatisch sind als auch bicyclische Reste, in denen nur ein Ring aromatisch ist. Beispiele für Aryl sind: Phenyl, Naphthyl, Indanyl, 1,2-Dihydronaphthenyl, 1,4-Dihydronaphthenyl, Indenyl oder 1,2,3,4-Tetrahydronaphthyl. Besonders bevorzugt ist Aryl Phenyl oder Naphthyl. Unter dem Begriff "Aryl" wird somit insbesondere eine Phenyl oder Naphthylgruppe verstanden.

Unter Heteroarylresten sind Reste zu verstehen, die von monocyclischen oder bicyclischen Aromaten abgeleitet sind, die Ringheteroatome, bevorzugt N, O oder S, enthalten.

Die Aryl- und Heteroarylreste können unsubstituiert oder mit einem oder mehreren weiteren Resten substituiert sein.

Die Verbindungen der Formel I können ein oder mehrere Asymmetriezentren enthalten. Daher können die Verbindungen der Formel I in Form ihrer Racemate, Enantiomeren angereicherten Mischungen, reinen Enantiomere, Diastereomere und Diastereomer-Mischungen vorliegen. Die vorliegende Erfindung umfasst alle diese isomeren Formen der Verbindungen der Formel I. Diese isomeren Formen können, wenn auch zum Teil nicht expressis verbis beschrieben, nach bekannnten Methoden erhalten werden.

Mono-, bi- oder spiro-cyclische Ringe können gesättigt, teilweise gesättigt oder ungesättigt und auch verbrückt sein.

Unter C=C ist eine Gruppe der Formel R'C=CR" zu verstehen, worin R' und R" unabhängig voneinander H, (C₁-C₈)-Alkyl, bevorzugt H, bedeuten.

In dem Fall, dass R1 und R2 zusammen mit dem Stickstoffatom an das sie gebunden sind, einen Ring bilden, kann dieser Ring mit einem oder mehreren der genannten Substituenten substituiert sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Trifluormethansulfon-, Bernstein-, p-Toluolsulfon-, Wein- und Trifluoressigsäure. Für medizinische Zwecke wird in besonders bevorzugter Weise das Chlorsalz verwendet. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze) und Salze von Trometamol (2-Amino-2-hydroxymethyl-1,3-propandiol), Diethanolamin, Lysin oder Ethylendiamin.

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z. B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel (I)" auf Verbindung(en) der Formel (I) wie vorstehend beschrieben, sowie ihre Salze, Solvate und physiologisch funktionelle Derivate wie hierin beschrieben.

Können Reste oder Substituenten mehrfach in den Verbindungen der Formel I auftreten, so können sie alle unabhängig voneinander die angegebenen Bedeutungen haben und gleich oder verschieden sein.

In einer bevorzugten Ausführungsform bedeuten in den Verbindungen der Formel I
- R1, R2: unabhängig voneinander H, (C₁-C₈)-Alkyl, -(CR13R14)ₒ-R12, (C₁-C₄)- Akoxy-(C₁-C₄)-alkyl, (C₃-C₈)-Alkenyl, (C₃-C₈)-Alkinyl, CO-(C₁-C₈)-Alkyl, -CO-(CH₂)ₒ -R12, CO(C(R15)(R16))_{q}N(R17)(R18), CO(C(R19)(R20))ₛO(R21); oder R1 und R2 bilden zusammen mit dem Stickstoffatom, an das sie gebunden sind, einen 4 bis 10-gliedrigen mono-, bi- oder spirocyclischen Ring welcher ausser dem Stickstoffatom 0 bis 4 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, Cl, Br, CF₃, NO₂, CN, (C₁-C₆)-Alkyl, O-(C₁-C₈)-Alkyl, (C₁-C₄)-Alkoxy-(C₁-C₄)-alkyl, Hydroxy-(C₁-C₄)-alkyl, (C₀-C₈)-Alkylen-aryl, Oxo, CO(R22), CON(R23)(R24), Hydroxy, COO(R25), N(R26)CO(C₁-C₆)-Alkyl oder N(R27)(R28);
- R12: OH, O-(C₁-C₆)-Alkyl, CN, COO(R29), CON(R30)(R31), N(R32)(R33), 3- 12 gliedriger mono-, bi- oder spirocyclischer Ring, der ein oder mehrere Heteroatome aus der Gruppe N, O und S enthalten kann und der 3-12 gliedrige Ring weitere Substituenten wie F, Cl, Br, J, OH, CF₃, NO₂, CN, OCF₃, Oxo, O-(C₁-C₆)-Alkyl, (C₁-C₄)-Alkoxy-(C₁-C₄)-alkyl, S-(C₁-C₆)- Alkyl, (C₁-C₆)-Alkyl, (C₂-C₆)-Alkenyl, (C₃-C₈)-Cycloalkyl, (C₂-C₆)- Alkinyl, O-(C₀-C₈)-Alkylen-aryl, (C₀-C₈)-Alkylen-aryl, N(R34)(R35), CO(C₁-C₆)-Alkyl und COO(R36) enthalten kann; und
- R6, R7, R8, R9: unabhängig voneinander H, (C₁-C₈)-Alkyl;
wobei die weiteren Reste und Gruppen in den Verbindungen der Formel I die vorstehend genannten Bedeutungen und nachstehend genannten bevorzugten Bedeutungen aufweisen.

In einer weiteren bevorzugten Ausführungsform betrifft die vorliegende Erfindung Verbindungen der Formel I,
worin bedeuten:
- R1, R2: unabhängig voneinander H, (C₁-C₈)-Alkyl, -(CR13R14)ₒ -R12, (C₁-C₄)-Alkoxy- (C₁-C₄)-alkyl, (C₃-C₈)-Alkenyl, CO-(C₁-C₈)-Alkyl, -CO-(CH₂)ₒ -R12, CO(C(R15)(R16))_{q}N(R17)(R18), CO(C(R19)(R20))_{S}O(R21); oder R1 und R2 bilden zusammen mit dem Stickstoffatom, an das sie gebunden sind, einen 4 bis 10-gliedrigen mono-, bi- oder spirocyclischen Ring welcher ausser dem Stickstoffatom 0 bis 2 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, Cl, CF₃, (C₁-C₆)-Alkyl, O-(C₁-C₄)-Alkyl, (C₁-C₄)-Alkoxy-(C₁-C₄)-alkyl, Hydroxy- (C₁-C₄)-alkyl, (C₀-C₂)-Alkylen-aryl, Oxo, CO(R22), CON(R23)(R24), Hydroxy, COO(R25), N(R26)CO(C₁-C₆)-Alkyl, N(R27)(R28) oder SO₂CH₃;
bevorzugt unabhängig voneinander H, (C₁-C₈)-Alkyl, -(CR13R14)ₒ-R12, (C₁-C₄)-Alkoxy-(C₁-C₄)-alkyl, CO-(C₁-C₈)-Alkyl, -CO- (CH₂)ₒ -R12, CO(C(R15)(R16))_{q}N(R17)(R18), CO(C(R19)(R20))ₛO(R21); oder R1 und R2 bilden zusammen mit dem Stickstoffatom, an das sie gebunden sind, einen 4 bis 10-gliedrigen mono- oder bicyclischen Ring welcher ausser dem Stickstoffatom 0 bis 2 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, Cl, CF₃, (C₁-C₆)-Alkyl, O-(C₁-C₄)-Alkyl, (C₁-C₄)-Alkoxy-(C₁- C₄)-alkyl, (C₀-C₂)-Alkylen-aryl, Oxo, CO(R22), Hydroxy, N(R27)(R28) oder SO₂CH₃;
besonders bevorzugt unabhängig voneinander H, (C₁-C₈)-Alkyl, - (CR13R14)ₒ -R12, (C₁-C₄)-Alkoxy-(C₁-C₄)-alkyl, CO-(C₁-C₈)-Alkyl, -CO- (CH₂)ₒ -R12, CO(C(R15)(R16))_{q}N(R17)(R18), oder R1 und R2 bilden zusammen mit dem Stickstoffatom, an das sie gebunden sind, einen 4 bis 10-gliedrigen mono- oder bicyclischen Ring welcher ausser dem Stickstoffatom 0 bis 2 zusätzliche Heteroatome beinhalten kann, ausgewählt aus der Gruppe Sauerstoff und Stickstoff, wobei das heterocyclische Ringsystem zusätzlich substituiert sein kann mit F, (C₁-C₆)-Alkyl, (C₁-C₄)- Alkoxy-(C₁-C₄)-alkyl, Oxo, CO(R22), Hydroxy, N(R27)(R28);
- o: 0, 1, 2, 3, 4, 5, 6; bevorzugt 0, 1, 2, 3, 4; besonders bevorzugt 0, 1, 2, 3;
- q: 1, 2, 3; bevorzugt 1 oder 2;
- s: 0, 1, 2, 3, 4; bevorzugt 0, 1, 2, 3; besonders bevorzugt 0, 1, 2;
- R15, R16, R17, R18, R19, R20, R21,:
- R22, R23, R24, R25, R26, R27, R28: unabhängig voneinander H, (C₁-C₆)-Alkyl;
oder
- R17 und R18, R23 und R24, R27 und R28: unabhängig voneinander optional zusammen mit dem Stickstoffatom, an das sie gebunden sind, einen 5-6 gliedrigen Ring, welcher ausser dem Stickstoffatom noch 0-1 weitere Heteroatome aus der Gruppe NH, N-(C₁- C₆)-Alkyl, Sauerstoff und Schwefel beinhalten kann; bevorzugt ist der Ring Pyrrolidin, Piperidin, N-Methylpiperazin, Morpholin;
- R12: OH, O-(C₁-C₆)-Alkyl, CN, COO(R29), CON(R30)(R31), 3-12 gliedriger mono-, bi- oder spirocyclischer Ring, der ein oder mehrere Heteroatome aus der Gruppe N, O und S enthalten kann und der 3-12 gliedrige Ring weitere Substituenten wie F, Cl, Br, OH, CF₃, CN, Oxo, O-(C₁-C₆)-Alkyl, (C₁-C₄)- Alkoxy-(C₁-C₄)-alkyl, (C₁-C₆)-Alkyl, O-(C₀-C₈)-Alkylen-Aryl, (C₀-C₈)- Alkylen-aryl, N(R34)(R35), CO(C₁-C₆)-Alkyl, COO(R36), S(O)ᵤ(R37) enthalten kann;
bevorzugt OH, O-(C₁-C₆)-Alkyl, CN, 3-10 gliedriger mono-, oder bicyclischer Ring, der 1-3 Heteroatome aus der Gruppe N, O und S enthalten kann und der 3-10 gliedrige Ring weitere Substituenten wie F, Cl, Br, OH, CF₃, CN, Oxo, O-(C₁-C₆)-Alkyl, (C₁-C₄)-Alkoxy-(C₁-C₄)-alkyl, (C₁-C₆)-Alkyl, (C₀-C₂)-Alkylen-aryl, N(R34)(R35), CO(C₁-C₆)-Alkyl enthalten kann;
besonders bevorzugt OH, O-(C₁-C₆)-Alkyl, 3-10 gliedriger mono-, oder bicyclischer Ring, der 1-2 Heteroatome aus der Gruppe N, O und S enthalten kann und der 3-10 gliedrige Ring weitere Substituenten wie F, OH, Oxo, (C₁-C₆)-Akyl, CO(C₁-C₆)-Alkyl enthalten kann;
- u: 0, 1, 2; bevorzugt 0 oder 2; besonders bevorzugt 2;
- R34, R35: unabhängig voneinander H, (C₁-C₈)-Akyl;
oder
- R34 und R35: optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten und optional mit 1-2 Oxo-Gruppen substituiert sein kann;
- R36, R37: H, (C₁-C₈)-Alkyl;
- R13, R14: unabhängig voneinander H, (C₁-C₈)-Alkyl, Hydroxy-(C₁-C₄)-Alkyl, OH, (C₁-C₄)-Alkoxy-(C₁-C₄)-alkyl;
- R29, R30, R31: unabhängig voneinander H, (C₁-C₈)-Alkyl;
- R3: H, (C₁-C₆)-Alkyl; bevorzugt H;
- R4, R5: unabhängig voneinander H, (C₁-C₆)-Alkyl, OH, O-(C₁-C₆)-Alkyl, O-CO(C₁- C₆)-Alkyl, S-(C₁-C₆)-Alkyl; bevorzugt unabhängig voneinander H, (C₁-C₆)- Alkyl, OH, O-(C₁-C₆)-Alkyl, O-CO(C₁-C₆)-Alkyl; besonders bevorzugt unabhängig voneinander H, OH, O-(C₁-C₆)-Alkyl; ganz besonders bevorzugt H;
- R6, R7, R8, R9: H;
oder
- R6 und R7, R8 und R9: unabhängig voneinander optional Oxo;
bevorzugt sind R6, R7, R8, R9 H;
- n: 1
- m: 1 oder 2; bevorzugt 1;
- A, B, D, G: unabhängig voneinander N, C(R38) und die Gesamtzahl der Stickstoffatome in diesem Ring 0 oder 1 beträgt, wobei für den Fall, dass die Gesamtzahl der Stickstoffatome 1 ist, A oder B N bedeutet;
oder
die Gruppen A und B oder D und G sind jeweils C(R38) und bilden gemeinsam eine ortho-Phenyleneinheit, so dass sich insgesamt ein 1,4- bisubstituiertes Naphthalinsystem ergibt;
insbesondere bevorzugt sind
A, B, D, G C(R38);
- R38: H, F, Cl, Br, CF₃, CN, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₀-C₈)-Alkylen-Aryl, O-(C₀-C₈)-Alkylen- Aryl, N(R39)(R40), SO₂-CH₃, CON(R41)(R42), N(R43)CO(R44), CO(R47), -(CR48R49)ₓ-O(R50);
bevorzugt H, F, Cl, Br, CF₃, CN, O-(C₁-C₆)-Alkyl, (C₁-C₆)-Alkyl, SO₂- CH₃, CON(R41)(R42), N(R43)CO(R44), CO(R47), -(CR48R49)ₓ-O(R50);
besonders bevorzugt H, F, Cl, CF₃, CN, (C₁-C₆)-Alkyl, -(CR48R49)ₓ-O(R50);
- R39, R40, R41, R42, R43: unabhängig voneinander H, (C₁-C₈)-Alkyl;
oder
- R39 und R40, R41 und R42: unabhängig voneinander optional zusammen mit dem Stickstoffatom, an das sie gebunden sind, einen 5-6 gliedrigen Ring, welcher ausser dem Stickstoffatom noch 0-1 weitere Heteroatome aus der Gruppe NH, N-(C₁- C₆)-Alkyl, Sauerstoff und Schwefel beinhalten kann;
- R44, R47: unabhängig voneinander H, (C₁-C₈)-Alkyl, Aryl; bevorzugt unabhängig voneinander H, (C₁-C₈)-Alkyl;
- R48, R49: H;
- R50: H, (C₁-C₆)-Alkyl;
- x: 1, 2; bevorzugt 1;
- Het: ausgewählt aus der Gruppe bestehend aus Oxadiazolen, Thiadiazolen, Triazolen, Thiophenen, Furanen und Pyrrolen; ganz besonders bevorzugt ist Het ausgewählt aus Oxadiazolen, Thiadiazolen;
- X: eine Bindung, eine Gruppe der Formel -(CR51R52)_{y}-, worin eine oder mehrere Gruppen -(CR51R52)- durch Y ersetzt sein können, wobei sich ein chemisch sinnvoller Rest ergibt, C=C, C≡C; bevorzugt
eine Bindung, CH₂-CH₂, CH₂Y, YCH₂, (R75)YCH₂, CH₂-NCO(R75), CH₂CON(R75); C(R76)(R77), C(R78)(R79)O, N(R75), C=C, C≡C; besonders bevorzugt eine Bindung, CH₂-CH₂, C(R76)(R77), N(R75), CH₂Y, CH₂Y(R75), CH₂-NCO(R75), CH₂CON(R75); C=C; ganz besonders bevorzugt eine Bindung, CH₂-CH₂, C(R76)(R77), C=C, (R75)YCH₂, CH₂- NCO(R75);
- Y: O, S, N(R53), CO; bevorzugt O, S, N(R53);
- R53: H, (C₁-C₈)-Alkyl;
- R75, R76, R77, R78, R79: unabhängig voneinander H, (C₁-C₈)-Alkyl;
- E: 3-8 gliedrige bivalente carbo- oder heterocyclische Ringstruktur mit 0-4 Heteroatomen aus der Gruppe N, O und S, die optional Substituenten aus der Gruppe H, F, Cl, Br, OH, CF₃, NO₂, CN, OCF₃, O-(C₁-C₆)-Alkyl, O- (C₁-C₄)-Alkoxy-(C₁-C₄)-alkyl, S-(C₁-C₆)-Alkyl, (C₁-C₆)-Alkyl, (C₂-C₆)- Alkenyl, O-(C₃-C₈)-Cycloalkyl, (C₂-C₆)-Alkinyl, (C₀-C₈)-Alkylen-aryl, O- (C₀-C₈)-Alkylen-aryl, S-Aryl, N(R54)(R55), SO₂-CH₃, N(R58)CO(R59), N(R60)SO₂(R61), CO(R62) tragen und mono- oder bicyclisch sein kann;
bevorzugt 5-7 gliedrige bivalente carbo- oder heterocyclische Ringstruktur mit 0-3 Heteroatomen aus der Gruppe N, O und S, die optional Substituenten aus der Gruppe H, F, Cl, Br, OH, CF₃, NO₂, CN, OCF₃, O- (C₁-C₆)-Alkyl, S-(C₁-C₆)-Alkyl, (C₁-C₆)-Alkyl, (C₂-C₆)-Alkenyl, O-(C₀-C₈)- Alkylen-Aryl, S-Aryl, N(R54)(R55), SO₂-CH₃, N(R58)CO(R59), CO(R62) tragen und mono- oder bicyclisch sein kann;
besonders bevorzugt 5-7 gliedrige bivalente carbo- oder heterocyclische Ringstruktur mit 0-2 Heteroatomen aus der Gruppe N, O und S, die optional Substituenten aus der Gruppe H, F, Cl, Br, OH, CF₃, NO₂, OCF₃, O-(C₁-C₆)- Alkyl, (C₁-C₆)-Alkyl, (C₂-C₆)-Alkenyl, N(R54)(R55), SO₂-CH₃, CO(R62) tragen kann, und die ganz besonders bevorzugt monocyclisch ist;
z.B. bedeutet E Benzol, Pyridin, Pyrimidin, Piperidin, Pyrrolidin, Cyclopentan, Cyclohexan, Piperazin, Homopiperazin, Thiazol, Thiophen, Furan, Pyrrol, Pyrazol, 1,2,3,6-Tetrahydro-pyridin, 4,5-Dihydroisoxazol, Oxazol;
- R54, R55, R58, R60: unabhängig voneinander H, (C₁-C₈)-Alkyl;
- R59, R61, R62: unabhängig voneinander H, (C₁-C₈)-Alkyl, Aryl; bevorzugt unabhängig voneinander H, (C₁-C₈)-Alkyl;
- K: O, eine Bindung, CH₂O, OCH₂, S, SO, SO₂, N(R80), N(R81)CO, CON(R82), (C(R83)(R84))ᵥ, CO, C=C, C≡C, SCH₂, SO₂CH₂;
bevorzugt O, eine Bindung, CH₂O, OCH₂, N(R80), N(R81)CO, CON(R82), (C(R83)(R84))ᵥ, CO, C≡C, SCH₂; besonders bevorzugt O, eine Bindung, CH₂O, OCH₂, CON(R82), (C(R83)(R84))ᵥ, CO, C≡C;
- v: 1, 2, 3, 4; bevorzugt 1, 2, 3; besonders bevorzugt 1,2;
- R80, R81, R82, R83, R84: unabhängig voneinander H, (C₁-C₈)-Alkyl;
- R11: H, (C₁-C₈)-Alkyl, (C₁-C₄)-Alkoxy-(C₁-C₄)-alkyl, (C₃-C₈)-Alkenyl, (C₃-C₈)- Alkinyl, ein 3 bis 10-gliedriger mono-, bi- oder spirocyclischer Ring, welcher 0 bis 4 Heteroatome beinhalten kann, ausgewählt aus der Gruppe Sauerstoff, Stickstoff und Schwefel, wobei das Ringsystem zusätzlich substituiert sein kann mit F, Cl, Br, CF₃, CN, (C₁-C₆)-Alkyl, O-(C₁-C₈)- Alkyl, (C₁-C₄)-Alkoxy-(C₁-C₄)-alkyl, Hydroxy-(C₁-C₄)-alkyl, (C₀-C₈)- Alkylen-aryl, Oxo, CO(R66), CON(R67)(R68), Hydroxy, COO(R69), N(R70)CO(C₁-C₆)-Alkyl, N(R71)(R72) oder SO₂CH₃;
bevorzugt (C₁-C₈)-Alkyl, (C₁-C₄)-Alkoxy-(C₁-C₄)-alkyl, ein 3 bis 10- gliedriger mono-, bi- oder spirocyclischer Ring, welcher 0 bis 3 Heteroatome beinhalten kann, ausgewählt aus der Gruppe Sauerstoff, Stickstoff und Schwefel, wobei das Ringsystem zusätzlich substituiert sein kann mit F, Cl, Br, CF₃, CN, (C₁-C₆)-Alkyl, O-(C₁-C₈)-Alkyl, (C₀-C₂)-Alkylen-aryl, Oxo, CO(R66), CON(R67)(R68), Hydroxy, N(R70)CO(C₁- C₆)-Alkyl, N(R71)(R72) oder SO₂CH₃; besonders bevorzugt (C₁-C₈)-Alkyl, (C₁-C₄)-Alkoxy-(C₁-C₄)-alkyl, ein 3 bis 10-gliedriger mono-oder bicyclischer Ring, welcher 0 bis 2 Heteroatome beinhalten kann, ausgewählt aus der Gruppe Sauerstoff, Stickstoff und Schwefel, wobei das Ringsystem zusätzlich substituiert sein kann mit F, Cl, Br, CF₃, CN, (C₁-C₆)-Alkyl, O-(C₁-C₈)-Alkyl, Oxo, CO(R66), CON(R67)(R68), N(R70)CO(C₁-C₆)-Alkyl, oder SO₂CH₃;
- R66, R67, R68, R69, R70, R71, R72: unabhängig voneinander H, (C₁-C₈)-Alkyl;
oder
- R67 und R68, R71 und R72: unabhängig voneinander optional zusammen mit dem Stickstoffatom, an das sie gebunden sind, einen 5-6 gliedrigen Ring, welcher ausser dem Stickstoffatom noch 0-1 weitere Heteroatome aus der Gruppe NH, N-(C₁- C₆)-Alkyl, Sauerstoff und Schwefel beinhalten kann; oder
deren N-Oxide sowie deren physiologisch verträgliche Salze.

In einer bevorzugten Ausführungsform der Erfindung weisen die Reste R1, R2, R11, R38 und Gruppen X, E, K die folgenden Bedeutungen auf:
- R1, R2: unabhängig voneinander H, (C₁-C₈)-Alkyl, -(CR13R14)ₒ -R12, (C₁-C₄)- Alkoxy-(C₁-C₄)-alkyl, oder R1 und R2 bilden zusammen mit dem Stickstoffatom, an das sie gebunden sind, einen 4 bis 10-gliedrigen mono-, bi- oder spirocyclischen Ring welcher ausser dem Stickstoffatom 0 bis 2 zusätzliche Heteroatome beinhalten kann, ausgewählt aus der Gruppe Sauerstoff, Stickstoff und Schwefel, wobei das heterocyclische Ringsystem zusätzlich substituiert sein kann mit (C₁-C₆)-Alkyl, O-(C₁-C₄)-Alkyl, (C₁- C₄)-Akoxy-(C₁-C₄)-alkyl, Hydroxy-(C₁-C₄)-alkyl, (C₀-C₂)-Alkylen-aryl, Oxo, CO(R22), CON(R23)(R24), Hydroxy, N(R27)(R28) oder SO₂CH₃;
bevorzugt unabhängig voneinander H, (C₁-C₈)-Alkyl, -(CR13R14)ₒ -R12, (C₁-C₄)-Alkoxy-(C₁-C₄)-akyl, oder R1 und R2 bilden zusammen mit dem Stickstoffatom, an das sie gebunden sind, einen 4 bis 10-gliedrigen mono-, bi- oder spirocyclischen Ring welcher ausser dem Stickstoffatom 0 bis 2 zusätzliche Heteroatome beinhalten kann, ausgewählt aus der Gruppe Sauerstoff, Stickstoff und Schwefel, wobei das heterocyclische Ringsystem zusätzlich substituiert sein kann mit (C₁-C₆)-Alkyl, O-(C₁-C₄)-Alkyl, (C₁- C₄)-Alkoxy-(C₁-C₄)-alkyl, Hydroxy-(C₁-C₄)-alkyl, (C₀-C₂)-Alkylen-aryl, Oxo, CO(R22), CON(R23)(R24), Hydroxy oder N(R27)(R28);
ganz besonders bevorzugt unabhängig voneinander H, (C₁-C₈)-Alkyl, oder R1 und R2 bilden zusammen mit dem Stickstoffatom, an das sie gebunden sind, einen 5 bis 6-gliedrigen monocyclischen Ring welcher ausser dem Stickstoffatom 0 bis 1 zusätzliche Heteroatome beinhalten kann, ausgewählt aus der Gruppe Sauerstoff, Stickstoff und Schwefel, wobei das heterocyclische Ringsystem zusätzlich substituiert sein kann mit (C₁-C₆)- Alkyl;
- o: 0, 1, 2, 3, 4;
- R22, R23, R24, R27, R28: unabhängig voneinander H, (C₁-C₆)-Alkyl;
oder
- R23 und R24, R27 und R28: unabhängig voneinander optional zusammen mit dem Stickstoffatom, an das sie gebunden sind, einen 5-6 gliedrigen Ring; welcher ausser dem Stickstoffatom noch 0-1 weitere Heteroatome aus der Gruppe NH, N-(C₁- C₆)-Alkyl, Sauerstoff und Schwefel beinhalten kann;
- R12: OH, O-(C₁-C₆)-Alkyl, CN, 3-12 gliedriger mono-, bi- oder spirocyclischer Ring, der 1 bis 3 Heteroatome aus der Gruppe N, O und S enthalten kann und der 3-12 gliedrige Ring weitere Substituenten wie F, OH, CF₃, CN, Oxo, (C₁-C₆)-Alkyl, (C₀-C₂)-Alkylen-aryl, N(R34)(R35), COO(R36), CO(C₁-C₆)-Alkyl enthalten kann;
- R34, R35: unabhängig voneinander H, (C₁-C₄)-Alkyl;
- R36: H, (C₁-C₆)-Alkyl;
- R13, R14: unabhängig voneinander H, (C₁-C₈)-Alkyl, Hydroxy-(C₁-C₄)-Alkyl, OH, (C₁-C₄)-Alkoxy-(C₁-C₄)-alkyl;
- R38: H, F, Cl, Br, CF₃, CN, (C₁-C₆)-Alkyl;
- X: eine Bindung, CH₂CH₂, C(R76)(R77), N(R75), C=C, (R75)YCH₂, CH₂- NCO(R75), CH₂CON(R75);
- Y: O, S, N(R53), CO
- R75, R76, R77: unabhängig voneinander H, (C₁-C₈)-Alkyl;
- R53: H, (C₁-C₈)-Alkyl;
- E: 5-7 gliedrige bivalente carbo- oder heterocyclische Ringstruktur mit 0-3 Heteroatomen aus der Gruppe N, O und S, die optional Substituenten aus der Gruppe H, F, Cl, Br, CF₃, OH, CN, OCF₃, O-(C₁-C₆)-Alkyl, (C₁-C₆)- Alkyl, SO₂-CH₃, CO(R65) tragen kann;
- R65: H, (C₁-C₈)-Alkyl;
- K: O, eine Bindung, CH₂O, CH₂, OCH₂, S, SO₂, N(R80), N(R81)CO, CON(R82), (C(R83)(R84))ᵥ, CO, C≡C, SCH₂, SO₂CH₂; bevorzugt O, eine Bindung, CH₂O, CH₂, OCH₂, N(R80), C≡C;
- v: 1, 2, 3;
- R80, R81, R82, R83, R84: unabhängig voneinander H, (C₁-C₈)-Alkyl;
- R11: (C₁-C₈)-Alkyl, (C₁-C₄)-Alkoxy-(C₁-C₄)-alkyl, ein 3 bis 10-gliedriger mono-, bi- oder spirocyclischer Ring, welcher 0 bis 4 Heteroatome beinhalten kann, ausgewählt aus der Gruppe Sauerstoff, Stickstoff und Schwefel, wobei das Ringsystem zusätzlich substituiert sein kann mit F, Cl, Br, CF₃, CN, (C₁- C₆)-Alkyl, O-(C₁-C₈)-Alkyl, Oxo, CO(R66), Hydroxy, N(R70)CO(C₁-C₆)- Alkyl, oder SO₂CH₃;
- R66, R70: unabhängig voneinander H, (C₁-C₈)-Alkyl;
deren N-Oxide sowie deren physiologisch verträgliche Salze.

Besonders bevorzugt sind Verbindungen der Formel I, worin
- A, B, D, G: unabhängig voneinander N oder C(R38) bedeuten und die Gesamtzahl der Stickstoffatome in diesem Ring 0 oder 1 beträgt, wobei für den Fall, dass die Gesamtzahl der Stickstoffatome 1 ist, B N bedeutet.

Ganz besonders bevorzugt sind die Verbindungen der Formel I, worin
- n: 1 und
- m: 1 oder 2 bedeuten.

Insbesondere bevorzugt sind Verbindungen der Formel I, worin
- A, B, D, G: unabhängig voneinander N oder C(R38) bedeuten und die Gesamtzahl der Stickstoffatome in diesem Ring 0 oder 1 beträgt, wobei für den Fall, dass die Gesamtzahl der Stickstoffatome 1 ist, B N bedeutet;
- n: 1 und
- m: 1 oder 2 bedeuten.

Der Rest R38 hat die vorstehend genannten Bedeutungen.

Insbesondere bevorzugt sind Verbindungen der Formel I, worin
- Het: ausgewählt ist aus der Gruppe bestehend aus Oxadiazolen, Thiadiazolen, Triazolen, Thiophenen, Furanen und Pyrrolen; ganz besonders bevorzugt ist Het ausgewählt aus Oxadiazolen, Thiadiazolen, Thiazolen und Oxazolen.

Die weiteren Reste können in den vorstehend genannten bevorzugten Ausführungsformen die vorstehend genannten Bedeutungen aufweisen.

Die erfindungsgemäßen Verbindungen der Formel I und deren Vorstufen können nach dem Fachmann bekannten Verfahren hegestellt werden.

Im Folgenden ist die Herstellung von erfindungsgemäßen Verbindungen der Formel I am Beispiel der Herstellung von[1,3,4]-Oxadiazolen (Ia), [1,3,4]-Thiadiazolen (Ib), [1,2,4]-Oxadiazolen (Ic) dargestellt: 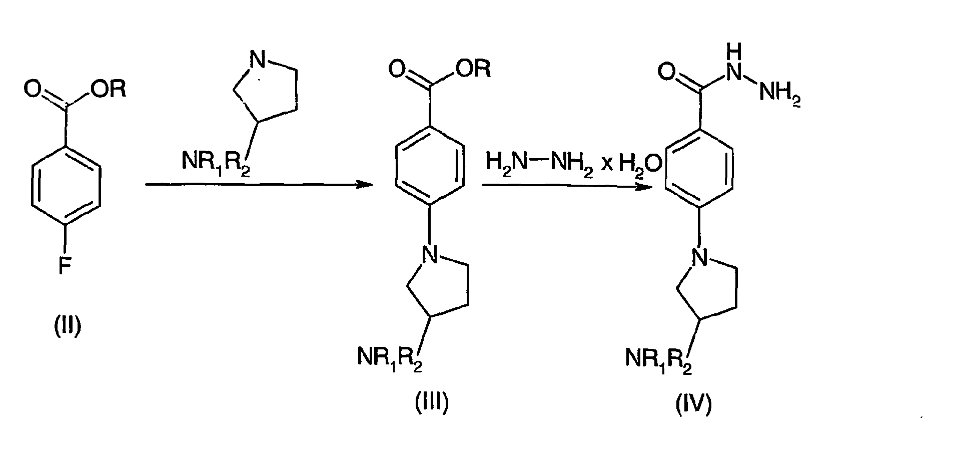 worin
- R: H, (C₁-C₈)-Alkyl bedeutet, und
- R1 und R2: die vorstehend genannten Bedeutungen aufweisen; 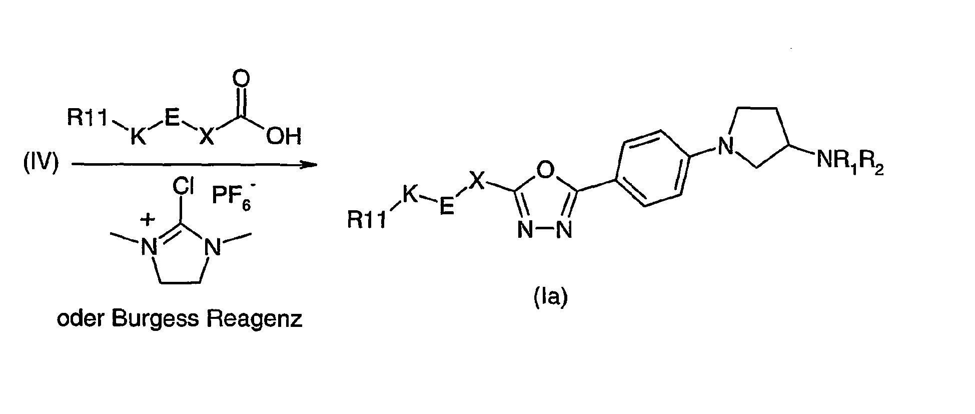 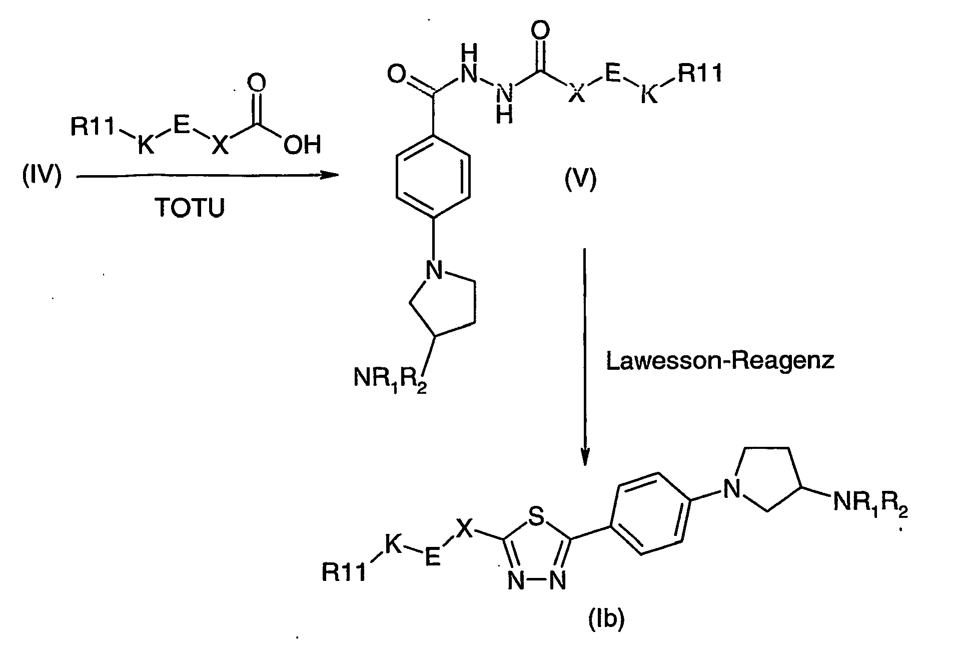
worin R1, R2, R11 und X, E und K die vorstehend genannten Bedeutungen aufweisen. 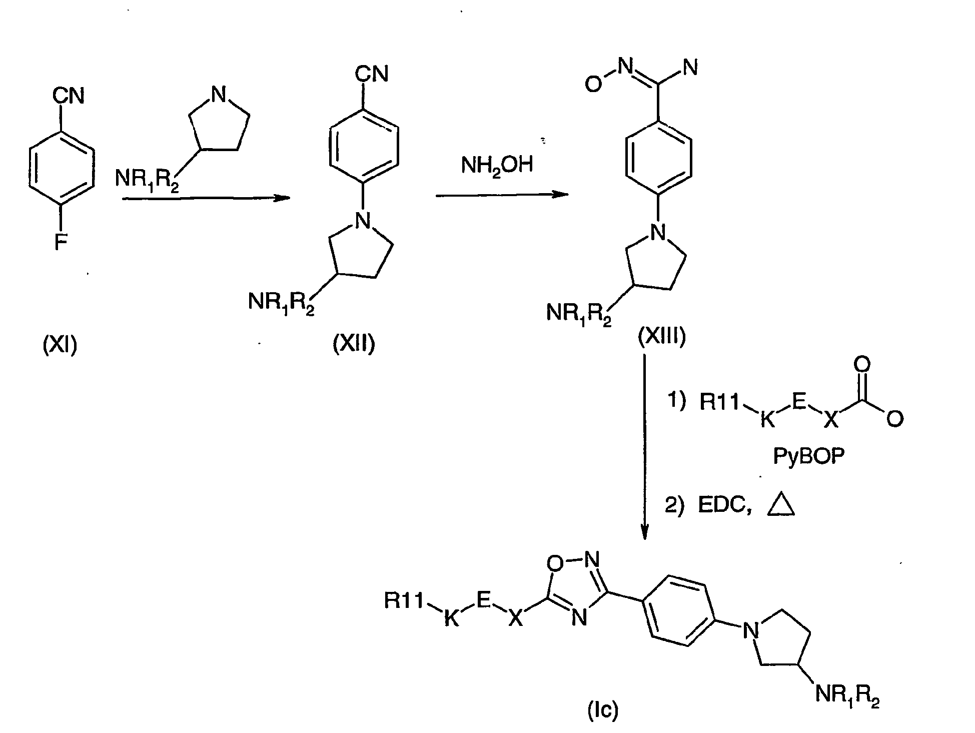 worin R1, R2, R11 und X, E und K die vorstehend genannten Bedeutungen aufweisen.

### Verwendung

Diese Erfindung bezieht sich weiterhin auf die Verwendung von Verbindungen der Formel I und ihren pharmazeutischen Zusammensetzungen als MCH-Rezeptor-Liganden. Die erfindungsgemäßen MCH-Rezeptor-Liganden eignen sich insbesondere als Modulatoren der Aktivität des MCH1R.

Die Rolle von MCH in der Regulation des Energiehaushalts ist inzwischen gut dokumentiert (Qu, D. et al.; Nature 1996, 380, 243-7; Shimada, M. et al. Nature 1998, 396, 670-4; Chen, Y. et al. Endocrinology 2002, 143, 2469-77; Endocrinology 2003, 144, 4831-40; Übersicht: G. Hervieu, Expert Opin. Ther. Targets 2003, 7, 495-511).

Auch gibt es Hinweise, dass MCH-Antagonisten zentral bedingte Störungen wie z.B. Depressionen günstig beeinflussen können (Borowsky, B. et al.; Nature Medicine 2002, 8, 825-30; Übersicht: G. Hervieu, Expert Opin. Ther. Targets 2003, 7, 495-511).

Besonders geeignet sind solche Verbindungen zur Behandlung und/oder Prävention von
1. Obesitas
2. Diabetes mellitus, insbesondere Typ 2 Diabetes einschließlich der Verhinderung der damit verbundenen Folgeerkrankungen. Besondere Aspekte sind dabei die
   - Hyperglykämie,
   - Verbesserung der Insulinresistenz,
   - Verbesserung der Glukose-Toleranz,
   - Schutz der β-Zellen der Bauchspeicheldrüse
   - Verhinderung makro- und mikrovaskulärer Erkrankungen
3. Dyslipidämien und deren Folgen, wie z.B. Atherosklerose, koronare Herzkrankheit, zerebrovaskuläre Erkrankungen etc, insbesondere solche (aber nicht beschränkt auf), die durch einen oder mehrerer folgender Faktoren charakterisiert sind:
   - hohe Plasma-Triglycerid-, hohe postprandiale Plasma-Triglycerid- Konzentrationen
   - niedrige HDL-Cholesterin Konzentration
4. Verschiedenen anderen Zuständen, die mit dem Metabolischen Syndrom assoziert sein können, wie:
   - Thrombosen, Stadien von Hyperkoagulabilität und Thromboseneigung (arteriell und venös)
   - Hoher Blutdruck
   - Herzinsuffizienz, wie z.B. (aber nicht beschränkt auf) bei Zustand nach Myokardinfarkt , hypertensive Herzerkrankung oder Kardiomyopathie
5. Psychiatrischen Indikationen wie
   - Depressionen
   - Angstzustände
   - Störungen des zirkadianen Rhythmus
   - Affektionsstörungen
   - Schizophrenie
   - Suchtkrankheiten

### Galenik

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,001 mg bis 100 mg, bevorzugt 0,3 mg bis 100 mg (typischerweise von 0,01 mg bis 50 mg, bevorzugt 3 mg bis 50 mg) pro Tag pro Kilogramm Körpergewicht, z.B. 0,1 -10 mg/kg/Tag, bevorzugt 3-10 mg/kg/Tag. Eine intravenöse Dosis kann z.B. im Bereich von 0,001 mg bis 1,0 mg/kg, bevorzugt 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 0,05 bis 1000 mg, typischerweise von 0,5 bis 600 mg enthalten, oder in einer weiteren Ausführungsform von 1,0 bis 1000 mg, typischerweise von 10 bis 600 mg. Im Falle pharmazeutisch verträglicher Salze beziehen sich die vorgenannten Gewichtsangaben auf das Gewicht der dem Salz zugrunde liegenden freien Verbindung. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Zubereitungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wässrigen oder nicht-wässrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mitteln in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel (I) mit einem oder mehreren herkömmlichen festen Trägem, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Die Verbindungen der Formel I zeichnen sich durch günstige Wirkungen auf den Fettstoffwechsel aus, insbesondere sind sie zur Gewichtsreduktion und nach erfolgter Gewichtsreduktion zum Erhalt eines reduzierten Gewichtes bei Säugetieren und als Anorektika geeignet. Die Verbindungen zeichnen sich durch ihre geringe Toxizität und ihre geringen Nebenwirkungen aus.

Die Verbindungen können allein oder in Kombination mit weiteren gewichtsreduzierenden oder anorektischen Wirkstoffen eingesetzt werden. Solche weiteren anorektischen Wirkstoffe werden z.B. in der Roten Liste, Kapitel 01 unter Abmagerungsmittel/Appetitzügler genannt und können auch solche Wirkstoffe beinhalten, die den Energieumsatz des Organismus erhöhen und damit zu einer Gewichtsreduktion führen oder auch solche, welche den allgemeinen Metabolismus des Organismus so beeinflussen, dass eine erhöhte Kalorienzufuhr nicht zu einer Vergrößerung der Fettdepots und eine normale Kalorienzufuhr zu einer Verringerung der Fettdepots des Organismus führt. Die Verbindungen eignen sich zur. Prophylaxe sowie insbesondere zur Behandlung von Übergewicht oder Obesitas. Die Verbindungen eignen sich weiterhin zur Prophylaxe sowie insbesondere zur Behandlung von Typ II Diabetes, der Arteriosklerose sowie zur Normalisierung des Lipidstoffwechsels und zur Behandlung des Bluthochdrucks.

### Kombinationen mit anderen Medikamenten

Bei einem weiteren Aspekt der Erfindung können die Verbindungen der Formel I in Kombination mit einer oder mehreren weiteren pharmakologisch wirksamen Substanzen verabreicht werden, die beispielsweise günstige Wirkungen auf Stoffwechselstörungen oder damit häufig assoziierte Erkrankungen haben. Solche Medikamente sind zum Beispiel
1. Blutzuckersenkende Medikamente, Antidiabetika,
2. Wirkstoffe zur Behandlung von Dyslipidemien,
3. Antiatherosklerotische Medikamente,
4. Antiadiposita,
5. Antiinflammatorische Wirkstoffe
6. Wirkstoffe zur Behandlung von malignen Tumoren
7. Antithrombotische Wirkstoffe
8. Wirkstoffe zur Behandlung von Bluthochdruck
9. Wirkstoffe zur Behandlung von Herzinsuffizienz sowie
10. Wirkstoffe zur Behandlung und/oder Prävention von Komplikationen, die von Diabetes verursacht werden oder mit Diabetes assoziiert sind.

Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen.

Als weitere pharmakologisch wirksame Substanzen sind insbesondere geeignet:

### Antidiabetika

Alle Antidiabetika, die in der Roten Liste 2001, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.

Geeignete Antidiabetika umfassen alle Insuline und Insulinderivate, wie z.B. Lantus® oder HMR 1964 oder Apidra^{®}, sowie andere schnell wirkende Insuline (siehe z.B. US 6 221 633), Amylin, GLP-1- und GLP-2-Derivate, wie in WO 01/04146 beschrieben oder auch wie z.B. diejenigen die in WO 98/08871 von Novo Nordisk A/S offenbart wurden, sowie oral wirksame hypoglykämische Wirkstoffe.

Die oral wirksamen hypoglykämischen Wirkstoffe umfassen vorzugsweise Sulphonylharnstoffe, Biguanidine, Meglitinide, Oxadiazolidindione, Thiazolidindione, Glukosidase-Inhibitoren, Glukagon-Rezeptor-Antagonisten, GLP-1-Agonisten, DPP-IV Inhibitoren, Kaliumkanalöffner wie z.B. diejenigen, die in WO 97/26265 und WO 99/03861 von Novo Nordisk A/S offenbart wurden, Insulin-Sensitizer, Aktivatoren der Insulin Rezeptor Kinase, Inhibitoren der GSK3-beta, Inhibitoren von Leberenzymen, die an der Stimulation der Glukoneogenese und/oder Glykogenolyse beteiligt sind, z.B. Inhibitoren der Glycogenphosphorylase, Modulatoren der Glukoseaufnahme und Glukoseausscheidung, den Fettstoffwechsel verändernde Verbindungen, die zur Veränderung der Lipidzusammensetzung des Blutes führen, wie antihyperlipidämische Wirkstoffe und antilipidämische Wirkstoffe, z.B. HMGCoA-Reduktase-Inhibitoren, Inhibitoren des Cholesteroltransports/der Cholesterolaufnahme, Inhibitoren der Gallensäurerückresorption oder Inhibitoren des mikrosomalen Triglycerid-Transfer Proteins (MTP), Verbindungen, die die Nahrungsmitteleinnahme und/oder Nahrungsmittelaufnahme verringern, PPAR- und RXR-Agonisten und Wirkstoffe, die auf den ATP-abhängigen Kaliumkanal der Betazellen wirken.

Bei einer Ausführungsform der Erfindung werden die vorliegenden Verbindungen in Kombination mit Insulin verabreicht.

Bei einer Ausführungsform der Erfindung werden die Verbindungen der Formel I in Kombination mit Substanzen, die Einfluss haben auf die hepatische Glukoseproduktion verabreicht, wie z.B. Glycogen Phosphorylase Inhibitoren (siehe: WO 01/94300, WO 02/096864, WO 03/084923, WO 03/084922, WO 03/104188)

Bei einer Ausführungsform werden die Verbindungen der Formel I in Kombination mit einem Sulphonylharnstoff, wie z.B. Tolbutamid, Glibenclamid, Glipizid oder Glimepirid verabreicht.

Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.

Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform werden die Verbindungen der Formel I verabreicht in Kombination mit einem DPPIV Inhibitor, wie z.B. in WO98/19998, WO99/61431, WO99/67278, WO99/67279, WO01/72290, WO 02/38541, WO03/040174 beschrieben, insbesondere P 93/01 (1-Cyclopentyl-3-methyl-1-oxo-2-pentanammonium chlorid), P-31/98, LAF237 (1-[2-[3-Hydroxyadamant-1-ylamino)acetyl]pyrrolidin-2-(S)-carbonitril), TS021 ((2S, 4S)-4-Fluoro-1-[[(2-hydroxy-1,1-dimethylethyl)amino]-acetyl]-pyrrolidin-2-carbonitril monobenzenesulfonat).

Bei einer Ausführungsform der Erfindung werden die Verbindungen der Formel I in Kombination mit einem PPAR gamma Agonisten, wie z.B. Rosiglitazon oder Pioglitazon, verabreicht.

Bei einer Aufführungsform werden die Verbindungen der Formel I in Kombination mit Verbindungen mit inhibitorischer Wirkung auf SGLT-1 und/oder 2, wie z.B. in PCT/EP03/06841, PCT/EP03/13454 und PCT/EP03/13455 direkt oder indirekt offenbart, verabreicht.

Bei einer Ausführungsform werden die Verbindungen der Formel I in Kombination mit einem α-Glukosidase-Inhibitor, wie z.B. Miglitol oder Acarbose, verabreicht.

Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylharnstoff und Acarbose, Repaglinid und Metformin, Insulin und einem Sulphonylharnstoff, Insulin und Metformin, Insulin und Troglitazon, Insulin und Lovastatin, etc. verabreicht.

### Lipidmodulatoren

Bei einer Ausführungsform der Erfindung werden die Verbindungen der Formel I in Kombination mit einem HMGCoA-Reduktase Inhibitor wie Lovastatin , Fluvastatin, Pravastatin, Simvastatin, Ivastatin, Itavastatin, Atorvastatin oder Rosuvastatin verabreicht.

Bei einer Ausführungsform der Erfindung werden die Verbindungen der Formel I in Kombination mit einem Gallensäureresorptionsinhibitor verabreicht (siehe z.B. US 6 245 744, US 6 221 897, US 6 277 831, EP 0 683 773, EP 0 683 774).

Bei einer Ausführungsform der Erfindung werden die Verbindungen der Formel I in Kombination mit einem polymeren Gallensäureadsorber, wie z.B. Cholestyramin oder Colesevelam, verabreicht.

Bei einer Ausführungsform der Erfindung werden die Verbindungen der Formel I in Kombination mit einem Cholesterinresorptionsinhibitor, wie z.B. in WO 0250027 beschrieben, oder Ezetimibe, Tiqueside, Pamaqueside verabreicht.

Bei einer Ausführungsform der Erfindung werden die Verbindungen der Formel I in Kombination mit einem LDL-Rezeptorinduktor (siehe z.B. US 6 342 512) verabreicht.

Bei einer Ausführungsform werden die Verbindungen der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6)); Caromax ist ein Carob enthaltendes Produkt der Fa. Nutrinova, Nutrition Specialties & Food Ingredients GmbH, Industriepark Höchst, 65926 Frankfurt / Main) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Bei einer Ausführungsform der Erfindung werden die Verbindungen der Formel I in Kombination mit einem PPAR alpha Agonisten verabreicht.

Bei einer Ausführungsform der Erfindung werden die Verbindungen der Formel I in Kombination mit einem Fibrat, wie z.B. Fenofibrat, Gemfibrozil, Clofibrat oder Bezafibrat, verabreicht.

Bei einer Ausführungsform der Erfindung werden die Verbindungen der Formel I in Kombination mit Nicotinsäure bzw. Niacin verabreicht.

Bei einer Ausführungsform der Erfindung werden die Verbindungen der Formel I in Kombination mit einem CETP-Inhibitor, z.B. CP- 529, 414 (Torcetrapib), verabreicht.

Bei einer Ausführungsform der Erfindung werden die Verbindungen der Formel I in Kombination mit einem ACAT-Inhibitor verabreicht

Bei einer Ausführungsform der Erfindung werden die Verbindungen der Formel I in Kombination mit einem MTP-Inhibitor, wie z.B. Implitapide, verabreicht.

Bei einer Ausführungsform der Erfindung werden die Verbindungen der Formel I in Kombination mit einem Antioxidanz verabreicht.

Bei einer Ausführungsform der Erfindung werden die Verbindungen der Formel I in Kombination mit einem Lipoprotein-Lipase Inhibitor, verabreicht.

Bei einer Ausführungsform der Erfindung werden die Verbindungen der Formel I in Kombination mit einem ATP-Citrat-Lyase Inhibitor verabreicht.

Bei einer Ausführungsform der Erfindung werden die Verbindungen der Formel I in Kombination mit einem Squalen Synthetase Inhibitor verabreicht.

Bei einer Ausführungsform der Erfindung werden die Verbindungen der Formel I in Kombination mit einem Lipoprotein(a) Antagonisten verabreicht.

### Antiobesita

Bei einer Ausführungsform der Erfindung werden die Verbindungen der Formel I in Kombination mit einem Lipase Inhibitor, wie z.B. Orlistat, verabreicht.

Bei einer Ausführungsform ist der weitere Wirkstoff Fenfluramin oder Dexfenfluramin.

Bei noch einer Ausführungsform ist der weitere Wirkstoff Sibutramin.

Bei einer anderen Ausführungsform ist der weitere Wirkstoff Rimonabant.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.:Hormone and Metabolic Research (2001), 33(9), 554-558), NPY-Antagonisten z.B. Naphthalin-1-sulfonsäure {4-[(4-amino-quinazolin-2-ylamino)-methyl]-cyclohexylmethyl}- amid; hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo- 2,3,3a,4,6,7-hexahydro-pyrazolo[4,3-c]pyridin-5-yl)-1-(4-chloro-phenyl)-2-oxo-ethyl]- amid; (WO 01/91752)) Orexin-Antagonisten (z.B. 1-(2-Methyl-benzoxazol-6-yl)-3-[1,5]naphthyridin-4-yl-harnstoff; hydrochloride (SB-334867-A)), CB1-Antagonisten /Inversen Agonisten, H3-Antagonisten / Inversen Agonisten (z.B. 3-Cyclohexyl-1-(4,4-dimethyl-1,4,6,7-tetrahydro-imidazo[4,5-c]pyridin-5-yl)-propan-1- on Oxalsäuresalz (WO 00/63208)); TNF-Agonisten, CRF-Antagonisten (z.B. [2-Methyl-9-(2,4,6-trimethylphenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2-(2,3-dimethyl-1H-indol-6-yloxy)- ethylamino]-ethanol; hydrochloride (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7- dimethyl-indol-1-yl}-acetic acid Trifluoressigsäuresalz (WO 99/15525)); Serotonin-Wiederaufnahme-Inhibitoren (z.B. Dexfenfluramine), gemischte Serotonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), BRS3-Agonisten, Galanin-Antagonisten, Ghrelin-Antagonisten, MCH-Antagonisten, mGluR5-Antagonisten, Opioid-Antagonisten, Wachstumshormon (z.B. humanes Wachstumshormon), Wachstumshormon freisetzende Verbindungen (6-Benzyloxy-1-(2-düsopropylamino-ethylcarbamoyl)-3,4-dihydro-1H-isoquinoline-2-carboxylic acid tert-butyl ester (WO 01/85695)), CNTF, CNTF-Derivaten (z.B. Axokine), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881),

DA-Agonisten (z.B. Bromocriptin, Doprexin), Lipase/Amylase-Inhibitoren (z.B. WO 00/40569), PPAR-Modulatoren (z.B. WO 00/78312), RXR-Modulatoren oder TR-β-Agonisten verabreicht.

Bei einer Ausführungsform der Erfindung ist der weitere Wirkstoff Leptin.

Bei einer Ausführungsform ist der weitere Wirkstoff Dexamphetamin, Amphetamin, Mazindol oder Phentermin.

Bei einer Ausführungsform werden die Verbindungen der Formel I in Kombination mit Medikamenten mit Wirkungen auf das Herz-Kreislauf- und das Blutgefäß-System, verabreicht, wie z.B. ACE-Hemmer (z.B. Ramipril), Medikamente, die auf das Angiotensin-Renin-System wirken, Calcium-Antagonisten, Beta-Blocker etc.

Bei einer Ausführungsform werden die Verbindungen der Formel I in Kombination mit anti-entzündlich wirkenden Medikamenten verabreicht.

Bei einer Ausführungsform werden die Verbindungen der Formel I in Kombination mit Medikamenten, die zur Krebstherapie und Krebsprävention eingesetzt werden, verabreicht.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

In zwei gleichzeitig erschienenen Artikeln in Nature (Nature 400, 261-264, 1999; Nature 400, 265-269, 1999) wurde separat von zwei Arbeitsgruppen ein hochspezifischer Rezeptor für das Melanin-Concentrating-Hormone (MCH) beschrieben. MCH übernimmt wichtige Funktionen bei der Steuerung der Nahrungsaufnahme. Verbindungen, die auf den MCH-Rezeptor wirken, besitzen daher eine anorektische Wirkung und sind zur Behandlung von Obesitas geeignet. Die Prüfung auf anorektische Wirkung der erfindungsgemäßen Verbindungen der Formel I wurde daher wie folgt durchgeführt.

### Funktionelle Messungen zur Ermittlung von IC50-Werten

Die Klonierung der cDNA für den humanen MCH-Rezeptor, Herstellung einer rekombinanten HEK293-Zellinie, welche den humanen MCH-Rezeptor exprimiert, sowie funktionelle Messungen mit der rekombinanten Zellinie erfolgten sinngemäß wie von Audinot et al. (J. Biol. Chem. 276, 13554-13562, 2001) beschrieben. Im Unterschied zur Literaturstelle wurde jedoch für die Konstruktion des Expressionsvektors das Plasmid pEAK8 der Fa. EDGE Biosystems (USA) verwendet. Als Wirt für die Transfektion diente eine transformierte HEK-Zellinie namens "PEAK Stable Cells" (ebenfalls von EDGE Biosystems). Die funktionellen Messungen des zellulären Calciumflusses nach Agonistenzugabe (MCH) in Gegenwart von erfindungsgemäßem Ligand erfolgten mit Hilfe des FLIPR-Gerätes der Fa. Molecular Devices (USA), unter Verwendung von Vorschriften des Geräteherstellers.

### Prüfung auf biologische Aktivität

Für die Beispielverbindungen 33, 96 und 97 wurden unter den vorstehend genannten Bedingungen IC50-Werte in der Größenordnung von 0,01 bis 10 µM gemessen.

### Allgemeine Erläuterungen

### a) Zeichenweise der Strukturformeln

In den Strukturformeln der gegebenen Beispiele sind zur Übersichtlichkeit nur nicht H-Atome dargestellt. Kohlenstoffatome werden nicht als "C" ausgeschrieben.

### b) Salzformen

Viele der erfindungsgemäßen Verbindungen sind Basen und können mit entsprechend starken Säuren Salze bilden. Insbesondere können die Verbindungen nach HPLCchromatograpischer Reinigung unter Verwendung eines Trifluoressigsäure enthaltenden Laufmittels als Hydrotrifluoracetate vorliegen. Diese können durch einfaches Behandeln einer Lösung der Salze z. B. mit Natriumcarbonatlösung in die gezeigten freien Basen überführt werden.

### c) Einheiten der Charakterisierungsdaten

Die Einheit der angegebenen Molekulargewichte ist "g/mol". Beobachtete Peaks im Massenspektrum sind angegeben als ganzzahliger Quotient der molaren Molekülionmasse und der Ladung des Molekülions (m/z).

### Abkürzungen

Wenn nicht anders angegeben, haben die Abkürzungen in den nachstehenden Beispielen folgende Bedeutung:
NaBH₃CN = Natriumcyanoborhydrid
DMF = N,N-Dimethylformamid
EDC = 1-(3-Dimethylaminopropyl)-3-ethylcarbodiimide
THF = Tetrahydrofuran
DMSO = Dimethylsulfoxid
HOBt = 1-Hydroxybenzotriazol
HOAt = 1-Hydroxy-7-azabenzotriazol
HCl = Salzsäure
HPLC = Hochleistungs-Flüssigkeitschromatographie
PyBOP = Benzotriazol-1-yloxytris(pyrrolidino)phosphonium hexafluorophospat
TOTU = O-[(Ethoxycarbonyl)cyanomethylenamino]-N,N,N',N'-tetramethyluronium tetrafluoroborate
eq = Äquivalente

### Beispiel 1

### (1-{4-[5-(4-Butoxy-phenyl)-[1,3,4]oxadiazol-2-yl]-phenyl}-pyrrolidin-3-yl)-dimethyl-amin

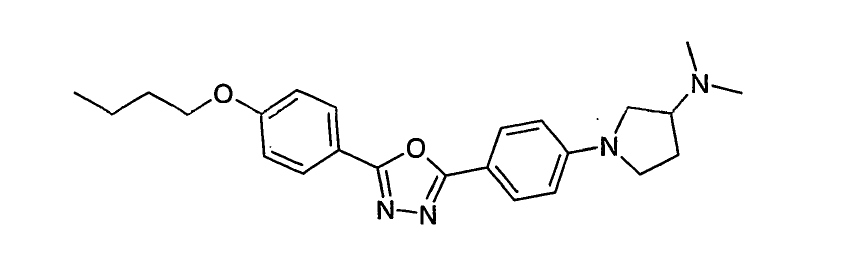

### Methode A

Eine Mischung aus 4-(3-Dimethylamino-pyrrolidin-1-yl)-benzoesäurehydrazid (100 mg), 4-Butoxybenzoesäure (78 mg), 2-Chlor-1,3-dimethyl-2-imidazoliumhexafluorphosphat (112 mg), N,N-Diisopropylethylamin (0,14 mL) und Dichlormethan (5 mL) wurde 12 Stunden gerührt. Flüchtige Anteile wurden entfernt und der Rückstand durch präparative HPLC gereingt. Man erhielt so das Produkt mit dem Molekulargewicht 406,53 (C24H30N402); MS (ESI): 407 (M+H+).

### 4-(3-Dimethylamino-pyrrolidin-1-yl)-benzoesäurehydrazid

Eine Mischung aus 4-(3-Dimethylamino-pyrrolidin-1-yl)-benzoesäureethylester (2,0 g), Hydrazin-Hydrat (3,8 g) und Ethanol (7 mL) wurde für 15 Stunden unter Rückfluss gekocht. Nach dem Abkühlen auf 5 °C wurde der entstandene Niederschlag abgesaugt. Man erhielt so das Produkt mit dem Molekulargewicht 248,33 (C13H20N40); MS (ESI): 249 (M+H+).

### 4-(3-Dimethylamino-pyrrolidin-1-yl)-benzoesäureethylester

Eine Mischung aus 4-Fluor-benzoesäureethylester (4,47 g), Dimethyl-pyrrolidin-3-yl-amin (3,04 g), Kaliumcarbonat (3,68 g) und Dimethylsulfoxid (20 mL) wurde für 7 Stunden auf 130 °C erwärmt. Nach dem Abkühlen wurde die Mischung mit Wasser verdünnt und mit Methyl tert.-butylether extrahiert. Die organische Phase wurde mit Wasser gewaschen, über Magnesiumsulfat getrocknet, filtriert und eingeengt. Man erhielt so das Produkt mit dem Molekulargewicht 262,35 (C15H22N202); MS (ESI): 263 (M+H+).

### Beispiel 2

### Dimethyl-(1-{4-[5-(2-phenoxy-ethylsulfanylmethyl)-[1,3,4]oxadiazol-2-yl]-phenyl}-pyrrolidin-3-yl)-amin

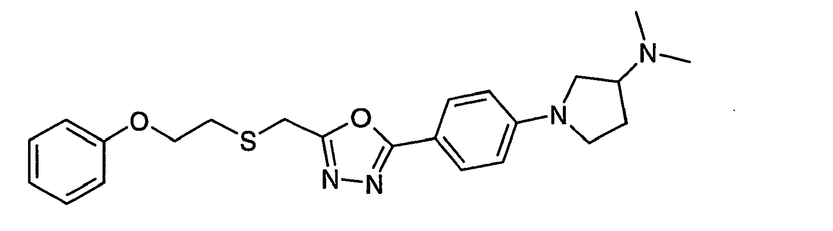

Nach Methode A wurde 4-(3-Dimethylamino-pyrrolidin-1-yl)-benzoesäurehydrazid mit (2-Phenoxy- ethylsulfanyl)-essigsäure umgesetzt. Man erhielt so das Produkt mit dem Molekulargewicht 424,57 (C23H28N4O2S); MS (ESI): 425 (M+H+).

### Beispiel 3

### Dimethyl-(1-14-[5-(4-phenoxy-phenyl)-[1,3,4]oxadiazol-2-yl]-phenyll-pyrrolidin-3-yl)-amine

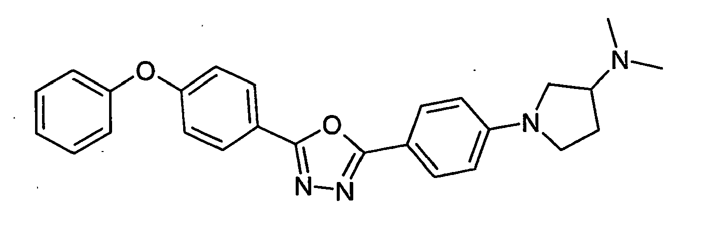

### Methode B

Eine Mischung aus 4-Phenoxy-benzoesäure-N'-[4-(3-dimethylamino-pyrrolidin-1-yl)-benzoyl]-hydrazid (178 mg), Methoxycarbonylsulfamoyl-triethylammmoniumhydroxid (Burgess-Reagenz) (96 mg) und 5 mL Toluol wurde 1 h bei 60 °C gerührt. Nach beendeter Reaktion wurde die Reaktionsmischung abfiltriert und das Filtrat zweimal mit Ethylacetat gewaschen. Anschließend wurden die vereinigten organischen Phasen mit 5%iger Natriumcarbonatlösung gewaschen und dann über Chromabond XTR getrocknet. Flüchtige Anteile wurden entfernt und der Rückstand durch präparative HPLC gereingt. Man erhielt so das Produkt mit dem Molekulargewicht 426,21 (C26H26N4O2); MS (ESI): 427,21 (M+H+).

### 4-Phenoxy-benzoesäure-N'-[4-(3-dimethylamino-pyrrolidin-1-yl)-benzoyl]-hydrazid

Eine Mischung aus 4-(3-Dimethylamino-pyrrolidin-1-yl)-benzoesäurehydrazid (100 mg), 4-Phenoxybenzoesäure (77,5 mg), 1-Propanphosphonsäureanhydrid 50% in Dichlormethan (256 mg), N,N-Diisopropylethylamin (0,208 mL) und DMF (3 mL) wurde 72 h bei Raumtemperatur gerührt. Anschließend wurde die Reaktionsmischung abfiltriert, zweimal mit Ethylacetat nachgewaschen und mit 0,5 N NaOH ausgeschüttelt. Flüchtige Anteile wurden entfernt und der Rückstand durch päparative HPLC gereinigt. Man erhielt so das Produkt mit dem Molekulargewicht 444,54; MS (ESI): 445,24 (M+H+).

4-(3-Dimethylamino-pyrrolidin-1-yl)-benzoesäurehydrazid wurde wie in Methode A beschrieben hergestellt, wobei im Cyclisierungsschritt nach 1 h erwärmen z.T. noch einmal 1 eq Burgess Reagenz zugegeben wurde und dann eine weitere Stunde auf 60 °C erwärmt wurde.

Die Verbindungen 4-93 in Tabelle 1 wurden nach Methode B synthetisiert.

| Bs p. | Struktur | Summenformel | MW Calc. | MW gemessen |
|---|---|---|---|---|
| 4 | 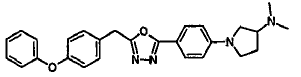 | C27H28N4O2 | 440,22 | 441,24 |
| 5 | 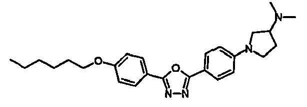 | C26H34N4O2 | 434,27 | 435,28 |
| 6 | 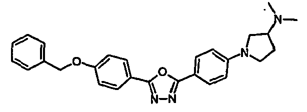 | C27H28N4O2 | 440,22 | 441,27 |
| 7 | 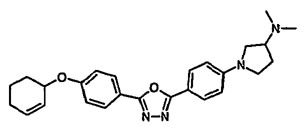 | C26H30N4O2 | 430,24 | 431,27 |
| 8 | 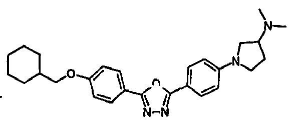 | C27H34N402 | 446,27 | 447,27 |
| 9 | 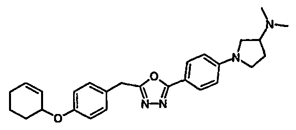 | C27H32N402 | 444,25 | 445,26 |
| 10 | 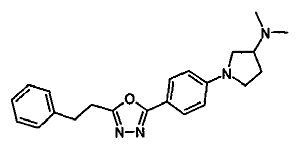 | C22H26N40 | 362,21 | 363,22 |
| 11 | 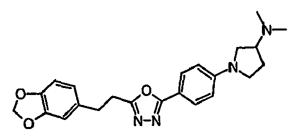 | C23H26N403 | 406,20 | 407,22 |
| 12 | 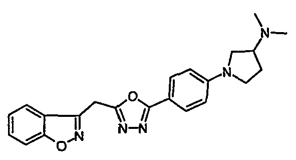 | C22H23N502 | 389,19 | 390,16 |
| 13 | 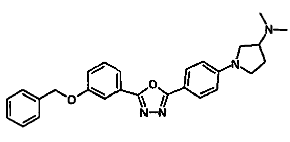 | C27H28N402 | 440,22 | 441,23 |
| 14 | 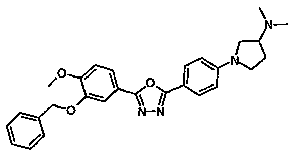 | C28H30N403 | 470,23 | 471,21 |
| 15 | 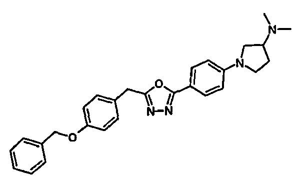 | C28H30N402 | 454,24 | 455,25 |
| 16 | 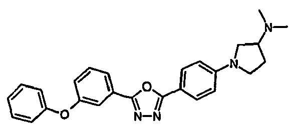 | C26H26N402 | 426,21 | 427,29 |
| 17 | 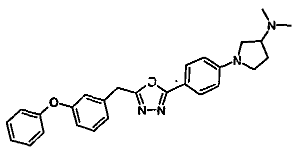 | C27H28N402 | 440,22 | 441,23 |
| 18 | 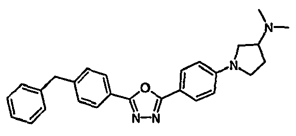 | C27H28N40 | 425,23 | 425,24 |
| 19 | 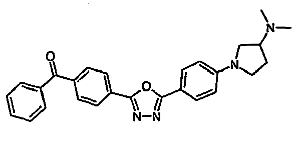 | C27H26N402 | 438,21 | 439,28 |
| 20 | 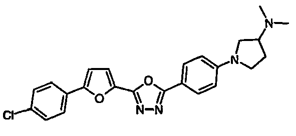 | C24H23ClN4O2 | 434,15 | 436,02 |
| 21 | 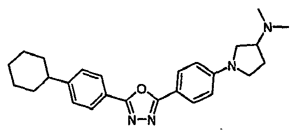 | C26H32N40 | 416,26 | 417,36 |
| 22 | 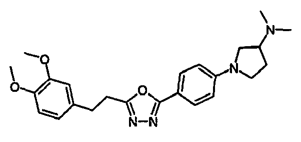 | C24H30N403 | 422,23 | 424,02 |
| 23 | 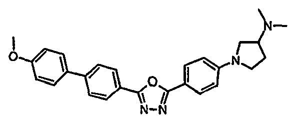 | C27H28N402 | 440,22 | 441,27 |
| 24 | 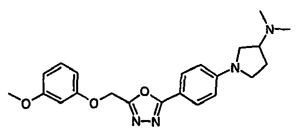 | C22H26N403 | 394,20 | 395,21 |
| 25 | 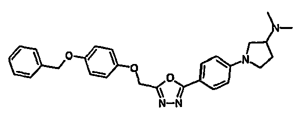 | C28H30N403 | 470,23 | 471,25 |
| 26 | 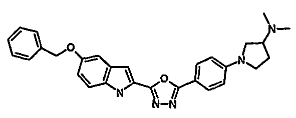 | C29H29N502 | 479,23 | 480,39 |
| 27 | 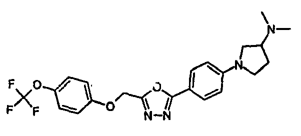 | C22H23F3N4O3 | 448,17 | 449,22 |
| 28 | 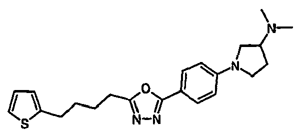 | C22H28N4OS | 396,20 | 397,22 |
| 29 | 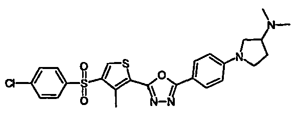 | C25H25ClN4O3S2 | 528,11 | 530,08 |
| 30 | 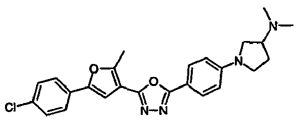 | C25H25ClN4O2 | 448,17 | 449,22 |
| 31 | 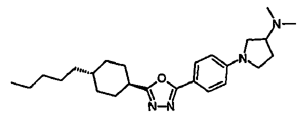 | C25H38N40 | 410,31 | 411,32 |
| 32 | 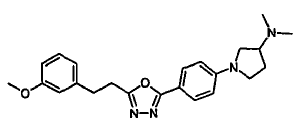 | C23H28N4O2 | 392,22 | 393,24 |
| 33 | 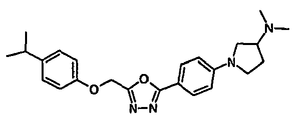 | C24H30N4O2 | 406,24 | 407,25 |
| 34 | 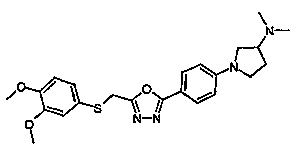 | C23H28N4O3S | 440,19 | 441,20 |
| 35 | 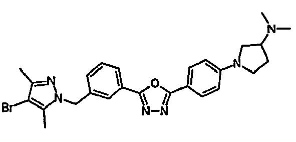 | C26H29BrN60 | 520,16 | 521,16 |
| 36 | 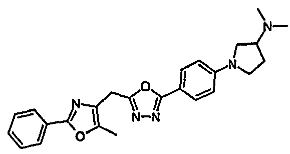 | C25H27N5O2 | 429,22 | 430,23 |
| 37 | 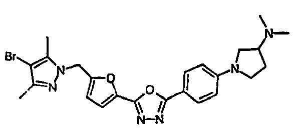 | C24H27BrN6O2 | 510,14 | 512,13 |
| 38 | 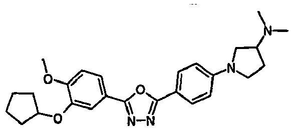 | C26H32N4O3 | 448,25 | 449,26 |
| 39 | 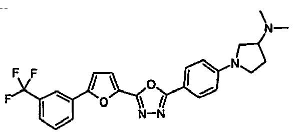 | C25H23F3N4O2 | 468,18 | 469,21 |
| 40 | 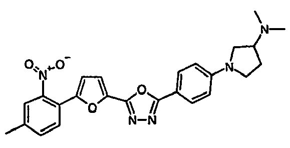 | C25H25N5O4 | 459,19 | 460,21 |
| 41 | 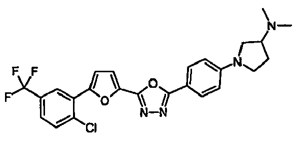 | C25H22C1F3N4O2 | 502,14 | 503,18 |
| 42 | 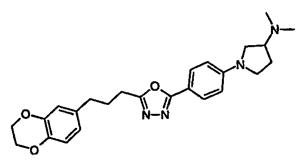 | C25H30N4O3 | 434,23 | 435,24 |
| 43 | 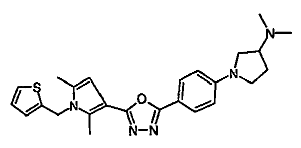 | C25H29N5OS | 447,21 | 448,21 |
| 44 | 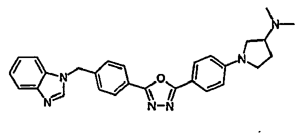 | C28H28N6O | 464,23 | 465,27 |
| 45 | 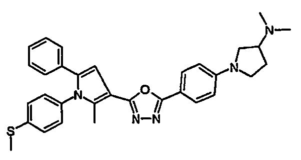 | C32H33N5OS | 535,24 | 536,25 |
| 46 | 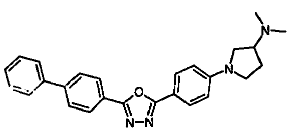 | C26H26N40 | 410,21 | 411,23 |
| 47 | 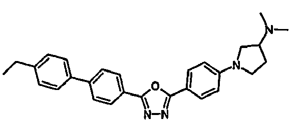 | C28H30N4O | 438,24 | 439,27 |
| 48 | 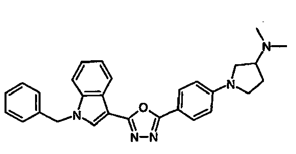 | C29H29N5O | 463,24 | 464,26 |
| 49 | 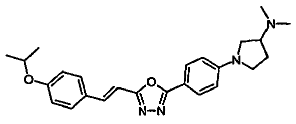 | C25H30N4O2 | 418,24 | 419,24 |
| 50 | 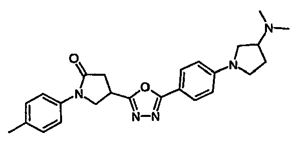 | C25H29N5O2 | 431,23 | 432,25 |
| 51 | 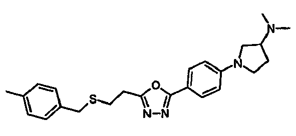 | C24H30N4OS | 422,21 | 423,23 |
| 52 | 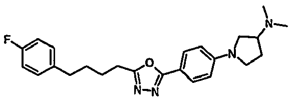 | C24H29FN4O | 408,23 | 409,24 |
| 53 | 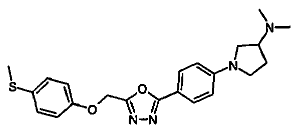 | C22H26N4O2S | 410,18 | 411,21 |
| 54 | 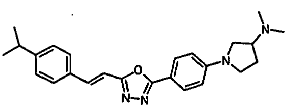 | C25H30N4O | 402,24 | 403,26 |
| 55 | 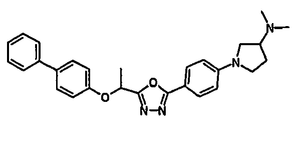 | C28H30N4O2 | 454,24 | 455,25 |
| 56 | 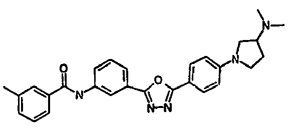 | C28H29N5O2 | 467,23 | 468,27 |
| 57 | 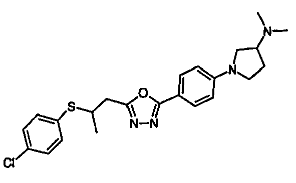 | C23H27ClN4OS | 442,16 | 443,17 |
| 58 | 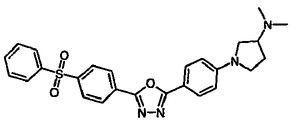 | C26H26N4O3S | 474,17 | 475,19 |
| 59 | 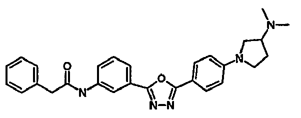 | C28H29N5O2 | 467,27 | 468,27 |
| 60 | 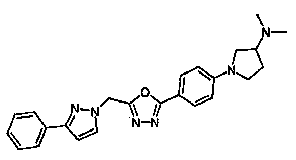 | C24H26N6O | 414,22 | 415,23 |
| 61 | 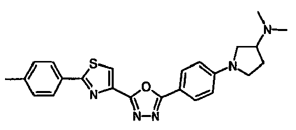 | C24H25N5OS | 431,18 | 432,19 |
| 62 | 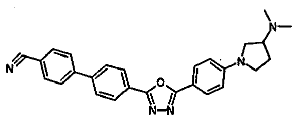 | C27H25N5O | 435,21 | 436,23 |
| 63 | 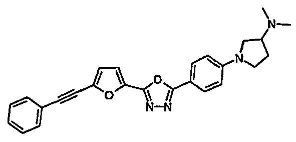 | C26H24N4O2 | 424,19 | 425,22 |
| 64 | 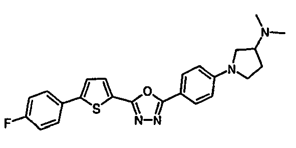 | C24H23FN4OS | 434,16 | 435,19 |
| 65 | 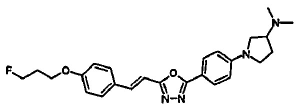 | C25H29FN4O2 | 436,23 | 437,26 |
| 66 | 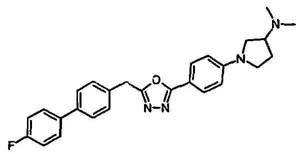 | C27H27FN4O | 442,22 | 443,24 |
| 67 | 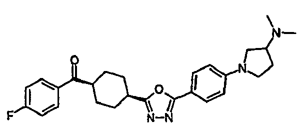 | C27H31FN4O2 | 462,24 | 464,36 |
| 68 | 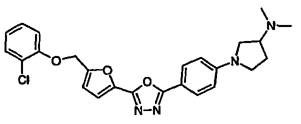 | C25H25ClN4O3 | 464,16 | 465,18 |
| 69 | 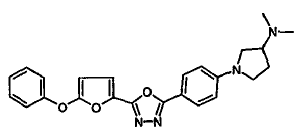 | C24H24N4O3 | 416,19 | 417,20 |
| 70 | 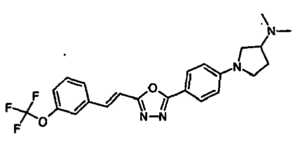 | C23H23F3N4O2 | 444,18 | 445,20 |
| 71 | 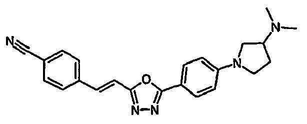 | C23H23N5O | 385,19 | 386,22 |
| 72 | 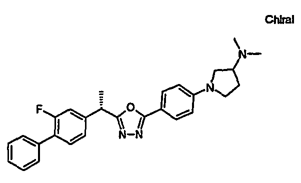 | C28H29FN4O | 456,23 | 457,25 |
| 73 | 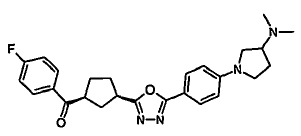 | C26H29FN4O2 | 448,23 | 449,24 |
| 74 | 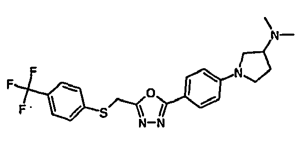 | C22H23F3N4OS | 448,15 | 449,16 |
| 75 | 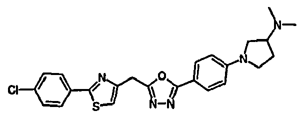 | C24H24ClN5OS | 465,14 | 466,15 |
| 76 | 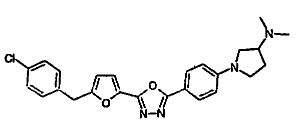 | C25H25ClN4O2 | 448,17 | 449,20 |
| 77 | 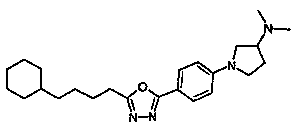 | C24H36N4O | 396,29 | 397,31 |
| 78 | 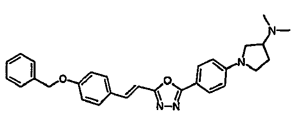 | C29H30N4O2 | 466,24 | 467,27 |
| 79 | 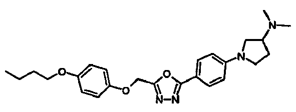 | C25H32N4O3 | 436,25 | 437,27 |
| 80 | 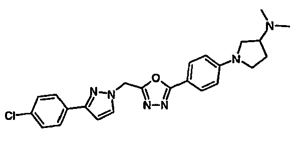 | C24H25ClN6O | 448,18 | 449,21 |
| 81 | 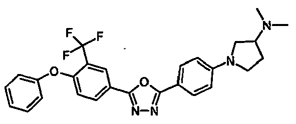 | C27H25F3N4O2 | 494,19 | 495,22 |
| 82 | 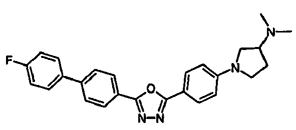 | C26H25FN4O | 428,20 | 429,23 |
| 83 | 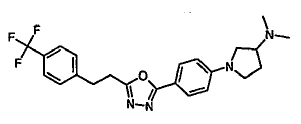 | C23H25F3N4O | 430,20 | 431,20 |
| 84 | 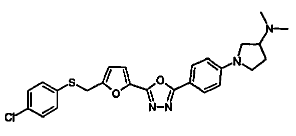 | C25H25ClN4O2S | 480,14 | 481,17 |
| 85 | 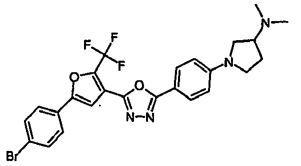 | C25H22BrF3N4O2 | 546,09 | 547,11 |
| 86 | 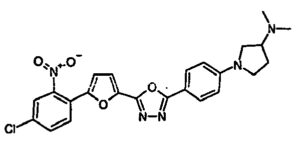 | C24H22ClN5O4 | 479,14 | 480,15 |
| 87 | 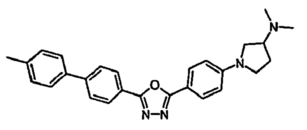 | C27H28N4O | 424,23 | 425,24 |
| 88 | 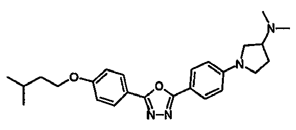 | C25H32N4O2 | 420,25 | 421,26 |
| 89 | 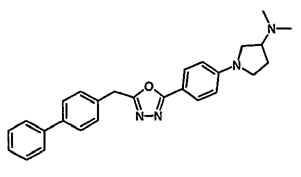 | C27H28N4O | 424,23 | 425,24 |
| 90 | 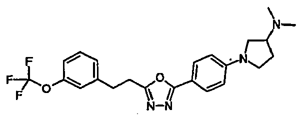 | C23H25F3N4O2 | 446,19 | 447,19 |
| 91 | 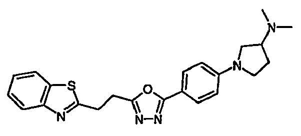 | C23H25N5OS | 419,18 | 420,11 |
| 92 | 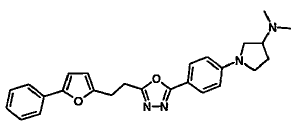 | C26H28N4O2 | 428,22 | 429,24 |

### Beispiel 93

### Dimethyl-(1-{4-[5-(4-phenoxy-butyl)-[1,3,4]oxadiazol-2-yl]-phenyl}-pyrrolidin-3-yl)-amin

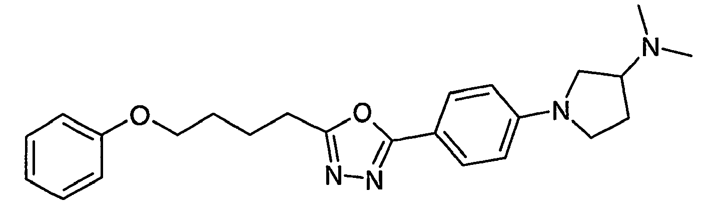

Eine Mischung aus 4-Phenoxy-butansäurehydrazid (10 mg), 4-(3-Dimethylamino-pyrrolidin-1-yl)-benzoesäure (12,1 mg), HOAt (6,6 mg), Triethylamin (13,5 µL) und DMF (0,15 mL) wurde bei 0 °C mit PyBOP (25,2 mg) versetzt. Es wurde 10 min bei 0 °C und anschließend 3h bei Raumtemperatur gerührt. Danach wurde die Reaktionslösung mit Ethylacetat und Wasser versetzt. Anschließend wurde die organische Phase je zweimal mit 10%iger Zitronensäurelösung, gesättigter Natriumhydrogencarbonatlösung und Wasser gewaschen, über Natriumsulfat getrocknet und das Lösungsmittel im Vakuum entfernt. Danach wurde der Rückstand in THF (0,4 mL) aufgenommen und mit EDC (6,1 mg) und Triethylamin (5,5 µL) versetzt und 16 h bei 50 °C gerührt. Anschließend wurde die Reaktionslösung mit Ethylacetat und Wasser versetzt. Danach die organische Phase je zweimal mit 10%iger Zitronensäurelösung, gesättigter Natriumhydrogencarbonatlösung und Wasser gewaschen, über Natriumsulfat getrocknet und das Lösungsmittel im Vakuum entfernt. Der Rückstand wurde durch präparative HPLC gereingt. Man erhielt so das Produkt mit dem Molekulargewicht 406,53 (C24H30N4O2); MS (ESI): 407,15 (M+H+).

### 4-Phenoxy-butansäurehydrazid

Eine Mischung aus Phenol (100 mg), Ethyl-5-bromvalerat (222,2 mg), Cäsiumcarbonat (693 mg) und DMF (1,6 mL) wurde 12 h bei Raumtemperatur gerührt. Anschließend wurde die Reaktionslösung mit Ethylacetat und Wasser versetzt und die wässrige Phase mit 2N HCl-Lösung auf pH 6 gestellt. Die wässrige Phase noch zweimal mit Ethylacetat extrahiert. Dann wurden die vereinigten organischen Phasen mit Wasser gewaschen, über Natriumsulfat getrocknet und das Lösungsmittel im Vakuum entfernt.

Das so erhaltene Produkt wurde in abs. Ethanol (0,5 mL) aufgenommen, mit 37,3 µL Hydrazinhydrat versetzt und 12 h am Rückfluß erhitzt. Anschließend wurde die Reaktionslösung mit Ethylacetat und Wasser versetzt und die wässrige Phase mit 2N HCl-Lösung neutralisiert. Die wässrige Phase wurde zweimal mit Ethylacetat extrahiert. Dann wurden die vereinigten organischen Phasen mit Wasser gewaschen, über Natriumsulfat getrocknet und das Lösungsmittel im Vakuum entfernt. Der Rückstand wurde durch präparative HPLC gereingt. Man erhielt so das Produkt mit dem Molekulargewicht 208,11 (C11H16ClN2O2); MS (ESI): 209,1 (M+H+).

### 4-(3-Dimethylamino-pyrrolidin-1-yl)-benzoesäure

Eine Mischung aus 4-(3-Amino-pyrrolidin-1-yl)-benzoesäuremethylester (300 mg), Essigsäure (81 µL), Formaldehyd (0,113 mL) und THF (3 mL) wurde mit einer 1 M Natriumcyanoborhydrid-Lösung (1,32 mL) versetzt. Die Reaktion wurde 1 h bei Raumtemperatur gerührt, dann das Lösungsmittel im Vakuum entfernt und der Rückstand in Ethylacetat/ Wasser wieder aufgenommen. Die organische Phase wurde mit Wasser gewaschen über Natriumsulfat getrocknet und das Lösungsmittel im Vakuum entfernt. Das so erhaltene Produkt wurde in einer THF/Wasser-Mischung (1:1) (3,1 mL) gelöst und mit Kaliumhydroxid (66,2 mg) versetzt. Die Reaktion wurde 4 h auf 70 °C erwärmt. Anschließend wurde die Reaktionslösung mit Ethylacetat und Wasser versetzt und die wässrige Phase mit 2N HCl-Lösung sauer gestellt. Die wässrige Phase wurde zweimal mit Ethylacetat extrahiert. Dann wurden die vereinigten organischen Phasen mit Wasser gewaschen, über Natriumsulfat getrocknet und das Lösungsmittel im Vakuum entfernt. Man erhielt so das Produkt mit dem Molekulargewicht 234,17 (C13H18N2O2); MS (ESI): 235,10 (M+H+).

### 4-(3-Amino-pyrrolidin-1-yl)-benzoesäuremethylester

Eine Mischung aus 3-(tert.-Butoxycarbonylamino)pyrrolidin (1g), Methyl 4-Fluorbenzoat (0,83 mL), Cäsiumcarbonat (1,7 g) und DMF (10 mL) wurde 12 h auf 100 °C erwärmt. Anschließend wurde die Reaktionslösung mit Ethylacetat und Wasser versetzt. Danach wurde die organische Phase zweimal mit Wasser gewaschen, über Natriumsulfat getrocknet und das Lösungsmittel im Vakuum entfernt.

Das so erhaltene Produkt wurde in Methylenchlorid (12 mL) gelöst, mit Trifluoressigsäure (6 mL) versetzt und 90 min bei Raumtemperatur gerührt. Das Lösungsmittel wurde im Vakuum entfernt und der Rückstand durch präparative HPLC gereingt. Man erhielt so das Produkt mit dem Molekulargewicht 220,12 (C12H16N2O2); MS (ESI): 221,10(M+H+).

### Beispiel 94

### (1-{4-[5-(4-Butoxy-phenyl)-[1,3,4]thiadiazol-2-yl]-phenyl}-pyrrolidin-3-yl)-dimethyl-amin

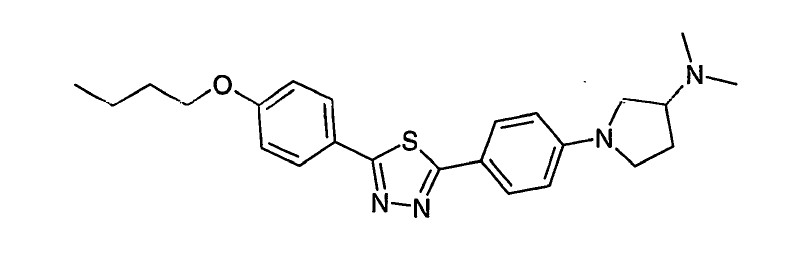

Eine Mischung aus 4-(3-Dimethylamino-pyrrolidin-1-yl)-benzoesäure-N'-(4-butoxy-benzoyl)-hydrazid (100 mg), Lawesson-Reagenz (191 mg) und Toluol (5 mL) wurde für 4 Stunden am Rückfluss gekocht. Flüchtige Anteile wurden entfernt und der Rückstand durch präparative HPLC gereingt. Man erhielt so das Produkt mit dem Molekulargewicht 422,60 (C24H30N4OS); MS (ESI): 423 (M+H+).

### 4-(3-Dimethylamino-pyrrolidin-1-yl)-benzoesäure-N'-(4-butoxy-benzoyl)-hydrazid

Eine Mischung aus 4-Butoxybenzoesäure (156 mg) und DMF (10 mL) wurde mit TOTU (264 mg) und N,N-Diisopropylethylamin (104 mg) versetzt. Nach 10 Minuten wurde 4-(3-Dimethylamino-pyrrolidin-1-yl)-benzoesäurehydrazid (200 mg) zugesetzt. Nach einer Stunde wurde die Mischung mit Wasser verdünnt und mit Ethylacetat extrahiert. Die organische Phase wurde mit Wasser gewaschen, über Magnesiumsulfat getrocknet, filtriert und eingeengt. Man erhielt so das Produkt mit dem Molekulargewicht 424,55 (C24H32N4O3); MS (ESI): 425 (M+H+).

### Beispiel 95

### ((R)-3-{4-[5-(4-Chlor-phenoxymethyl)-[1,2,4]oxadiazol-3-yl]-phenyl}-cyclopentyl)-methyl-amin

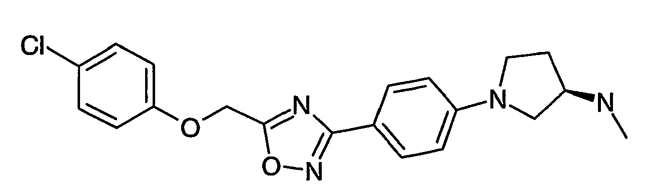

Zu einer Mischung aus {(R)-1-[4-(N-Hydroxycarbamimidoyl)-phenyl]-pyrrolidin-3-yl}methyl-carbaminsäure tert- butylester (358,5 mg), (4-Chlor-phenoxy)-essigsäure (200 mg) und 3 mL DMF wurden bei 0 °C PyBOP (558 mg), HOAt (146 mg) und Triethylamin (0,299 mL) gegeben und die Reaktionsmischung wurde 10 min bei dieser Temperatur nachgerührt. Anschließend wurde 2 h bei Raumtemperatur gerührt, die Reaktionslösung wurde mit Ethylacetat und Wasser versetzt. Danach wurde die organische Phase je zweimal mit 10%iger Zitronensäurelösung, gesättigter Natriumhydrogencarbonatlösung und Wasser gewaschen, über Natriumsulfat getrocknet, abfiltriert und das Lösungsmittel im Vakuum entfernt.

Danach wurde der Rückstand in 10 mL THF aufgenommen und mit EDC (160 mg) und Triethylamin (0,144 mL) versetzt und 16 h bei 50 °C gerührt. Anschließend wurde die Reaktionslösung mit Ethylacetat und Wasser versetzt. Danach wurde die organische Phase je zweimal mit 10%iger Zitronensäurelösung, gesättigter Natriumhydrogencarbonatlösung und Wasser gewaschen, über Natriumsulfat getrocknet, abfiltriert und das Lösungsmittel im Vakuum entfernt.

Das Rohprodukt wurde in Methylenchlorid (7,2 mL) aufgenommen und mit Trifluoressigsäure versetzt (1,8 mL). Die Reaktion wurde 5 h bei Raumtemperatur gerührt und anschließend das Lösungsmittel im Vakuum entfernt. Der Rückstand wurde durch präparative HPLC gereingt. Man erhielt so das Produkt mit dem Molekulargewicht 384,14 (C20H21ClN4O2); MS (ESI): 385,26 (M+H+).

### (4-Chlor-phenoxy)-essigsäure

Eine Mischung aus 4-Chlorphenol (500 mg), Methylbromacetat (0,368 mL), Cäsiumcarbonat (1,9 g) und 5 mL Aceton wurde 12 h bei Raumtemperatur gerührt. Anschließend wurde die Reaktionslösung mit Ethylacetat und Wasser versetzt und die organische Phase zweimal mit Wasser gewaschen, über Natriumsulfat getrocknet, abfiltriert und das Lösungsmittel im Vakuum entfernt. Das Rohprodukt wurde in einer 1:1 THF/Wasser-Mischung (10 mL) gelöst und mit Kaliumhydroxid (417 mg) versetzt. Die Reaktion wurde 12 h bei Raumtemperatur gerührt. Anschließend wurde die Reaktionslösung mit Ethylacetat und Wasser versetzt, die Wasserphase auf pH 2 gestellt und mehrfach mit Ethylacetat extrahiert. Die vereinigten organischen Phasen wurden zweimal mit Wasser gewaschen, über Natriumsulfat getrocknet, abfiltriert und das Lösungsmittel im Vakuum entfernt. Man erhielt so das Produkt mit dem Molekulargewicht 186,6 (C8H7ClO3); MS (ESI): 227,0 (M+CH3CN).

### {(R)-1-[4-(N-Hydroxycarbamimidoyl)-phenyl]-pyrrolidin-3-yl}-methyl-carbamic acid tert-butyl ester

Zu einer Lösung aus [(R)-1-(4-Cyano-phenyl)-pyrrolidin-3-yl]-carbaminsäure tertbutylester (1,06 g) in 12 mL DMF wurde bei 0 °C portionsweise Natriumhydrid (253,4 mg) zugegeben. Nach Entfernen des Eisbades wurde die Reaktionslösung tropfenweise mit Methyliodid (0,498 mL) versetzt und 4 h bei Raumtemperatur gerührt. Anschließend wurde die Reaktionslösung mit Ethylacetat und Wasser versetzt und die organische Phase zweimal mit Wasser gewaschen, über Natriumsulfat getrocknet,abfiltriert und das Lösungsmittel im Vakuum entfernt.

Das Rohprodukt wurde in abs. Ethanol (12 mL) gelöst und mit Hydroxylamin (0,687 mL) versetzt. Die Reaktionslösung wurde 12 h am Rückfluß erhitzt. Anschließend wurde die Reaktionslösung mit Ethylacetat und Wasser versetzt und die organische Phase zweimal mit Wasser gewaschen, über Natriumsulfat getrocknet und das Lösungsmittel im Vakuum entfernt. Der Rückstand wurde durch präparative HPLC gereingt. Man erhielt so das Produkt mit dem Molekulargewicht 334,42 (C17H26N4O3); MS (ESI): 335,20 (M+H+).

### [(R)-1-(4-Cyano-phenyl)-pyrrolidin-3-yl]-carbaminsäure

Eine Mischung aus (3R)-(+) -(tert-Butoxycarbonylaminopyrrolidin (1,0 g), p-Fluorbenzonitril (650 mg), Cäsiumcarbonat (1,75 g) und 5 mL DMF wurde 12 h bei 50 °C gerührt. Anschließend wurde die Reaktionslösung mit Ethylacetat und Wasser versetzt und die organische Phase zweimal mit Wasser gewaschen, über Natriumsulfat getrocknet, abfiltriert und das Lösungsmittel im Vakuum entfernt. Der Rückstand wurde durch präparative HPLC gereingt. Man erhielt so das Produkt mit dem Molekulargewicht 287,36 (C16H21N3O2); MS (ESI): 288,20 (M+H+).

### Beispiel 96

### ((R)-1-{4-[5-(4-Chlor-benzyloxymethyl)-[1,2,4]oxadiazol-3-yl]-phenyl} -pyrrolidin-3-yl)-methyl-amin

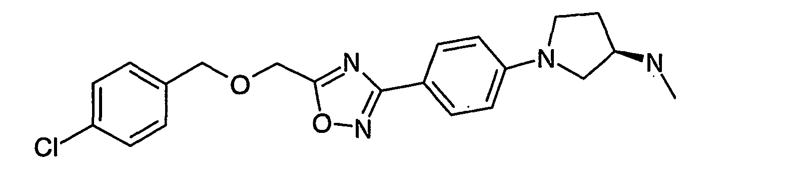

Eine Lösung aus (4-Chlor-benzyloxy)-essigsäure (70 mg) in Thionylchlorid (1 mL) wurde 1 h bei Raumtemperatur gerührt, anschließend mit Toluol versetzt und das Lösungsmittel im Vakuum abgezogen. Diese Prozedur wurde noch zweimal wiederholt. Anschließend wurde das Säurechlorid in Methylenchlorid (1,4 mL) aufgenommen, mit {(R)-1-[4-(N-Hydroxycarbamimidoyl)-phenyl]-pyrrolidin-3-yl}-methyl-carbamic acid tert- butyl ester (116,7 mg), Triethylamin (0,05 mL) versetzt und 12 h bei Raumtemperatur gerührt. Die leichtflüchtigen Komponenten wurden im Vakuum entfernt und der Rückstand in Ethylacetat/Wasser aufgenommen. Die organische Phase wurde zweimal mit Wasser gewaschen, über Natriumsulfat getrocknet und das Lösungsmittel im Vakuum entfernt. Der Rückstand wurde durch präparative HPLC gereingt. Man erhielt so das Produkt mit dem Molekulargewicht 398,15 (C21H23ClN4O2); MS (ESI): 399,41 (M+H+).

(4-Chlor-benzyloxy)-essigsäure wurde analog der (4-Chlor-phenoxy)-essigsäure unter Verwendung von 4-Chlorbenzylbromid als Edukt dargestellt. Man erhielt so das Produkt mit dem Molekulargewicht 200,62 (C9H9ClO3); MS (ESI): 222.95 (M+Na-H).

### Beispiel 97

### 4-Chlor-N-{3-[4-(3-dimethylamino-pyrrolidin-1-yl)-phenyl]-[1,2,4]oxadiazol-5-ylmethyl}-benzamid

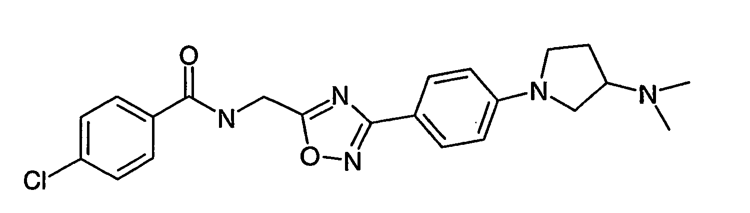

### Methode C

Zu einer Mischung aus {1-[4-(5-Aminomethyl-[1,2,4]oxadiazol-3-yl)-phenyl]-pyrrolidin-3-yl}-dimethyl-amin (75,3 mg), 4-Chlorbenzoesäure (23,5 mg) und DMF (0,96 mL) wurden TOTU (51,4 mg) und N,N-Diisopropylethylamin (0,05 mL) gegeben und die Lösung 12 h bei Raumtemperatur gerührt. Das Rohprodukt wurde über präparative HPLC (RP) gereinigt und anschließend nochmals chromatographisch an Kieselgel gereinigt (Dichlormethan/MeOH/AcOH/H2O = 90/10/1/1). Man erhielt so das Produkt mit dem Molekulargewicht 425,16 (C22H24ClN5O2); MS (ESI): 426,17 (M+H).

{1-[4-(5-Aminomethyl-[1,2,4]oxadiazol-3-yl)-phenyl]-pyrrolidin-3-yl}-dimethyl-amin Zu einer Mischung aus 4-(3-Dimethylamino-pyrrolidin-1-yl)-N-hydroxy-benzamidin (248,3 mg), tert-Butoxycarbonylamino-essigsäure (175,2 mg) und DMF (2,8 mL ) wurden bei 0 °C PyBOP (546,4 mg), HOAt (136,1 mg) und Triethylamin (0,279 mL) gegeben und es wurde 10 min bei dieser Temperatur nachgerührt. Anschließend wurde 2 h bei Raumtemperatur gerührt und dann die Reaktionslösung mit Ethylacetat und Wasser versetzt. Anschließend die organische Phase je zweimal mit 10%iger Zitronensäurelsg., gesättigter Natriumhydrogencarbonatlösung und Wasser gewaschen, über Natriumsulfat getrocknet, abfiltriert und das Lösungsmittel im Vakuum entfernt.

Danach wurde der Rückstand in 6,2 mL THF aufgenommen und mit EDC (105 mg) und Triethylamin (0,09 mL) versetzt und 16 h bei 50 °C gerührt. Anschließend wurde die Reaktionslösung mit Ethylacetat und Wasser versetzt. Danach die organische Phase je zweimal mit 10%iger Zitronensäurelösung., gesättigter Natriumhydrogencarbonatlösung und Wasser gewaschen, über Natriumsulfat getrocknet und das Lösungsmittel im Vakuum entfernt.

Das Rohprodukt wurde in Methylenchlorid (9,6 mL) aufgenommen und mit Trifluoressigsäure versetzt (2,3 mL). Die Reaktion wurde 5 h bei Raumtemperatur gerührt und anschließend das Lösungsmittel im Vakuum entfernt. Der Rückstand wurde durch präparative HPLC gereingt. Man erhielt so das Produkt mit dem Molekulargewicht 287,37 (C15H21N50); MS (ESI): 288,05 (M+H+).

### 4-(3-Dimethylamino-pyrrolidin-1-yl)-N-hydroxy-benzamidin

Eine Mischung aus 3-(Dimethylamino)pyrrolidin (1,1 g), p-Fluorbenzonitril (1,2 g), Kaliumcarbonat (2,8 g) und Acetonitril (15 mL) wurde 12 h bei 80 °C gerührt. Anschließend wurde die Reaktionslösung abfiltriert und der Niederschlag mit Acetonitril gewaschen, das Lösungsmittel im Vakuum entfernt und der Rückstand in Ethylacetat wieder aufgenommen. Die Ethylacetatphase wurde zweimal mit Wasser gewaschen, dann über Natriumsulfat getrocknet und das Lösungsmittel im Vakuum entfernt.

Das so erhaltene Produkt wurde in abs. Ethanol (48,7 mL) gelöst und die Lösung mit Hydroxylamin (1,4 mL) versetzt. Die Reaktionsmischung wurde 12 h am Rückfluß erhitzt. Nach dem Abkühlen der Reaktionslösung wurde der erhaltene Niederschlag abfiltriert und mit wenig Ethanol gewaschen.

Man erhielt so das Produkt mit dem Molekulargewicht 248,33 (C13H20N40); MS (ESI): 249,15 (M+H+).

Die Beispiele 98-110 in Tabelle 2 wurden nach Methode C synthetisiert.

| Bsp. | Struktur | Summenformel | MW Calc. | MW gemessen |
|---|---|---|---|---|
| 98 | 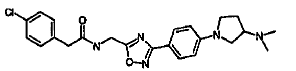 | C23H26ClN5O2 | 439,18 | 440,19 |
| 99 | 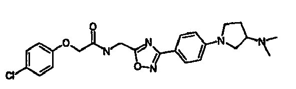 | C23H26ClN5O3 | 455,17 | 456,17 |
| 100 | 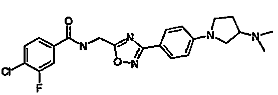 | C22H23ClFN5O2 | 443,15 | 444,21 |
| 101 | 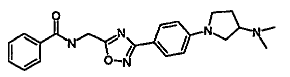 | C22H25N5O2 | 391,20 | 392,25 |
| 102 | 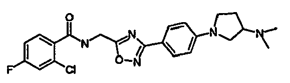 | C22H23ClFN5O2 | 443,15 | 444,21 |
| 103 | 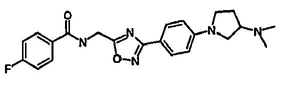 | C22H24FN5O2 | 409,19 | 410,22 |
| 104 | 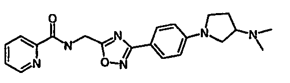 | C21H24N6O2 | 392,20 | 393,24 |
| 105 | 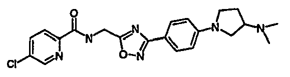 | C21H23ClN6O2 | 426,16 | 427,19 |
| 106 | 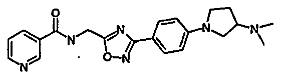 | C21H24N6O2 | 392,20 | 393,25 |
| 107 | 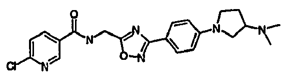 | C21H23ClN6O2 | 426,16 | 427,20 |
| 108 | 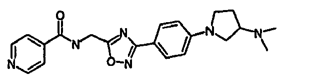 | C21H24N6O2 | 392,20 | 393,26 |
| 109 | 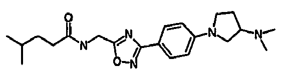 | C21H31N5O2 | 385,25 | 386,27 |
| 110 | 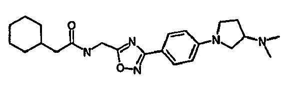 | C23H33N5O2 | 411,26 | 412,30 |

### Beispiel 111

### [1-(4-{5-[(4-Chlor-benzylamino)-methyl]-[1,2,4]oxadiazol-3-yl}-phenyl)-pyrrolidin-3-yl]-dimethyl-amin

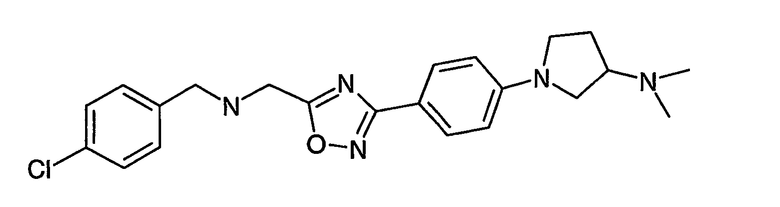

### Methode D

Zu einer Mischung aus {1-[4-(5-Aminomethyl-[1,2,4]oxadiazol-3-yl)-phenyl]-pyrrolidin-3-yl}-dimethyl- amin (40,1 mg), 4-Chlorbenzaldehyd (42,2 mg), Essigsäure (17,2 µL), Triethylamin (27,9 µL) und THF (1mL) wurde polymergebundenes Natriumcyanoborhydrid (5 eq) gegeben und die Reaktionsmischung 12 h bei Raumtemperatur gerührt. Das polymergebundene Reagenz wurde abfiltriert und das Lösungsmittel im Vakuum entfernt. Der Rückstand wurde durch präparative HPLC gereingt. Man erhielt so das Produkt mit dem Molekulargewicht 411,18 (C22H26ClN5O); MS (ESI): 412,23 (M+H+).

### Beispiel 112

### [1-(4-{5-[(4-Methoxy-benzylamino)-methyl]-[1,2,4]oxadiazol-3-yl}-phenyl)-pyrrolidin-3-yl]- dimethyl-amin

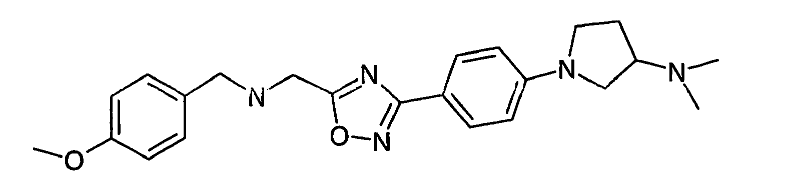

Nach Methode D wurde {1-[4-(5-Aminomethyl-[1,2,4]oxadiazol-3-yl)-phenyl]-pyrrolidin-3-yl}-dimethyl- amin mit p-Anisaldehyd umgesetzt. Man erhielt so das Produkt mit dem Molekulargewicht 407,23 (C23H29N502); MS (ESI): 408,30 (M+H+).

### Beispiel 113

### (1-{4-[5-(4-Fluor-benzyloxymethyl)-[1,2,4]oxadiazol-3-yl]-phenyl}-pyrrolidin-3-yl)-dimethyl-amin

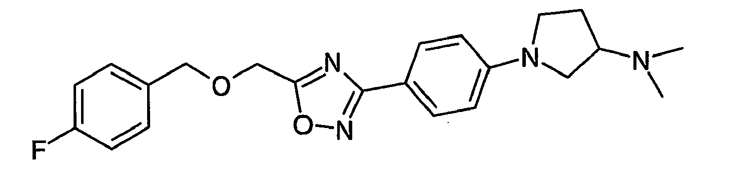

Zu einer Mischung aus {3-[4-(3-Dimethylamino-pyrrolidin-1-yl)-phenyl]-[1,2,4]oxadiazol-5-yl}-methanol (28,8 mg) und DMF (0,3 mL) wurden 4-Fluorbenzylbromid (18,9 mg) und Kaliumcarbonat (27,6 mg) gegeben. Die Reaktionsmischung wurde 3h bei 60 °C gerührt, abfiltriert und durch präparative HPLC gereingt. Man erhielt so das Produkt mit dem Molekulargewicht 396,20 (C22H25FN4O2); MS (ESI): 397,27 (M+H+).

{3-[4-(3-Dimethylamino-pyrrolidin-1-yl)-phenyl]-[1,2,4]oxadiazol-5-yl}-methanol Zu einer Mischung aus 4-(3-Dimethylamino-pyrrolidin-1-yl)-N-hydroxy-benzamidin (248,3 mg), Benzyloxyesssigsäure (0,143 mL) und DMF (3,0 mL ) wurden bei 0 °C PyBOP (546,4 mg), HOAt (136,1 mg) und Triethylamin (0,139 mL) gegeben und 10 min bei dieser Temperatur nachgerührt. Anschließend wurde 1 h bei Raumtemperatur gerührt und dann die Reaktionslösung mit Ethylacetat und Wasser versetzt. Anschließend wurde die organische Phase je zweimal mit 10%iger Zitronensäurelösung, gesättigter Natriumhydrogencarbonatlösung und Wasser gewaschen, über Natriumsulfat getrocknet und das Lösungsmittel im Vakuum entfernt.

Danach wurde der Rückstand in 3 mL THF aufgenommen und mit EDC (170,8 mg) und Triethylamin (0,139 mL) versetzt und 16 h bei 80 °C gerührt. Anschließend wurde die Reaktionslösung mit Ethylacetat und Wasser versetzt. Danach wurde die organische Phase je zweimal mit 10%iger Zitronensäurelsg., gesättigter Natriumhydrogencarbonatlsg. und Wasser gewaschen, über Natriumsulfat getrocknet und das Lösungsmittel im Vakuum entfernt.

Der Rückstand wurde in Methylenchlorid (4,4 mL) gelöst und die Lösung auf -78 °C abgekühlt. Bei dieser Temperatur wurde eine 1 M Bortrichloridlösung zugegeben (3 mL). Man ließ die Reaktion über Nacht auf Raumtemperatur erwärmen und versetzte sie anschließend mit Ethylacetat und Wasser. Man erhielt so das Produkt mit dem Molekulargewicht 288,2 (C15H20N4O2); MS (ESI): 289,2 (M+H+).

Analog könnten die folgenden Beispiele synthetisiert werden:

| | | | |
|---|---|---|---|
| A | 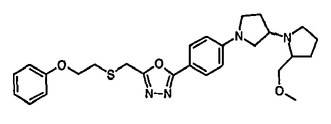 | T | 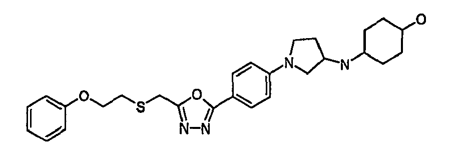 |
| B | 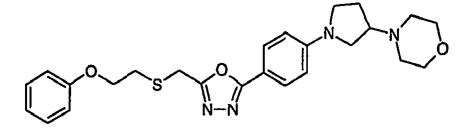 | U | 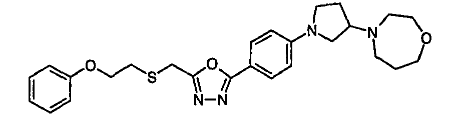 |
| C | 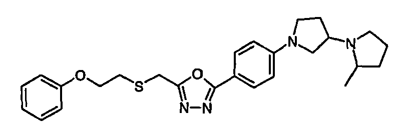 | V | 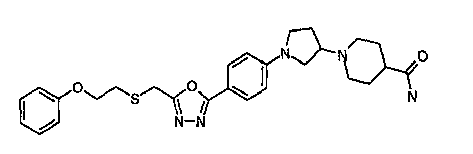 |
| D | 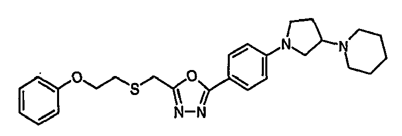 | W | 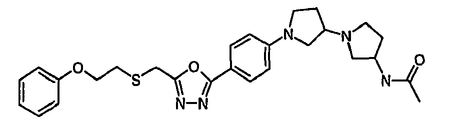 |
| E | 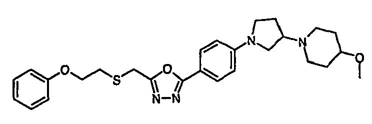 | X | 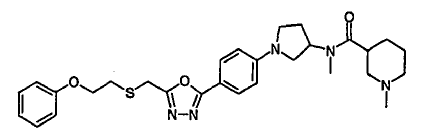 |
| F | 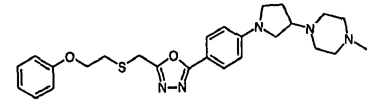 | Y | 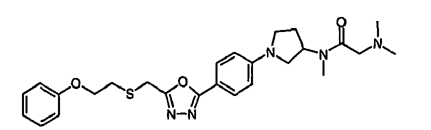 |
| G | 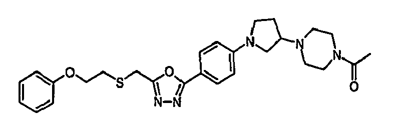 | Z | 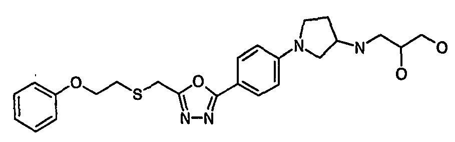 |
| H | 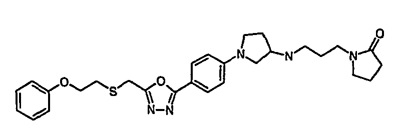 | AA | 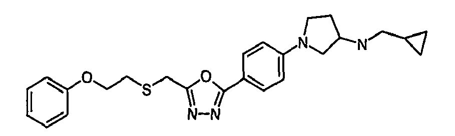 |
| I | 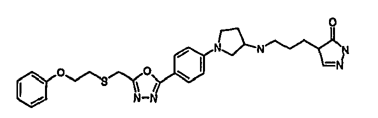 | AB | 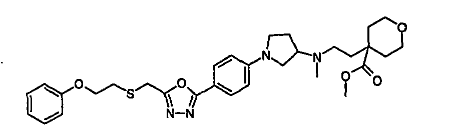 |
| K | 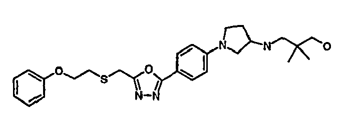 | AC | 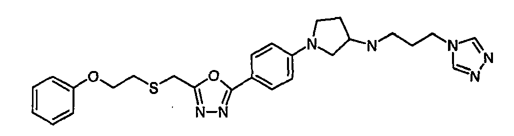 |
| L | 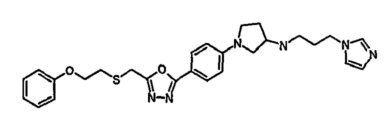 | AD | 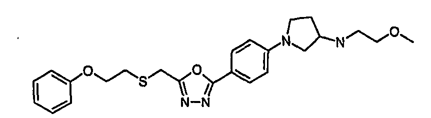 |
| M | 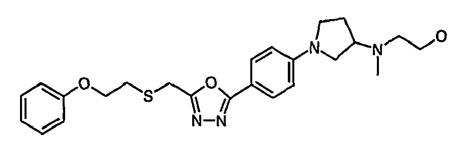 | AE | 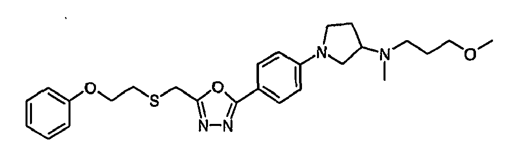 |
| N | 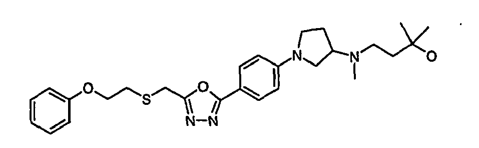 | AF | 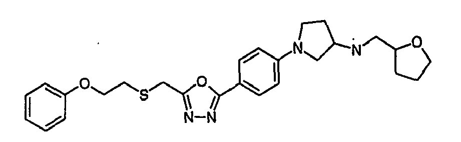 |
| O | 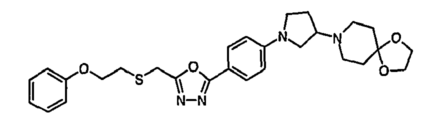 | AG | 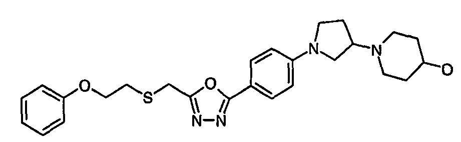 |
| P | 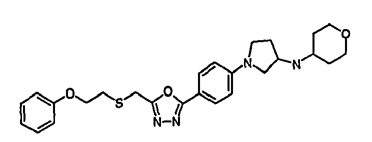 | AH | 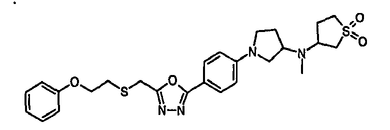 |
| Q | 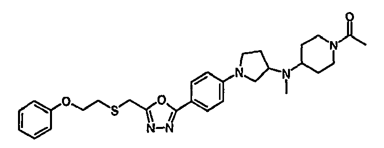 | AI | 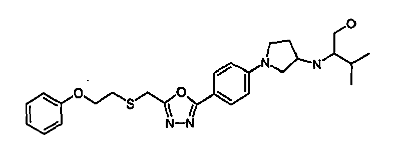 |
| R | 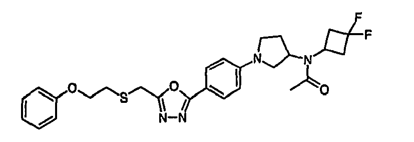 | | |
| S | 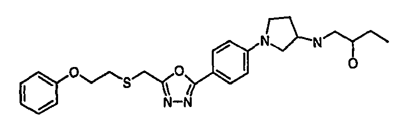 | | |

## Patentansprüche

1. Verbindungen der Formel I, 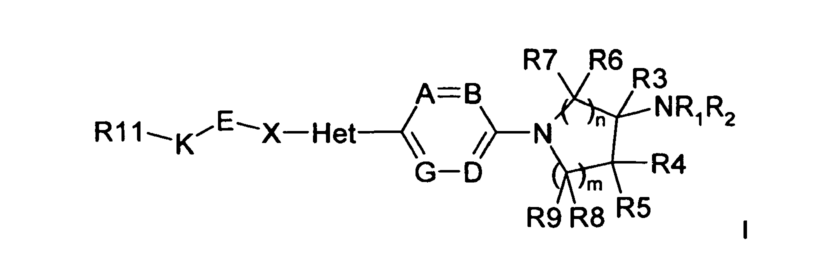 worin bedeuten
R1, R2 unabhängig voneinander H, (C₁-C₈)-Alkyl, -(CR13R14)ₒ-R12, (C₁-C₄)-Alkoxy-(C₁-C₄)-alkyl, (C₃-C₈)-Alkenyl, (C₃-C₈)-Alkinyl, CO-(C₁-C₈)-Alkyl, -CO-(CH₂)ₒ -R12, CO(C(R15)(R16))_{q}N(R17)(R18), CO(C(R19)(R20))ₛO(R21); oder R1 und R2 bilden zusammen mit dem Stickstoffatom, an das sie gebunden sind, einen 4 bis 10-gliedrigen mono-, bi- oder spirocyclischen Ring welcher ausser dem Stickstoffatom 0 bis 4 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, Cl, Br, CF₃, NO₂, CN, (C₁-C₆)-Alkyl, O-(C₁-C₈)-Alkyl, (C₁-C₄)- Alkoxy-(C₁-C₄)-alkyl, Hydroxy-(C₁-C₄)-Alkyl, (C₀-C₈)-Alkylen-aryl, Oxo, CO(R22), CON(R23)(R24), Hydroxy, COO(R25), N(R26)CO(C₁-C₆)-Alkyl, N(R27)(R28) oder SO₂CH₃;
o 0, 1, 2, 3, 4, 5, 6;
q, s unabhängig voneinander 0, 1, 2, 3, 4;
R15, R16, R17, R18, R19, R20, R21, R22, R23, R24, R25, R26, R27, R28 unabhängig voneinander H, (C₁-C₆)-Alkyl;
R17 und R18, R23 und R24, R27 und R28 unabhängig voneinander optional zusammen mit dem Stickstoffatom, an das sie gebunden sind, einen 5-6 gliedrigen Ring, welcher ausser dem Stickstoffatom noch 0-1 weitere Heteroatome aus der Gruppe NH, N-(C₁-C₆)-Alkyl, Sauerstoff und Schwefel beinhalten kann;
R12 OH, O-(C₁-C₆)-Alkyl, CN, COO(R29), CON(R30)(R31), N(R32)(R33), 3-12 gliedriger mono-, bi- oder spirocyclischer Ring, der ein oder mehrere Heteroatome aus der Gruppe N, O und S enthalten kann und der 3-12 gliedrige Ring weitere Substituenten wie F, Cl, Br, J, OH, CF₃, NO₂, CN, OCF₃, Oxo, O- (C₁-C₆)-Alkyl, (C₁-C₄)-Alkoxy-(C₁-C₄)-alkyl, S-(C₁-C₆)-Alkyl, (C₁- C₆)-Alkyl, (C₂-C₆)-Alkenyl, (C₃-C₈)-Cycloalkyl, (C₂-C₆)-Alkinyl, O- (C₀-C₈)-Alkylen-aryl, (C₀-C₈)-Alkylen-aryl, N(R34)(R35), CO(C₁- C₆)-Alkyl, COO(R36) und S(O)ᵤ (R37) enthalten kann;
u 0, 1, 2;
R34, R35 unabhängig voneinander H, (C₁-C₈)-Alkyl;
R34 und R35 optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten und optional mit 1-2 Oxo-Gruppen substituiert sein kann;
R36, R37 H, (C₁-C₈)-Alkyl;
R13, R14 unabhängig voneinander H, (C₁-C₈)-Alkyl, Hydroxy-(C₁-C₄)-Alkyl, OH, (C₁-C₄)-Alkoxy-(C₁-C₄)-Alkyl;
R29, R30, R31 unabhängig voneinander H, (C₁-C₈)-Alkyl, (C₂-C₆)-Alkenyl, (C₀- C₈)-Alkylen-Aryl;
R32, R33 unabhängig voneinander H, (C₁-C₆)-Alkyl
oder
R32 und R33 optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten und optional mit 1-2 Oxo-Gruppen substituiert sein kann;
R3 H, (C₁-C₆)-Alkyl;
R4, R5 unabhängig voneinander H, (C₁-C₆)-Alkyl, OH, O-(C₁-C₆)-Alkyl, O-CO(C₁-C₆)-Alkyl, S-(C₁-C₆)-Alkyl;
R6, R7, R8, R9 unabhängig voneinander H, (C₁-C₈)-Alkyl,
oder
R6 und R7, R8 und R9 unabhängig voneinander optional Oxo;
n, m unabhängig voneinander 0, 1, 2;
A, B, D, G unabhängig voneinander N, C(R38) und die Gesamtzahl der Stickstoffatome in diesem Ring 0 oder 1 beträgt, wobei für den Fall, dass die Gesamtzahl der Stickstoffatome 1 ist, A oder B N bedeutet;
R38 H, F, Cl, Br, I, OH, CF₃, NO₂, CN, OCF₃, O-(C₁-C₆)-Alkyl, O-(C₁- C₄)-Alkoxy-(C₁-C₄)-alkyl, S-(C₁-C₆)-Alkyl, (C₁-C₆)-Alkyl, (C₂-C₆)- Alkenyl, (C₃-C₈)-Cycloalkyl, O-(C₃-C₈)-Cycloalkyl, (C₃-C₈)-Cycloalkenyl, O-(C₃-C₈)-Cycloalkenyl, (C₂-C₆)-Alkinyl, (C₀-C₈)- Alkylen-Aryl, O-(C₀-C₈)-Alkylen-Aryl, S-Aryl, N(R39)(R40), SO₂- CH₃, COOH, COO-(C₁-C₆)-Alkyl, CON(R41)(R42), N(R43)CO(R44), N(R45)SO₂(R46), CO(R47), -(CR48R49)ₓ- O(R50);
R39, R40, R41, R42, R43, R45 unabhängig voneinander H, (C₁-C₈)-Alkyl;
oder
R39 und R40, R41 und R42 unabhängig voneinander optional zusammen mit dem Stickstoffatom, an das sie gebunden sind, einen 5-6 gliedrigen Ring, welcher ausser dem Stickstoffatom noch 0-1 weitere Heteroatome aus der Gruppe NH, N-(C₁-C₆)-Alkyl, Sauerstoff und Schwefel beinhalten kann;
R44, R46, R47 unabhängig voneinander H, (C₁-C₈)-Alkyl, Aryl;
R48, R49 unabhängig voneinander H, (C₁-C₈)-Alkyl;
R50 H, (C₁-C₆)-Alkyl;
x 1, 2, 3, 4;
Het ausgewählt ist aus der Gruppe bestehend aus Oxadiazolen, Thiadiazolen, Triazolen, Thiophenen Furanen und Pyrrolen;
X eine Bindung, eine Gruppe der Formel -(CR51 R52)y-, worin eine oder mehrere Gruppen -(CR51 R52)- durch Y ersetzt sein können, wobei sich ein chemisch sinnvoller Rest ergibt, C=C, C≡C;
Y O, S, N(R53), CO, SO, SO₂;
R51, R52 unabhängig voneinander H, (C₁-C₄)-Alkyl, wobei R51 und R52 in den y Gruppen jeweils die gleichen oder verschiedene Bedeutungen aufweisen können;
y 1, 2, 3, 4, 5, 6;
R53 H, (C₁-C₈)-Alkyl;
E 3-14 gliedrige bivalente carbo- oder heterocyclische Ringstruktur mit 0-4 Heteroatomen aus der Gruppe N, O und S, die optional Substituenten aus der Gruppe H, F, Cl, Br, I, OH, CF₃, NO₂, CN, OCF₃, Oxo, O-(C₁-C₆)-Alkyl, O-(C₁-C₄)-Alkoxy-(C₁-C₄)-alkyl, S- (C₁-C₆)-Alkyl, (C₁-C₆)-Alkyl, (C₂-C₆)-Alkenyl, (C₃-C₈)-Cycloalkyl, O-(C₃-C₈)-Cycloalkyl, (C₂-C₆)-Alkinyl, (C₀-C₈)-Alkylen-aryl, O-(C₀- C₈)-Alkylen-aryl, S-Aryl, N(R54)(R55), SO₂-CH_{3,} COOH, COO- (C₁-C₆)-Alkyl, CON(R56)(R57), N(R58)CO(R59), N(R60)SO₂(R61), CO(R62) tragen und mono- oder bicyclisch sein kann;
R54, R55, R56, R57, R58, R60 unabhängig voneinander H, (C₁-C₈)-Alkyl;
R54 und R55, R56 und R57 unabhängig voneinander optional zusammen mit dem Stickstoffatom, an das sie gebunden sind, einen 5-6 gliedrigen Ring, welcher ausser dem Stickstoffatom noch 0-1 weitere Heteroatome aus der Gruppe NH, N-(C₁-C₆)-Alkyl, Sauerstoff und Schwefel beinhalten kann;
R59, R61, R62 unabhängig voneinander H, (C₁-C₈)-Alkyl, Aryl;
K eine Bindung, eine Gruppe der Formel -(CR63R64)_{z}-, worin eine oder mehrere Gruppen -(CR63R64)- durch Z ersetzt sein können, wobei sich ein chemisch sinnvoller Rest ergibt, C≡C, C=C;
Z O, S, N(R65), CO, SO, SO₂;
R63, R64 unabhängig voneinander H, (C₁-C₈)-Alkyl, Hydroxy, (C₁-C₆)-Alkoxy, wobei R63 und R64 in den z Gruppen jeweils die gleichen oder verschiedene Bedeutungen aufweisen können;
z 1, 2, 3, 4, 5, 6;
R65 H, (C₁-C₈)-Alkyl;
R11 H, (C₁-C₈)-Alkyl, (C₁-C₄)-Alkoxy-(C₁-C₄)-alkyl, (C₃-C₈)-Alkenyl, (C₃-C₈)-Alkinyl, ein 3 bis 10-gliedriger mono-, bi- oder spirocyclischer Ring, welcher 0 bis 4 Heteroatome beinhalten kann, ausgewählt aus der Gruppe Sauerstoff, Stickstoff und Schwefel, wobei das Ringsystem zusätzlich substituiert sein kann mit F, Cl, Br, CF₃, NO₂, CN, (C₁-C₆)-Alkyl, O-(C₁-C₈)-Alkyl, (C₁-C₄)-Alkoxy-(C₁-C₄)-alkyl, (C₀-C₈)-Alkylen-aryl, Oxo, CO(R66), CON(R67)(R68), Hydroxy, Hydroxy-(C₁-C₄)-alkyl, COO(R69), N(R70)CO(C₁-C₆)-Alkyl, N(R71)(R72) oder SO₂CH₃;
R66, R67, R68, R69, R70, R71, R72 unabhängig voneinander H, (C₁-C₈)-Alkyl;
oder
R67 und R68, R71 und R72 unabhängig voneinander optional zusammen mit dem Stickstoffatom, an das sie gebunden sind, einen 5-6 gliedrigen Ring, welcher ausser dem Stickstoffatom noch 0-1 weitere Heteroatome aus der Gruppe NH, N-(C₁-C₆)-Alkyl, Sauerstoff und Schwefel beinhalten kann; oder
deren N-Oxide sowie deren physiologisch verträgliche Salze.

2. Verbindungen der Formel I nach Anspruch 1,
worin bedeuten
R1, R2 unabhängig voneinander H, (C₁-C₈)-Alkyl, -(CR13R14)ₒ -R12, (C₁-C₄)-Alkoxy-(C₁-C₄)-alkyl, (C₃-C₈)-Alkenyl, (C₃-C₈)-Alkinyl, CO-(C₁-C₈)-Alkyl, -CO-(CH₂)o -R12, CO(C(R15)(R16))_{q}N(R17)(R18), CO(C(R19)(R20))ₛO(R21); oder R1 und R2 bilden zusammen mit dem Stickstoffatom, an das sie gebunden sind, einen 4 bis 10-gliedrigen mono-, bi- oder spirocyclischen Ring welcher ausser dem Stickstoffatom 0 bis 4 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, Cl, Br, CF₃, NO₂, CN, (C₁-C₆)-Alkyl, O-(C₁-C₄)-Alkyl, (C₁-C₄)- Alkoxy-(C₁-C₄)-alkyl, Hydroxy-(C₁-C₄)-alkyl, (C₀-C₂)-Alkylen-aryl, Oxo, CO(R22), CON(R23)(R24), Hydroxy, COO(R25), N(R26)CO(C₁-C₆)-Alkyl oder N(R27)(R28);
R12 OH, O-(C₁-C₆)-Alkyl, CN, COO(R29), CON(R30)(R31), N(R32)(R33), 3-12 gliedriger mono-, bi- oder spirocyclischer Ring, der ein oder mehrere Heteroatome aus der Gruppe N, O und S enthalten kann und der 3-12 gliedrige Ring weitere Substituenten wie F, Cl, Br, I, OH, CF₃, NO₂, CN, OCF₃, Oxo, O- (C₁-C₆)-Alkyl, (C₁-C₄)-Alkoxy-(C₁-C₄)-alkyl, S-(C₁-C₆)-Alkyl, (C₁- C₆)-Alkyl, (C₂-C₆)-Alkenyl, (C₃-C₈)-Cycloalkyl, (C₂-C₆)-Alkinyl, O- (C₀-C₈)-Alkylen-Aryl, (C₀-C₈)-Alkylen-aryl, N(R34)(R35), CO(C₁- C₆)-Alkyl und COO(R36) enthalten kann; und
R6, R7, R8, R9 unabhängig voneinander H, (C₁-C₈)-Alkyl;
wobei die weiteren Reste und Gruppen in den Verbindungen der Formel I die in Anspruch 1 genannten Bedeutungen aufweisen.

3. Verbindungen der Formel I nach Anspruch 1,
worin bedeuten
R1, R2 unabhängig voneinander H, (C₁-C₈)-Alkyl, -(CR13R14)ₒ -R12, (C₁-C₄)-Alkoxy-(C₁-C₄)-alkyl, (C₃-C₈)-Alkenyl, CO-(C₁-C₈)-Alkyl, - CO-(CH₂)ₒ -R12, CO(C(R15)(R16))_{q}N(R17)(R18), CO(C(R19)(R20))ₛO(R21); oder R1 und R2 bilden zusammen mit dem Stickstoffatom, an das sie gebunden sind, einen 4 bis 10- gliedrigen mono-, bi- oder spirocyclischen Ring welcher ausser dem Stickstoffatom 0 bis 2 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, Cl, CF₃, (C₁-C₆)-Alkyl, O-(C₁-C₄)- Alkyl, (C₁-C₄)-Alkoxy-(C₁-C₄)-alkyl, Hydroxy-(C₁-C₄)-alkyl, (C₀- C₂)-Alkylen-aryl, Oxo, CO(R22), CON(R23)(R24), Hydroxy, COO(R25), N(R26)CO(C₁-C₆)-Alkyl, N(R27)(R28) oder SO₂CH₃;
o 0, 1, 2, 3, 4, 5, 6;
q 1, 2, 3;
s 0, 1, 2, 3, 4;
R15, R16, R17, R18, R19, R20, R21, R22, R23, R24, R25, R26, R27, R28 unabhängig voneinander H, (C₁-C₆)-Alkyl;
oder
R17 und R18, R23 und R24, R27 und R28 unabhängig voneinander optional zusammen mit dem Stickstoffatom, an das sie gebunden sind, einen 5-6 gliedrigen Ring, welcher ausser dem Stickstoffatom noch 0-1 weitere Heteroatome aus der Gruppe NH, N-(C₁-C₆)-Alkyl, Sauerstoff und Schwefel beinhalten kann;
R12 OH, O-(C₁-C₆)-Alkyl, CN, COO(R29), CON(R30)(R31), 3-12 gliedriger mono-, bi- oder spirocyclischer Ring, der ein oder mehrere Heteroatome aus der Gruppe N, O und S enthalten kann und der 3-12 gliedrige Ring weitere Substituenten wie F, Cl, Br, OH, CF₃, CN, Oxo, O-(C₁-C₆)-Alkyl, (C₁-C₄)-Alkoxy-(C₁-C₄)- alkyl, (C₁-C₆)-Alkyl, O-(C₀-C₈)-Alkylen-Aryl, (C₀-C₈)-Alkylen-aryl, N(R34)(R35), CO(C₁-C₆)-Alkyl, COO(R36), S(O)ᵤ(R37) enthalten kann;
u 0, 1, 2;
R34, R35 unabhängig voneinander H, (C₁-C₈)-Alkyl;
oder
R34 und R35 optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten und optional mit 1-2 Oxo-Gruppen substituiert sein kann;
R36, R37 H, (C₁-C₈)-Alkyl;
R13, R14 unabhängig voneinander H, (C₁-C₈)-Alkyl, Hydroxy-(C₁-C₄)-Alkyl, OH, (C₁-C₄)-Alkoxy-(C₁-C₄)-alkyl;
R29, R30, R31 unabhängig voneinander H, (C₁-C₈)-Alkyl;
R3 H, (C₁-C₆)-Alkyl;
R4, R5 unabhängig voneinander H, (C₁-C₆)-Alkyl, OH, O-(C₁-C₆)-Alkyl, O-CO(C₁-C₆)-Alkyl, S-(C₁-C₆)-Alkyl;
R6, R7, R8, R9 H;
oder
R6 und R7, R8 und R9 unabhängig voneinander optional Oxo;
n 1
m 1 oder 2;
A,B,D,G unabhängig voneinander N, C(R38) und die Gesamtzahl der Stickstoffatome in diesem Ring 0 oder 1 beträgt, wobei für den Fall, dass die Gesamtzahl der Stickstoffatome 1 ist, A oder B N bedeutet; oder die Gruppen A und B oder D und G sind jeweils C(R38) und bilden gemeinsam eine ortho-Phenyleneinheit, so dass sich insgesamt ein 1,4-bisubstituiertes Naphthalinsystem ergibt;
R38 H, F, Cl, Br, CF₃, CN, O-(C₁-C₆)-Alkyl, O-(C₁-C₄)-Alkoxy-(C₁-C₄)- alkyl, S-(C₁-C₆)-Alkyl, (C₁-C₆)-Alkyl, (C₀-C₈)-Alkylen-Aryl, O-(C₀- C₈)-Alkylen-Aryl, N(R39)(R40), SO₂-CH₃, CON(R41)(R42), N(R43)CO(R44), CO(R47), -(CR48R49)ₓ-O(R50); R39, R40, R41, R42, R43 unabhängig voneinander H, (C₁-C₈)-Alkyl;
oder
R39 und R40, R41 und R42 unabhängig voneinander optional zusammen mit dem Stickstoffatom, an das sie gebunden sind, einen 5-6 gliedrigen Ring, welcher ausser dem Stickstoffatom noch 0-1 weitere Heteroatome aus der Gruppe NH, N-(C₁-C₆)-Alkyl, Sauerstoff und Schwefel beinhalten kann;
R44, R47 unabhängig voneinander H, (C₁-C₈)-Alkyl, Aryl;
R48, R49 H;
R50 H, (C₁-C₆)-Alkyl;
x 1, 2;
Het ausgewählt ist aus der Gruppe bestehend aus Oxadiazolen, Thiadiazolen, Triazolen, Thiophenen, Furanen und Pyrrolen;
X eine Bindung, eine Gruppe der Formel -(CR51R52)_{y}-, worin eine oder mehrere Gruppen -(CR51R52)- durch Y ersetzt sein können, wobei sich ein chemisch sinnvoller Rest ergibt, C=C, C≡C;
R51, R52 unabhängig voneinander H, (C₁-C₄)-Alkyl, wobei R51 und R52 in den y Gruppen jeweils die gleichen oder verschiedene Bedeutungen aufweisen können;
Y O, S, N(R53), CO;
R53 H, (C₁-C₈)-Alkyl;
E 3-8 gliedrige bivalente carbo- oder heterocyclische Ringstruktur mit 0-4 Heteroatomen aus der Gruppe N, O und S, die optional Substituenten aus der Gruppe H, F, Cl, Br, OH, CF₃, NO₂, CN, OCF₃, O-(C₁-C₆)-Alkyl, O-(C₁-C₄)-Alkoxy-(C₁-C₄)-alkyl, S-(C₁-C₆)- Alkyl, (C₁-C₆)-Alkyl, (C₂-C₆)-Alkenyl, O-(C₃-C₈)-Cycloalkyl, (C₂- C₆)-Alkinyl, (C₀-C₈)-Alkylen-aryl, O-(C₀-C₈)-Alkylen-aryl, S-Aryl, N(R54)(R55), SO₂-CH₃, N(R58)CO(R59), N(R60)SO₂(R61), CO(R62) tragen und mono- oder bicyclisch sein kann;
R54, R55, R58, R60 unabhängig voneinander H, (C₁-C₈)-Alkyl;
R59, R61, R62 unabhängig voneinander H, (C₁-C₈)-Alkyl, Aryl;
K O, eine Bindung, CH₂O, OCH₂, S, SO, SO₂, N(R80), N(R81)CO, CON(R82), (C(R83)(R84))ᵥ, CO, C=C, C≡C, SCH₂, SO₂CH₂;
v 1, 2, 3, 4;
R80, R81, R82, R83, R84 unabhängig voneinander H, (C₁-C₈)-Alkyl;
R11 H, (C₁-C₈)-Alkyl, (C₁-C₄)-Alkoxy-(C₁-C₄)-alkyl, (C₃-C₈)-Alkenyl, (C₃-C₈)-Alkinyl, ein 3 bis 10-gliedriger mono-, bi- oder spirocyclischer Ring, welcher 0 bis 4 Heteroatome beinhalten kann, ausgewählt aus der Gruppe Sauerstoff, Stickstoff und Schwefel, wobei das Ringsystem zusätzlich substituiert sein kann mit F, Cl, Br, CF₃, CN, (C₁-C₆)-Alkyl, O-(C₁-C₈)-Alkyl, (C₁- C₄)-Alkoxy-(C₁-C₄)-alkyl, Hydroxy-(C₁-C₄)-alkyl, (C₀-C₈)-Alkylen- aryl, Oxo, CO(R66), CON(R67)(R68), Hydroxy, COO(R69), N(R70)CO(C₁-C₆)-Alkyl, N(R71)(R72) oder SO₂CH₃;
R66, R67, R68, R69, R70, R71, R72 unabhängig voneinander H, (C₁-C₈)-Alkyl;
oder
R67 und R68, R71 und R72 unabhängig voneinander optional zusammen mit dem Stickstoffatom, an das sie gebunden sind, einen 5-6 gliedrigen Ring, welcher ausser dem Stickstoffatom noch 0-1 weitere Heteroatome aus der Gruppe NH, N-(C₁-C₆)-Alkyl, Sauerstoff und Schwefel beinhalten kann; oder
deren N-Oxide sowie deren physiologisch verträgliche Salze.

4. Verbindungen der Formel I nach Anspruch 1 oder 3,
worin bedeuten:
R1, R2 unabhängig voneinander H, (C₁-C₈)-Alkyl, -(CR13R14)ₒ -R12, (C₁-C₄)-Alkoxy-(C₁-C₄)-alkyl, CO-(C₁-C₈)- Alkyl, -CO-(CH₂)ₒ -R12, CO(C(R15)(R16))_{q}N(R17)(R18), CO(C(R19)(R20))ₛO(R21); oder R1 und R2 bilden zusammen mit dem Stickstoffatom, an das sie gebunden sind, einen 4 bis 10- gliedrigen mono- oder bicyclischen Ring welcher ausser dem Stickstoffatom 0 bis 2 zusätzliche Heteroatome beinhalten kann, ausgewählt aus der Gruppe Sauerstoff, Stickstoff und Schwefel, wobei das heterocyclische Ringsystem zusätzlich substituiert sein kann mit F, Cl, CF₃, (C₁-C₆)-Alkyl, O-(C₁-C₄)-Alkyl, (C₁-C₄)- Alkoxy-(C₁-C₄)-alkyl, (C₀-C₂)-Alkylen-Aryl, Oxo, CO(R22), Hydroxy, N(R27)(R28) oder SO₂CH₃; bevorzugt unabhängig voneinander H, (C₁-C₈)-Alkyl, - (CR13R14)ₒ -R12, (C₁-C₄)-Alkoxy-(C₁-C₄)-alkyl, CO-(C₁-C₈)- Alkyl, -CO-(CH₂)ₒ -R12, CO(C(R15)(R16))_{q}N(R17)(R18), oder R1 und R2 bilden zusammen mit dem Stickstoffatom, an das sie gebunden sind, einen 4 bis 10-gliedrigen mono- oder bicyclischen Ring welcher ausser dem Stickstoffatom 0 bis 2 zusätzliche Heteroatome beinhalten kann, ausgewählt aus der Gruppe Sauerstoff und Stickstoff, wobei das heterocyclische Ringsystem zusätzlich substituiert sein kann mit F, (C₁-C₆)-Alkyl, (C₁-C₄)-Alkoxy-(C₁-C₄)-alkyl, Oxo, CO(R22), Hydroxy, N(R27)(R28);
o 0, 1, 2, 3, 4; bevorzugt 0, 1, 2, 3;
q 1 oder 2;
s 0, 1, 2, 3; bevorzugt 0, 1, 2;
R15, R16, R17, R18, R19, R20, R21, R22, R27, R28 unabhängig voneinander H, (C₁-C₆)-Alkyl;
oder
R17 und R18, R27 und R28 unabhängig voneinander optional zusammen mit dem Stickstoffatom, an das sie gebunden sind, einen 5-6 gliedrigen Ring ausgewählt aus Pyrrolidin, Piperidin, N-Methylpiperazin und Morpholin;
R12 OH, O-(C₁-C₆)-Alkyl, CN, 3-10 gliedriger mono-, oder bicyclischer Ring, der 1-3 Heteroatome aus der Gruppe N, O und S enthalten kann und der 3-10 gliedrige Ring weitere Substituenten wie F, Cl, Br, OH, CF₃, CN, Oxo, O-(C₁-C₆)-Alkyl, (C₁-C₄)-Alkoxy-(C₁-C₄)-alkyl, (C₁-C₆)-Alkyl, (C₀-C₂)-Alkylen-aryl, N(R34)(R35), CO(C₁-C₆)-Alkyl enthalten kann; bevorzugt OH, O-(C₁-C₆)-Alkyl, 3-10 gliedriger mono-, oder bicyclischer Ring, der 1-2 Heteroatome aus der Gruppe N, O und S enthalten kann und der 3-10 gliedrige Ring weitere Substituenten wie F, OH, Oxo, (C₁-C₆)-Alkyl, CO(C₁-C₆)-Alkyl enthalten kann;
u 0 oder 2; bevorzugt 2;
R34, R35 unabhängig voneinander H, (C₁-C₈)-Alkyl;
oder
R34 und R35 optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten und optional mit 1-2 Oxo-Gruppen substituiert sein kann;
R36, R37 H, (C₁-C₈)-Alkyl;
R13, R14 unabhängig voneinander H, (C₁-C₈)-Alkyl, Hydroxy-(C₁-C₄)-Alkyl, OH, (C₁-C₄)-Alkoxy-(C₁-C₄)-alkyl;
R3 H;
R4, R5 unabhängig voneinander H, (C₁-C₆)-Alkyl, OH, O-(C₁-C₆)-Alkyl, O-CO(C₁-C₆)-Alkyl; bevorzugt unabhängig voneinander H, OH, O-(C₁-C₆)-Alkyl; ganz besonders bevorzugt H;
R6, R7, R8, R9 H;
n 1
m 1;
A, B, D, G C(R38);
R38 H, F, Cl, Br, CF₃, CN, O-(C₁-C₆)-Alkyl, (C₁-C₆)-Alkyl, SO₂-CH₃, CON(R41)(R42), N(R43)CO(R44), CO(R47), -(CR48R49)ₓ-O(R50); besonders bevorzugt H, F, Cl, CF₃, CN, (C₁-C₆)-Alkyl, -(CR48R49)ₓ-O(R50);
R41, R42, R43 unabhängig voneinander H, (C₁-C₈)-Alkyl;
oder
R39 und R40, R41 und R42 unabhängig voneinander optional zusammen mit dem Stickstoffatom, an das sie gebunden sind, einen 5-6 gliedrigen Ring, welcher ausser dem Stickstoffatom noch 0-1 weitere Heteroatome aus der Gruppe NH, N-(C₁-C₆)-Alkyl, Sauerstoff und Schwefel beinhalten kann;
R47 H, (C₁-C₈)-Alkyl;
R48, R49 H;
R50 H, (C₁-C₆)-Alkyl;
x 1;
Het heteroaromatische Gruppe, ausgewählt aus der Gruppe bestehend aus Oxadiazolen, Thiadiazolen, Triazolen, Thiophenen, Furanen und Pyrrolen; ganz besonders bevorzugt ist Het ausgewählt aus Oxadiazolen und Thiadiazolen;
X eine Bindung, CH₂-CH₂, CH₂-Y, YCH₂, (R75)YCH₂, CH₂- NCO(R75), CH₂CON(R75), C(R76)(R77), C(R78)(R79)O, N(R75), C=C, C≡C; bevorzugt eine Bindung, CH₂-CH₂, C(R76)(R77), N(R75), CH₂Y, CH₂Y(R75), CH₂-NCO(R75), CH₂CON(R75), C=C; besonders bevorzugt eine Bindung, CH₂- CH₂, C(R76)(R77), C=C, (R75)YCH₂, CH₂-NCO(R75);
Y O, S, N(R53);
R53 H, (C₁-C₈)-Alkyl;
R75, R76, R77, R78, R79 unabhängig voneinander H, (C₁-C₈)-Alkyl;
E 5-7 gliedrige bivalente carbo- oder heterocyclische Ringstruktur mit 0-3 Heteroatomen aus der Gruppe N, O und S, die optional Substituenten aus der Gruppe H, F, Cl, Br, OH, CF₃, NO_{2,} CN, OCF₃, O-(C₁-C₆)-Alkyl, S-(C₁-C₆)-Alkyl, (C₁-C₆)-Alkyl, (C₂-C₆)- Alkenyl, O-(C₀-C₈)-Alkylen-Aryl, S-Aryl, N(R54)(R55), SO₂-CH₃, N(R58)CO(R59), CO(R62) tragen und mono- oder bicyclisch sein kann; bevorzugt 5-7 gliedrige bivalente carbo- oder heterocyclische Ringstruktur mit 0-2 Heteroatomen aus der Gruppe N, O und S, die optional Substituenten aus der Gruppe H, F, Cl, Br, OH, CF₃, NO₂, OCF₃, O-(C₁-C₆)-Alkyl, (C₁-C₆)-Alkyl, (C₂-C₆)-Alkenyl, N(R54)(R55), SO₂-CH₃, CO(R62) tragen kann, und die besonders bevorzugt monocyclisch ist; z.B. bedeutet E Benzol, Pyridin, Pyrimidin, Piperidin, Pyrrolidin, Cyclopentan, Cyclohexan, Piperazin, Homopiperazin, Thiazol, Thiophen, Furan, Pyrrol, Pyrazol, 1,2,3,6-Tetrahydro-pyridin, 4,5- Dihydroisoxazol, Oxazol;
R54, R55, R58 unabhängig voneinander H, (C₁-C₈)-Alkyl;
R59, R62 unabhängig voneinander H, (C₁-C₈)-Alkyl;
K O, eine Bindung, CH₂O_{,} OCH₂, N(R80), N(R81)CO, CON(R82), (C(R83)(R84))ᵥ, CO, C≡C, SCH₂; bevorzugt eine O, Bindung, CH₂O, OCH₂, CON(R82), (C(R83)(R84))ᵥ, CO, C≡C;
v 1, 2, 3; bevorzugt 1,2;
R80, R81, R83, R84 unabhängig voneinander H, (C₁-C₈)-Alkyl;
R11 (C₁-C₈)-Alkyl, (C₁-C₄)-Alkoxy-(C₁-C₄)-alkyl, ein 3 bis 10-gliedriger mono-, bi- oder spirocyclischer Ring, welcher 0 bis 3 Heteroatome beinhalten kann, ausgewählt aus der Gruppe Sauerstoff, Stickstoff und Schwefel, wobei das Ringsystem zusätzlich substituiert sein kann mit F, Cl, Br, CF₃, CN, (C₁-C₆)- Alkyl, O-(C₁-C₈)-Alkyl, (C₀-C₂)-Alkylen-aryl, Oxo, CO(R66), CON(R67)(R68), Hydroxy, N(R70)CO(C₁-C₆)-Alkyl, N(R71)(R72) oder SO₂CH₃; bevorzugt (C₁-C₈)-Alkyl, (C₁-C₄)-Alkoxy-(C₁-C₄)-alkyl, ein 3 bis 1 0-gliedriger mono-oder bicyclischer Ring, welcher 0 bis 2 Heteroatome beinhalten kann, ausgewählt aus der Gruppe Sauerstoff, Stickstoff und Schwefel, wobei das Ringsystem zusätzlich substituiert sein kann mit F, Cl, Br, CF₃, CN, (C₁-C₆)- Alkyl, O-(C₁-C₈)-Alkyl, Oxo, CO(R66), CON(R67)(R68), N(R70)CO(C₁-C₆)-Alkyl, oder SO₂CH₃;
R66, R67, R68, R70, R71, R72 unabhängig voneinander H, (C₁-C₈)-Alkyl;
oder
R67 und R68, R71 und R72 unabhängig voneinander optional zusammen mit dem Stickstoffatom, an das sie gebunden sind, einen 5-6 gliedrigen Ring, welcher ausser dem Stickstoffatom noch 0-1 weitere Heteroatome aus der Gruppe NH, N-(C₁-C₆)-Alkyl, Sauerstoff und Schwefel beinhalten kann; oder
deren N-Oxide sowie deren physiologisch verträgliche Salze.

5. Verbindungen der Formel I nach einem der Ansprüche 1 oder 2 bis 4
worin Reste R1, R2, R11, R38 und die Gruppen X, E und K die folgenden Bedeutungen aufweisen:
R1, R2 unabhängig voneinander H, (C₁-C₈)-Alkyl, -(CR13R14)ₒ -R12, (C₁-C₄)-Alkoxy-(C₁-C₄)-alkyl, oder R1 und R2 bilden zusammen mit dem Stickstoffatom, an das sie gebunden sind, einen 4 bis 10-gliedrigen mono-, bi- oder spirocyclischen Ring welcher ausser dem Stickstoffatom 0 bis 2 zusätzliche Heteroatome beinhalten kann, ausgewählt aus der Gruppe Sauerstoff, Stickstoff und Schwefel, wobei das heterocyclische Ringsystem zusätzlich substituiert sein kann mit (C₁-C₆)-Alkyl, O-(C₁-C₄)- Alkyl, (C₁-C₄)-Alkoxy-(C₁-C₄)-alkyl, Hydroxy-(C₁-C₄)-alkyl, (C₀- C₂)-Alkylen-aryl, Oxo, CO(R22), CON(R23)(R24), Hydroxy, N(R27)(R28) oder S0₂CH₃; bevorzugt unabhängig voneinander H, (C₁-C₈)-Alkyl, - (CR13R14)ₒ -R12, (C₁-C₄)-Alkoxy-(C₁-C₄)-alkyl, oder R1 und R2 bilden zusammen mit dem Stickstoffatom, an das sie gebunden sind, einen 4 bis 10-gliedrigen mono-, bi- oder spirocyclischen Ring welcher ausser dem Stickstoffatom 0 bis 2 zusätzliche Heteroatome beinhalten kann, ausgewählt aus der Gruppe Sauerstoff, Stickstoff und Schwefel, wobei das heterocyclische Ringsystem zusätzlich substituiert sein kann mit (C₁-C₆)-Alkyl, O-(C₁-C₄)-Alkyl, (C₁-C₄)-Alkoxy-(C₁-C₄)-alkyl, Hydroxy-(C₁-C₄)- alkyl, (C₀-C₂)-Alkylen-aryl, Oxo, CO(R22), CON(R23)(R24), Hydroxy oder N(R27)(R28); ganz besonders bevorzugt unabhängig voneinander H, (C₁-C₈)- Alkyl, oder R1 und R2 bilden zusammen mit dem Stickstoffatom, an das sie gebunden sind, einen 5 bis 6-gliedrigen monocyclischen Ring welcher ausser dem Stickstoffatom 0 bis 1 zusätzliche Heteroatome beinhalten kann, ausgewählt aus der Gruppe Sauerstoff, Stickstoff und Schwefel, wobei das heterocyclische Ringsystem zusätzlich substituiert sein kann mit (C₁-C₆)-Alkyl;
o 0, 1, 2, 3, 4;
R22, R23, R24, R27, R28 unabhängig voneinander H, (C₁-C₆)-Alkyl;
oder
R23 und R24, R27 und R28 unabhängig voneinander optional zusammen mit dem Stickstoffatom, an das sie gebunden sind, einen 5-6 gliedrigen Ring, welcher ausser dem Stickstoffatom noch 0-1 weitere Heteroatome aus der Gruppe NH, N-(C₁-C₆)-Alkyl, Sauerstoff und Schwefel beinhalten kann;
R12 OH, O-(C₁-C₆)-Alkyl, CN, 3-12 gliedriger mono-, bi- oder spirocyclischer Ring, der 1 bis 3 Heteroatome aus der Gruppe N, O und S enthalten kann und der 3-12 gliedrige Ring weitere Substituenten wie F, OH, CF₃, CN, Oxo, (C₁-C₆)-Alkyl, (C₀-C₂)- Alkylen-aryl, N(R34)(R35), COO(R36), CO(C₁-C₆)-Alkyl enthalten kann;
R34, R35 unabhängig voneinander H, (C₁-C₄)-Alkyl;
R36 H, (C₁-C₆)-Alkyl, (C₀-C₂)-Alkylen-aryl;
R13, R14 unabhängig voneinander H, (C₁-C₈)-Alkyl, Hydroxy-(C₁-C₄)-Alkyl, OH, (C₁-C₄)-Alkoxy-(C₁-C₄)-alkyl;
R38 H, F, Cl, Br, CF₃, CN, (C₁-C₆)-Alkyl;
X eine Bindung, CH₂CH₂, C(R76)(R77), N(R75), C=C, (R75)YCH₂, CH₂NCO(R75), CH₂CON(R75);
Y O, S, N(R53), CO
R75, R76, R77 unabhängig voneinander H, (C₁-C₈)-Alkyl;
R53 H, (C₁-C₈)-Alkyl;
E 5-7 gliedrige bivalente carbo- oder heterocyclische Ringstruktur mit 0-3 Heteroatomen aus der Gruppe N, O und S, die optional Substituenten aus der Gruppe H, F, Cl, Br, CF₃, OH, CN, OCF₃, O-(C₁-C₆)-Alkyl, (C₁-C₆)-Alkyl, SO₂-CH₃, CO(R65) tragen kann;
R65 H, (C₁-C₈)-Alkyl;
K O, eine Bindung, CH₂O, CH₂, OCH₂, S, SO₂, N(R80), N(R81)CO, CON(R82), (C(R83)(R84))ᵥ, CO, C≡C, SCH₂, SO₂CH₂; bevorzugt O, eine Bindung, CH₂O, CH₂, OCH₂, N(R80), C≡C;
v 1, 2, 3;
R80, R81, R82, R83, R84 unabhängig voneinander H, (C₁-C₈)-Alkyl;
R11 (C₁-C₈)-Alkyl, (C₁-C₄)-Alkoxy-(C₁-C₄)-alkyl, ein 3 bis 10-gliedriger mono-, bi- oder spirocyclischer Ring, welcher 0 bis 4 Heteroatome beinhalten kann, ausgewählt aus der Gruppe Sauerstoff, Stickstoff und Schwefel, wobei das Ringsystem zusätzlich substituiert sein kann mit F, Cl, Br, CF₃, CN, (C₁-C₆)- Alkyl, O-(C₁-C₈)-Alkyl, Oxo, CO(R66), Hydroxy, N(R70)CO(C₁- C₆)-Alkyl, oder SO₂CH₃;
R66, R70 unabhängig voneinander H, (C₁-C₈)-Alkyl;
deren N-Oxide sowie deren physiologisch verträgliche Salze.

6. Verbindungen der Formel I nach einem der Ansprüche 1 bis 5,
worin
A, B, D, G unabhängig voneinander N oder C(R38) bedeuten und die Gesamtzahl der Stickstoffatome in diesem Ring 0 oder 1 beträgt, wobei für den Fall, dass die Gesamtzahl der Stickstoffatome 1 ist, B N bedeutet.

7. Verbindungen der Formel I nach einem der Ansprüche 1 bis 6,
worin
n 1 und
m 1 oder 2 bedeuten.

8. Arzneimittel enthaltend eine oder mehrere der Verbindungen gemäß einem der Ansprüche 1 bis 7.

9. Arzneimittel enthaltend eine oder mehrere der Verbindungen gemäß einem der Ansprüche 1 bis 7 und einen oder mehrere anorektische Wirkstoffe.

10. Verbindungen der Formel I gemäß einem der Ansprüche 1 bis 7 zur Anwendung als Medikament zur Prophylaxe oder Behandlung der Obesitas.

11. Verbindungen der Formel I gemäß einem der Ansprüche 1 bis 7 zur Anwendung als Medikament zur Prophylaxe oder Behandlung des Typ II Diabetes.

12. Verbindungen der Formel I gemäß einem der Ansprüche 1 bis 7 in Kombination mit mindestens einem weiteren anorektischen Wirkstoff zur Anwendung als Medikament zur Prophylaxe oder Behandlung der Obesitas.

13. Verbindungen der Formel I gemäß einem der Ansprüche 1 bis 7 in Kombination mit mindestens einem weiteren anorektischen Wirkstoff zur Anwendung als Medikament zur Prophylaxe oder Behandlung der des Typ II Diabetes.

14. Verfahren zur Herstellung eines Arzneimittels enthaltend eine oder mehrere der Verbindungen der Formel I gemäß einem der Ansprüche 1 bis 7, **dadurch gekennzeichnet, dass** der Wirkstoff mit einem pharmazeutisch geeigneten Träger vermischt wird und diese Mischung in eine für die Verabreichung geeignete Form gebracht wird.

15. Verwendung der Verbindungen der Formel I gemäß einem der Ansprüche 1 bis 7 zur Herstellung eines Medikaments zur Gewichtsreduktion bei Säugetieren.

16. Verwendung der Verbindungen der Formel I gemäß einem der Ansprüche 1 bis 7 zur Herstellung eines Medikaments zur Prophylaxe oder Behandlung der Obesitas.

17. Verwendung der Verbindungen der Formel I gemäß einem der Ansprüche 1 bis 7 zur Herstellung eines Medikaments zur Prophylaxe oder Behandlung des Typ II Diabetes.

## Claims

1. A compound of the formula I, 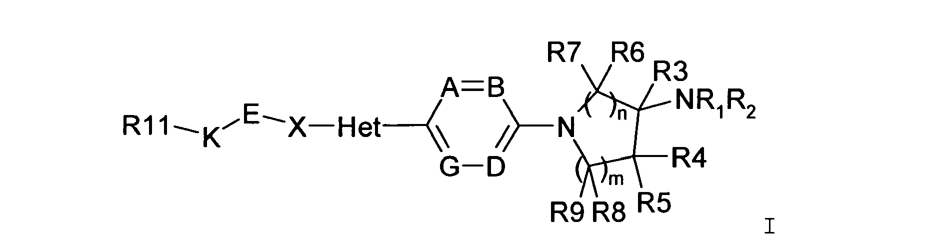 in which the meanings are
R1, R2 independently of one another H, (C₁-C₈)- alkyl, -(CR13R14)ₒ-R12, (C₁-C₄)-alkoxy- (C₁-C₄)-alkyl, (C₃-C₈)-alkenyl, (C₃-C₈)- alkynyl, CO-(C₁-C₈)-alkyl, -CO-(CH₂)ₒ-R12, CO (C (R15) (R16))_{q}N(R17) (R18), CO(C (R19) (R20))ₛO (R21); or R1 and R2 form together with the nitrogen atom to which they are bonded a 4 to 10-membered mono-, bi- or spirocyclic ring which, apart from the nitrogen atom, may include 0 to 4 additional heteroatoms selected from the group of oxygen, nitrogen and sulfur, where the heterocyclic ring system may additionally be substituted by F, Cl,
o Br, CF₃, NO₂, CN, (C₁-C₆)-alkyl, O-(C₁- C₈)-alkyl, (C₁-C₄)-alkoxy-(C₁-C₄)-alkyl, hydroxy-(C₁-C₄)-alkyl, (C₀-C₈)-alkylene- aryl, oxo, CO(R22), CON(R23)(R24), hydroxy, COO(R25), N(R26)CO(C₁-C₆)-alkyl, N(R27)(R28) or SO₂CH₃; 0, 1, 2, 3, 4, 5, 6;
q, s independently of one another 0, 1, 2, 3, 4;
R15, R16, R17, R18, R19, R20, R21, R22, R23, R24, R25, R26, R27, R28 independently of one another H, (C₁-C₆)- alkyl;
R17 and R18, R23 and R24, R27 and R28 independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
R12 OH, O-(C₁-C₆)-alkyl, CN, COO(R29), CON(R30)(R31), N(R32)(R33), 3-12 membered mono-, bi- or spirocyclic ring which may comprise one or more heteroatoms from the group of N, O and S, and the 3-12 membered ring may comprise further substituents such as F, Cl, Br, I, OH, CF₃, NO₂, CN, OCF₃, oxo, O- (C₁-C₆)-alkyl, (C₁-C₄)-alkoxy-(C₁-C₄)- alkyl, S-(C₁-C₆)-alkyl, (C₁-C₆)-alkyl, (C₂-C₆)-alkenyl, (C₃-C₈)-cycloalkyl, (C₂- C₆)-alkynyl, O-(C₀-C₈)-alkylene-aryl, (C₀- C₈)-alkylene-aryl, N(R34)(R35), CO(C₁- C₆)-alkyl, COO(R36) and S(O)ᵤ (R37);
u 0, 1, 2;
R34, R35 independently of one another H, (C₁-C₈)- alkyl;
R34 and R35 optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur, and optionally be substituted by 1-2 oxo groups;
R36, R37 H, (C₁-C₈)-alkyl;
R13, R14 independently of one another H, (C₁-C₈)- alkyl, hydroxy-(C₁-C₄)-alkyl, OH, (C₁-C₄)- alkoxy-(C₁-C₄)-alkyl;
R29, R30, R31 independently of one another H, (C₁-C₈)- alkyl, (C₂-C₆)-alkenyl, (C₀-C₈)-alkylene- aryl;
R32, R33 independently of one another H, (C₁-C₆)- alkyl
or
R32 and R33 optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur and optionally be substituted by 1-2 oxo groups;
R3 H, (C₁-C₆)-alkyl;
R4, R5 independently of one another H, (C₁-C₆)- alkyl, OH, O-(C₁-C₆)-alkyl, O-CO(C₁-C₆)- alkyl, S-(C₁-C₆)-alkyl;
R6, R7, R8, R9 independently of one another H, (C₁-C₈)- alkyl,
or
R6 and R7, R8 and R9 independently of one another optionally oxo;
n, m independently of one another 0, 1, 2;
A, B, D, G independently of one another N, C(R38), and the total number of nitrogen atoms in this ring is 0 or 1, and for the case where the total number of nitrogen atoms is 1, A or B is N;
R38 H, F, Cl, Br, I, OH, CF₃, NO₂, CN, OCF₃, O-(C₁-C₆)-alkyl, O-(C₁-C₄)-alkoxy-(C₁-C₄)- alkyl, S-(C₁-C₆)-alkyl, (C₁-C₆)-alkyl, (C₂-C₆)-alkenyl, (C₃-C₈)-cycloalkyl, O- (C₃-C₈)-cycloalkyl, (C₃-C₈)-cycloalkenyl, O- (C₃-C₈)-cycloalkenyl, (C₂-C₆)-alkynyl, (C₀-C₈)-alkylene-aryl, O-(C₀-C₈)-alkylene- aryl, S-aryl, N(R39)(R40), SO₂-CH₃, COOH, COO-(C₁-C₆)-alkyl, CON(R41)(R42), N(R43)CO(R44), N(R45)SO₂(R46), CO(R47), - (CR48R49)ₓ-O(R50);
R39, R40, R41, R42, R43, R45 independently of one another H, (C₁-C₈)- alkyl;
or
R39 and R40, R41 and R42 independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
R44, R46, R47 independently of one another H, (C₁-C₈)- alkyl, aryl;
R48, R49 independently of one another H, (C₁-C₈)- alkyl;
R50 H, (C₁-C₆)-alkyl;
x 1, 2, 3, 4;
Het is selected from the group consisting of oxadiazoles, thiadiazoles, triazoles, thiophenes, furans and pyrroles;
X a bond, a group of the formula - (CR51R52)y- in which one or more - (CR51R52)- groups may be replaced by Y to result in a chemically reasonable radical, C=C, C=C;
Y O, S, N(R53), CO, SO, SO₂;
R51, R52 independently of one another H, (C₁-C₄)- alkyl, where R51 and R52 in the y groups may in each case have the same or different meanings;
y 1, 2, 3, 4, 5, 6;
R53 H, (C₁-C₈)-alkyl;
E 3-14 membered bivalent carbo- or heterocyclic ring structure having 0-4 heteroatoms from the group of N, 0 and S, which may optionally have substituents from the group of H, F, Cl, Br, I, OH, CF₃, NO₂, CN, OCF₃, oxo, O-(C₁- C₆)-alkyl, O-(C₁-C₄)-alkoxy-(C₁-C₄)-alkyl, S-(C₁-C₆)-alkyl, (C₁-C₆)-alkyl, (C₂-C₆)- alkenyl, (C₃-C₈)-cycloalkyl, O-(C₃-C₈)- cycloalkyl, (C₂-C₆)-alkynyl, (C₀-C₈)- alkylene-aryl, O-(C₀-C₈)-alkylene-aryl, S-aryl, N(R54)(R55), SO₂-CH₃, COOH, COO- (C₁-C₆)-alkyl, CON(R56)(R57), N(R58)CO(R59), N(R60)S02(R61), CO(R62) and be mono- or bicyclic;
R54, R55, R56, R57, R58, R60 independently of one another H, (C₁-C₈)- alkyl;
R54 and R55, R56 and R57 independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
R59, R61, R62 independently of one another H, (C₁-C₈)- alkyl, aryl;
K a bond, a group of the formula - (CR63R64)_{z}- in which one or more - (CR63R64)- groups may be replaced by Z to result in a chemically reasonable radical, C≡C, C=C;
Z 0, S, N(R65), CO, SO, SO₂;
R63, R64 independently of one another H, (C₁-C₈)- alkyl, hydroxy, (C₁-C₆)-alkoxy, where R63 and R64 in the z groups may in each case have the same or different meanings;
z 1, 2, 3, 4, 5, 6;
R65 H, (C₁-C₈)-alkyl;
R11 H, (C₁-C₈)-alkyl, (C₁-C₄)-alkoxy-(C₁-C₄)- alkyl, (C₃-C₈)-alkenyl, (C₃-C₈)-alkynyl, a 3 to 10-membered mono-, bi- or spirocyclic ring which may include 0 to 4 heteroatoms selected from the group of oxygen, nitrogen and sulfur, where the ring system may additionally be substituted by F, Cl, Br, CF₃, NO₂, CN, (C₁-C₆)-alkyl, O-(C₁-C₈)-alkyl, (C₁-C₄)- alkoxy- (C₁-C₄)-alkyl, (C₀-C₈)-alkylene- aryl, oxo, CO(R66), CON(R67)(R68), hydroxy, hydroxy-(C₁-C₄)-alkyl, COO(R69), N(R70)CO(C₁-C₆)-alkyl, N(R71)(R72) or SO₂CH₃;
R66, R67, R68, R69, R70, R71, R72 independently of one another H, (C₁-C₈)- alkyl;
or
R67 and R68, R71 and R72 independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur; or
the N-oxides thereof and the physiologically tolerated salts thereof.

2. A compound of the formula I as claimed in claim 1,
in which the meanings are
R1, R2 independently of one another H, (C₁-C₈)- alkyl, -(CR13R14)ₒ-R12, (C₁-C₄)-alkoxy- (C₁-C₄)-alkyl, (C₃-C₈)-alkenyl, (C₃-C₈)- alkynyl, CO-(C₁-C₈)-alkyl, -CO-(CH₂)ₒ-R12, CO(C(R15)(R16))_{q}N(R17)(R18), CO(C(R19)(R20))ₛO(R21); or R1 and R2 form together with the nitrogen atom to which they are bonded a 4 to 10-membered mono-, bi- or spirocyclic ring which, apart from the nitrogen atom, may include 0 to 4 additional heteroatoms selected from the group of oxygen, nitrogen and sulfur, where the heterocyclic ring system may additionally be substituted by F, Cl, Br, CF₃, NO₂, CN, (C₁-C₆)-alkyl, O-(C₁-C₄)-alkyl, (C₁-C₄)-alkoxy-(C₁-C₄)- alkyl, hydroxy-(C₁-C₄)-alkyl, (C₀-C₂)- alkylene-aryl, oxo, CO(R22), CON(R23)(R24), hydroxy, COO(R25), N(R26)CO(C₁-C₆)-alkyl or N(R27)(R28);
R12 OH, O-(C₁-C₆)-alkyl, CN, COO(R29), CON(R30)(R31), N(R32)(R33), 3-12 membered mono-, bi- or spirocyclic ring which may comprise one or more heteroatoms from the group of N, 0 and S, and the 3-12 membered ring may comprise further substituents such as F, Cl, Br, I, OH, CF₃, NO₂, CN, OCF₃, oxo, O-(C₁-C₆)-alkyl, (C₁-C₄)-alkoxy-(C₁-C₄)- alkyl, S-(C₁-C₆)-alkyl, (C₁-C₆)-alkyl, (C₂-C₆)-alkenyl, (C₃-C₈)-cycloalkyl, (C₂- C₆)-alkynyl, O-(C₀-C₈)-alkylene-aryl, (C₀- C₈)-alkylene-aryl, N(R34)(R35), CO(C₁- C₆)-alkyl and COO(R36); and
R6, R7, R8, R9 independently of one another H, (C₁-C₈)- alkyl;
where the further radicals and groups in the compound of the formula I have the meanings stated in claim 1.

3. A compound of the formula I as claimed in claim 1, in which the meanings are
R1, R2 independently of one another H, (C₁-C₈)- alkyl, -(CR13R14)ₒ -R12, (C₁-C₄)-alkoxy- (C₁-C₄)-alkyl, (C₃-C₈)-alkenyl, CO-(C₁- C₈)-alkyl, -CO-(CH₂)ₒ -R12, CO(C(R15) (R16))_{q}N(R17) (R18), CO(C(R19) (R20))ₛO(R21); or R1 and R2 form together with the nitrogen atom to which they are bonded a 4 to 10-membered mono-, bi- or spirocyclic ring which, apart from the nitrogen atom, may include 0 to 2 additional heteroatoms selected from the group of oxygen, nitrogen and sulfur, where the heterocyclic ring system may additionally be substituted by F, Cl, CF₃, (C₁-C₆)-alkyl, O-(C₁-C₄)-alkyl, (C₁- C₄)-alkoxy-(C₁-C₄)-alkyl, hydroxy-(C₁-C₄)- alkyl, (C₀-C₂)-alkylene-aryl, oxo, CO(R22), CON(R23)(R24), hydroxy, COO(R25), N(R26)CO(C₁-C₆)-alkyl, N(R27) (R28) or SO₂CH₃;
o 0, 1, 2, 3, 4, 5, 6;
q 1, 2, 3;
s 0, 1, 2, 3, 4;
R15, R16, R17, R18, R19, R20, R21, R22, R23, R24, R25, R26, R27, R28 independently of one another H, (C₁-C₆)- alkyl;
or
R17 and R18, R23 and R24, R27 and R28 independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
R12 OH, O-(C₁-C₆)-alkyl, CN, COO(R29), CON(R30)(R31), 3-12 membered mono-, bi- or spirocyclic ring which may comprise one or more heteroatoms from the group of N, O and S, and the 3-12 membered ring may comprise further substituents such as F, Cl, Br, OH, CF₃, CN, oxo, O- (_{C1}-_{C6})-alkyl, (C₁-C₄)-alkoxy-(C₁-C₄)- alkyl, (C₁-C₆)-alkyl, O-(C₀-C₈)-alkylene-
u aryl, (C₀-C₈)-alkylene-aryl, N(R34)(R35), CO(C₁-C₆)-alkyl, COO(R36), S(O)ᵤ₍R37); 0, 1, 2;
R34, R35 independently of one another H, (C₁-C₈)- alkyl;
or
R34 and R35 optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur, and optionally be substituted by 1-2 oxo groups;
R36, R37 H, (C₁-C₈)-alkyl;
R13, R14 independently of one another H, (C₁-C₈)- alkyl, hydroxy-(C₁-C₄)-alkyl, OH, (C₁-C₄)- alkoxy-(C₁-C₄)-alkyl;
R29, R30, R31 independently of one another H, (C₁-C₈)- alkyl;
R3 H, (C₁-C₆)-alkyl;
R4, R5 independently of one another H, (C₁-C₆)- alkyl, OH, O-(C₁-C₆)-alkyl, O-CO(C₁-C₆)- alkyl, S-(C₁-C₆)-alkyl;
R6, R7, R8, R9 H;
or
R6 and R7, R8 and R9 independently of one another optionally oxo;
n 1
m 1 or 2;
A, B, D, G independently of one another N, C(R38), and the total number of nitrogen atoms in this ring is 0 or 1, and for the case where the total number of nitrogen atoms is 1, A or B is N; or the groups A and B or D and G are in each case C(R38) and together form an ortho-phenylene unit so that the overall result is a 1,4-bisubstituted naphthalene system;
R38 H, F, Cl, Br, CF₃, CN, O-(C₁-C₆)-alkyl, O-(C₁-C₄)-alkoxy-(C₁-C₄)-alkyl, S-(C₁-C₆)- alkyl, (C₁-C₆)-alkyl, (C₀-C₈)-alkylene- aryl, O-(C₀-C₈)-alkylene-aryl, N(R39)(R40), SO₂-CH₃, CON(R41)(R42), N(R43)CO(R44), CO(R47), -(CR48R49)ₓ- O(R50);
R39, R40, R41, R42, R43 independently of one another H, (C₁-C₈)- alkyl;
or
R39 and R40, R41 and R42 independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
R44, R47 independently of one another H, (C₁-C₈)- alkyl, aryl;
R48, R49 H;
R50 H, (C₁-C₆)-alkyl;
x 1, 2;
Het is selected from the group consisting of oxidiazoles, thiadiazoles, triazoles, thiophenes, furans and pyrroles;
X a bond, a group of the formula - (CR51R52)_{y}-, in which one or more - (CR51R52)- groups may be replaced by Y to result in a chemically reasonable radical, C=C, C=C;
R51, R52 independently of one another H, (C₁-C₄)- alkyl, where R51 and R52 in the y groups may in each case have the same or different meanings;
Y O, S, N(R53), CO;
R53 H, (C₁-C₈)-alkyl;
E 3-8 membered bivalent carbo- or heterocyclic ring structure having 0-4 heteroatoms from the group of N, O and S, which may optionally have substituents from the group of H, F, Cl, Br, OH, CF₃, NO₂, CN, OCF₃, O-(C₁-C₆)- alkyl, O-(C₁-C₄)-alkoxy-(C₁-C₄)-alkyl, S- (C₁-C₆)-alkyl, (C₁-C₆)-alkyl, (C₂-C₆)- alkenyl, O-(C₃-C₈)-cycloalkyl, (C₂-C₆)- alkynyl, (C₀-C₈)-alkylene-aryl, O-(C₀-C₈)- alkylene-aryl, S-aryl, N(R54)(R55), SO₂- CH₃, N(R58)CO(R59), N(R60)SO₂(R61), CO(R62) and be mono- or bicyclic;
R54, R55, R58, R60 independently of one another H, (C₁-C₈)- alkyl;
R59, R61, R62 independently of one another H, (C₁-C₈)- alkyl, aryl;
K 0, a bond, CH₂O, OCH₂, S, SO, SO₂, N(R80), N(R81)CO, CON(R82), (C(R83)(R84))ᵥ, CO, C=C, C≡C, SCH₂, SO₂CH₂;
v 1, 2, 3, 4;
R80, R81, R82, R83, R84 independently of one another H, (C₁-C₈)- alkyl;
R11 H, (C₁-C₈)-alkyl, (C₁-C₄)-alkoxy-(C₁-C₄)- alkyl, (C₃-C₈)-alkenyl, (C₃-C₈)-alkynyl, a 3 to 10-membered mono-, bi- or spirocyclic ring which may include 0 to 4 heteroatoms selected from the group of oxygen, nitrogen and sulfur, where the ring system may additionally be substituted by F, Cl, Br, CF₃, CN, (C₁- C₆)-alkyl, O-(C₁-C₈)-alkyl, (C₁-C₄)- alkoxy-(C₁-C₄)-alkyl, hydroxy-(C₁-C₄- alkyl, (C₀-C₈)-alkylene-aryl, oxo, CO(R66), CON(R67)(R68), hydroxy, COO(R69), N(R70)CO(C₁-C₆)-alkyl, N(R71)(R72) or SO₂CH₃;
R66, R67, R68, R69, R70, R71, R72 independently of one another H, (C₁-C₈)- alkyl;
or
R67 and R68, R71 and R72 independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur; or
the N-oxides thereof and the physiologically tolerated salts thereof.

4. A compound of the formula I as claimed in claim 1 or 3,
in which the meanings are:
R1, R2 independently of one another H, (C₁-C₈)- alkyl, -(CR13R14)ₒ-R12, (C₁-C₄)-alkoxy-(C₁-C₄)- alkyl, CO- (C₁-C₈)-alkyl, -CO-(CH₂)ₒ-R12, CO(C(R15)(R16))_{q}N(R17)(R18), CO(C(R19)(R20))ₛO(R21); or R1 and R2 form together with the nitrogen atom to which they are bonded a 4 to 10-membered mono- or bicyclic ring which, apart from the nitrogen atom, may include 0 to 2 additional heteroatoms selected from the group of oxygen, nitrogen and sulfur, where the heterocyclic ring system may additionally be substituted by F, Cl, CF₃, (C₁-C₆)-alkyl, O-(C₁-C₄)-alkyl, (C₁- C₄)-alkoxy-(C₁-C₄)-alkyl, (C₀-C₂)- alkylene-aryl, oxo, CO(R22), hydroxy, N(R27)(R28) or SO₂CH₃; preferably independently of one another H, (C₁-C₈)-alkyl, -(CR13R14)ₒ-R12, (C₁- C₄)-alkoxy-(C₁-C₄)-alkyl, CO-(C₁-C₈)- alkyl, -CO-(CH₂)ₒ-R12, CO(C(R15)(R16))_{q}N(R17) (R18), or R1 and R2 form together with the nitrogen atom to which they are bonded a 4 to 10-membered mono- or bicyclic ring which, apart from the nitrogen atom, may include 0 to 2 additional heteroatoms selected from the group of oxygen and nitrogen, where the heterocyclic ring system may additionally be substituted by F, (C₁- C₆)-alkyl, (C₁-C₄)-alkoxy-(C₁-C₄)-alkyl, oxo, CO(R22), hydroxy, N(R27)(R28);
O 0, 1, 2, 3, 4; preferably 0, 1, 2, 3;
q 1 or 2;
s 0, 1, 2, 3; preferably 0, 1, 2;
R15, R16, R17, R18, R19, R20, R21, R22, R27, R28 independently of one another H, (C₁-C₆)- alkyl;
or
R17 and R18, R27 and R28 independently of one another optionally together with the nitrogen atom to which they are bonded a 5-6 membered ring selected from pyrrolidine, piperidine, N-methylpiperazine and morpholine;
R12 OH, O-(C₁-C₆)-alkyl, CN, 3-10 membered mono- or bicyclic ring which may comprise 1-3 heteroatoms from the group of N, O and S, and the 3-10 membered ring may comprise further substituents such as F, Cl, Br, OH, CF₃, CN, oxo, O- (C₁-C₆)-alkyl, (C₁-C₄)-alkoxy-(C₁-C₄)- alkyl, (C₁-C₆)-alkyl, (C₀-C₂)-alkylene- aryl, N(R34)(R35), CO(C₁-C₆)-alkyl; preferably OH, O-(C₁-C₆)-alkyl, 3-10 membered mono- or bicyclic ring which may comprise 1-2 heteroatoms from the group of N, O and S, and the 3-10 membered ring may comprise further substituents such as F, OH, oxo, (C₁-C₆)- alkyl, CO(C₁-C₆)-alkyl;
u 0 or 2; preferably 2;
R34, R35 independently of one another H, (C₁-C₈)- alkyl;
or
R34 and R35 optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur, and optionally be substituted by 1-2 oxo groups;
R36, R37 H, (C₁-C₈)-alkyl;
R13, R14 independently of one another H, (C₁-C₈)- alkyl, hydroxy-(C₁-C₄)-alkyl, OH, (C₁-C₄)- alkoxy-(C₁-C₄)-alkyl;
R3 H;
R4, R5 independently of one another H, (C₁-C₆)- alkyl, OH, O-(C₁-C₆)-alkyl, O-CO(C₁-C₆)- alkyl; preferably independently of one another H, OH, O-(C₁-C₆)-alkyl, very particularly preferably H;
R6, R7, R8, R9 H;
n 1
m 1;
A, B, D, G C(R38);
R38 H, F, Cl, Br, CF₃, CN, O-(C₁-C₆)-alkyl, (C₁-C₆)-alkyl, SO₂-CH₃, CON(R41)(R42), N(R43)CO(R44), CO(R47), -(CR48R49)ₓ-O(R50); particularly preferably H, F, Cl, CF₃, CN, (C₁-C₆)-alkyl, -(CR48R49)ₓ-O(R50);
R41, R42, R43 independently of one another H, (C₁-C₈)- alkyl;
or
R39 and R40, R41 and R42 independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
R47 H, (C₁-C₈)-alkyl;
R48, R49 H;
R50 H, (C₁-C₆)-alkyl;
x 1;
Het heteroaromatic group selected from the group consisting of oxadiazoles, thiadiazoles, triazoles, thiophenes, furans and pyrroles; Het is very particularly preferably selected from oxadiazoles and thiadiazoles;
X a bond, CH₂-CH₂, CH₂Y, YCH₂, (R75)YCH₂, CH₂-NCO(R75), CH₂CON(R75); C(R76)(R77), C(R78)(R79)O, N(R75), C=C, C≡C; preferably a bond, CH₂-CH₂, C(R76)(R77), N(R75), CH₂Y, CH₂Y(R75), CH₂-NCO(R75), CH₂-CON(R75); C=C; particularly preferably a bond, CH₂-CH₂, C(R76)(R77), C=C, (R75)YCH₂, CH₂-NCO(R75);
Y O, S, N(R53);
R53 H, (C₁-C₈)-alkyl;
R75, R76, R77, R78, R79 independently of one another H, (C₁-C₈)- alkyl;
E 5-7 membered bivalent carbo- or heterocyclic ring structure having 0-3 heteroatoms from the group of N, O and S, which may optionally have substituents from the group of H, F, Cl, Br, OH, CF₃, NO₂, CN, OCF₃, O-(C₁-C₆)- alkyl, S-(C₁-C₆)-alkyl, (C₁-C₆)-alkyl, (C₂-C₆)-alkenyl, O-(C₀-C₈)-alkylene-aryl, S-aryl, N(R54)(R55), SO₂-CH₃, N(R58)CO(R59), CO(R62) and be mono- or bicyclic; preferably 5-7 membered bivalent carbo- or heterocyclic ring structure having O- 2 heteroatoms from the group of N, O and S, which may optionally have substituents from the group of H, F, Cl, Br, OH, CF₃, NO₂, OCF₃, O-(C₁-C₆)-alkyl, (C₁-C₆)-alkyl, (C₂-C₆)-alkenyl, N(R54)(R55), SO₂-CH₃, CO(R62), and which is particularly preferably monocyclic; e.g. E is benzene, pyridine, pyrimidine, piperidine, pyrrolidine, cyclopentane, cyclohexane, piperazine, homopiperazine, thiazole, thiophene, furan, pyrrole, pyrazole, 1,2,3,6-tetrahydropyridine, 4,5-dihydroisoxazole, oxazole;
R54, R55, R58 independently of one another H, (C₁-C₈)- alkyl;
R59, R62 independently of one another H, (C₁-C₈)- alkyl;
K O, a bond, CH₂O, OCH₂, N(R80), N(R81)CO, CON(R82), (C(R83)(R84))ᵥ, CO, C=C, SCH₂; preferably O, a bond, CH₂O, OCH₂, CON(R82), (C(R83)(R84))ᵥ, CO, C=C;
v 1, 2, 3; preferably 1,2;
R80, R81, R83, R84 independently of one another H, (C₁-C₈)- alkyl;
R11 (C₁-C₈)-alkyl, (C₁-C₄)-alkoxy- (C₁-C₄)- alkyl, a 3 to 10-membered mono-, bi- or spirocyclic ring which may include 0 to 3 heteroatoms selected from the group of oxygen, nitrogen and sulfur, where the
R66, R67, ring system may additionally be substituted by F, Cl, Br, CF₃, CN, (C₁- C₆)-alkyl, O-(C₁-C₈)-alkyl, (C₀-C₂)- alkylene-aryl, oxo, CO(R66), CON(R67)(R68), hydroxy, N(R70)CO(C₁-C₆)- alkyl, N(R71)(R72) or SO₂CH₃; preferably (C₁-C₈)-alkyl, (C₁-C₄)-alkoxy- (C₁-C₄)-alkyl, a 3 to 10-membered mono- or bicyclic ring which may include 0 to 2 heteroatoms selected from the group of oxygen, nitrogen and sulfur, where the ring system may additionally be substituted by F, Cl, Br, CF₃, CN, (C₁- C₆)-alkyl, O-(C₁-C₈)-alkyl, oxo, CO(R66), CON(R67)(R68), N(R70)CO(C₁-C₆)-alkyl, or SO₂CH₃; R68, R70, R71, R72 independently of one another H, (C₁-C₈)- alkyl;
or
R67 and R68, R71 and R72 independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur; or
the N-oxides thereof and the physiologically tolerated salts thereof.

5. A compound of the formula I as claimed in any of claims 1 or 2 to 4,
in which radicals R1, R2, R11, R38 and the groups X, E and K have the following meanings:
R1, R2 independently of one another H, (C₁-C₈)- alkyl, -(CR13R14)ₒ-R12, (C₁-C₄)-alkoxy- (C₁-C₄)-alkyl, or R1 and R2 form together with the nitrogen atom to which they are bonded a 4 to 10-membered mono-, bi- or spirocyclic ring which, apart from the nitrogen atom, may include 0 to 2 additional heteroatoms selected from the group of oxygen, nitrogen and sulfur, where the heterocyclic ring system may additionally be substituted by (C₁-C₆)- alkyl, O-(C₁-C₄)-alkyl, (C₁-C₄)-alkoxy- (C₁-C₄)-alkyl, hydroxy-(C₁-C₄)-alkyl, (C₀- C₂)-alkylene-aryl, oxo, CO(R22), CON(R23)(R24), hydroxy, N(R27)(R28) or SO₂CH₃; preferably independently of one another H, (C₁-C₈)-alkyl, -(CR13R14)ₒ-, R12, (C₁- C₄)-alkoxy-(C₁-C₄)-alkyl, or R1 and R2 form together with the nitrogen atom to which they are bonded a 4 to 10-membered mono-, bi- or spirocyclic ring which, apart from the nitrogen atom, may include 0 to 2 additional heteroatoms selected from the group of oxygen, nitrogen and sulfur, where the heterocyclic ring system may additionally be substituted by (C₁-C₆)- alkyl, O-(C₁-C₄)-alkyl, (C₁-C₄)-alkoxy- (C₁-C₄)-alkyl, hydroxy-(C₁-C₄)-alkyl, (C₀-
O C₂)-alkylene-aryl, oxo, CO(R22), CON(R23)(R24), hydroxy or N(R27)(R28); very particularly preferably independently of one another H, (C₁-C₈)- alkyl, or R1 and R2 form together with the nitrogen atom to which they are bonded a 5 to 6-membered monocyclic ring which, apart from the nitrogen atom, may include 0 to 1 additional heteroatoms selected from the group of oxygen, nitrogen and sulfur, where the heterocyclic ring system may additionally be substituted by (C₁-C₆)- alkyl; 0, 1, 2, 3, 4;
R22, R23, R24, R27, R28 independently of one another H, (C₁-C₆)- alkyl;
or
R23 and R24, R27 and R28 independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
R12 OH, O-(C₁-C₆)-alkyl, CN 3-12 membered mono-, bi- or spirocyclic ring which may comprise 1 to 3 heteroatoms from the group of N, O and S, and the 3-12 membered ring may comprise further
R34, R35 substituents such as F, OH, CF₃, CN, oxo, (C₁-C₆)-alkyl, (C₀-C₂)-alkylene-aryl, N(R34)(R35), COO(R36), CO(C₁-C₆)-alkyl; independently of one another H, (C₁-C₄)- alkyl;
R36 H, (C₁-C₆)-alkyl, (C₀-C₂)-alkylene-aryl;
R13, R14 independently of one another H, (C₁-C₈)- alkyl, hydroxy- (C₁-C₄) -alkyl, OH, (C₁-C₄)- alkoxy-(C₁-C₄)-alkyl;
R38 H, F, Cl, Br, CF₃, CN, (C₁-C₆)-alkyl;
X a bond, CH₂CH₂, C(R76)(R77), N(R75), C=C, (R75)YCH₂, CH₂-NCO(R75), CH₂CON(R75);
Y O, S, N(R53), CO
R75, R76, R77 independently of one another H, (C₁-C₈)- alkyl;
R53 H, (C₁-C₈)-alkyl;
E 5-7 membered bivalent carbo- or heterocyclic ring structure having 0-3 heteroatoms from the group of N, O and S, which may optionally have substituents from the group of H, F, Cl, Br, CF₃, OH, CN, OCF₃, O-(C₁-C₆)-alkyl, (C₁-C₆)-alkyl, SO₂-CH₃, CO(R65);
R65 H, (C₁-C₈)-alkyl;
K O, a bond, CH₂O, CH₂, OCH₂, S, SO₂, N(R80), N(R81)CO, CON(R82), (C(R83)(R84))ᵥ, CO, C=C, SCH₂, SO₂CH₂; preferably 0, a bond, CH₂O, CH₂, OCH₂, N(R80), C=C;
v 1, 2, 3;
R80, R81, R82, R83, R84 independently of one another H, (C₁-C₈)- alkyl;
R11 (C₁-C₈)-alkyl, (C₁-C₄)-alkoxy-(C₁-C₄)- alkyl, a 3 to 10-membered mono-, bi- or spirocyclic ring which may include 0 to 4 heteroatoms selected from the group of oxygen, nitrogen and sulfur, where the ring system may additionally be substituted by F, Cl, Br, CF₃, CN, (C₁- C₆)-alkyl, O-(C₁-C₈)-alkyl, oxo, CO(R66), hydroxy, N(R70)CO(C₁-C₆)-alkyl, or SO₂CH₃;
R66, R70 independently of one another H, (C₁-C₈)- alkyl;
the N-oxides thereof and the physiologically tolerated salts thereof.

6. A compound of the formula I as claimed in any of claims 1 to 5,
in which
A, B, D, G are independently of one another N or C(R38), and the total number of nitrogen atoms in this ring is 0 or 1, and for the case where the total number of nitrogen atoms is 1, B is N.

7. A compound of the formula I as claimed in any of claims 1 to 6,
in which
n is 1 and
m is 1 or 2.

8. A medicament comprising one or more of the compounds as claimed in any of claims 1 to 7.

9. A medicament comprising one or more of the compounds as claimed in any of claims 1 to 7 and one or more anorectic active ingredients.

10. A compound of the formula I as claimed in any of claims 1 to 7 for use as medicament for the prophylaxis or treatment of obesity.

11. A compound of the formula I as claimed in any of claims 1 to 7 for use as medicament for the prophylaxis or treatment of type II diabetes.

12. A compound of the formula I as claimed in any of claims 1 to 7 in combination with at least one further anorectic active ingredient for use as medicament for the prophylaxis or treatment of obesity.

13. A compound of the formula I as claimed in any of claims 1 to 7 in combination with at least one further anorectic active ingredient for use as medicament for the prophylaxis or treatment of type II diabetes.

14. A process for producing a medicament comprising one or more of the compounds of the formula I as claimed in any of claims 1 to 7, which comprises mixing the active ingredient with a pharmaceutically suitable carrier, and converting this mixture into a form suitable for administration.

15. The use of the compounds of the formula I as claimed in any of claims 1 to 7 for producing a medicament for weight reduction in mammals.

16. The use of the compounds of the formula I as claimed in any of claims 1 to 7 for producing a medicament for the prophylaxis or treatment of obesity.

17. The use of the compounds of the formula I as claimed in any of claims 1 to 7 for producing a medicament for the prophylaxis or treatment of type II diabetes.

## Revendications

1. Composés de formule I, 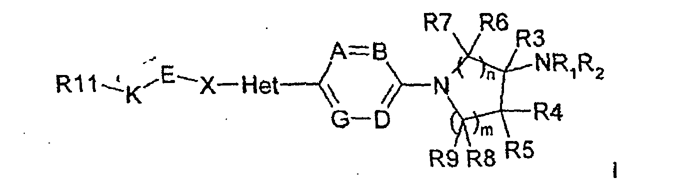 dans laquelle
R1, R2 signifient, indépendamment l'un de l'autre, H, (C₁-C₈)-alkyle, -(CR13R14)ₒ-R12, (C₁-C₄)-alcoxy-(C₁- C₄)-alkyle, (C₃-C₈)-alcényle, (C₃-C₈)-alcynyle, CO- (C₁-C₈)-alkyle, -CO-(CH₂)ₒ-R12, CO(C(R15)(R16))_{q}N(R17)(R18), CO(C(R19)(R20))ₛO(R21); ou R1 et R2 forment ensemble avec l'atome d'azote auquel ils sont liés un cycle monocyclique, bicyclique ou spirocyclique de 4 à 10 chaînons, qui peut contenir, outre l'atome d'azote, 0 à 4 hétéroatomes supplémentaires, choisis dans le groupe formé par l'oxygène, l'azote et le soufre, le système hétérocyclique pouvant en outre être substitué par F, Cl, Br, CF₃, NO₂, CN, (C₁-C₆)-alkyle, O-(C₁-C₈)- alkyle, (C₁-C₄)-alcoxy-(C₁-C₄)-alkyle, hydroxy- (C₁- C₄)-alkyle, (C₀-C₈)-alkylène-aryle, oxo, CO(R22), CON(R23)(R24), hydroxy, COO(R25), N(R26)CO(C₁-C₆)- alkyle, N(R27)(R28) ou SO₂CH₃ ;
O vaut 0, 1, 2, 3, 4, 5, 6 ;
q, s valent, indépendamment l'un de l'autre 0, 1, 2, 3, 4 ;
R15, R16, R17, R18, R19, R20, R21, R22, R23, R24, R25, R26, R27, R28 signifient, indépendamment l'un de l'autre H, (C₁-C₆)-alkyle ;
R17 et R18, R23 et R24, R27 et R28 forment, indépendamment l'un de l'autre, éventuellement ensemble avec l'atome d'azote auquel ils sont liés, un cycle de 5-6 chaînons, qui peut encore contenir, outre l'atome d'azote, 0-1 autre hétéroatome du groupe NH, N-(C₁-C₆)-alkyle, oxygène et soufre ;
R12 signifie OH, O-(C₁-C₆)-alkyle, CN, COO(R29), CON(R30)(R31), N(R32)(R33), un cycle monocyclique, bicyclique ou spirocyclique de 3-12 chaînons, qui peut contenir un ou plusieurs hétéroatomes du groupe N, 0 et S et le cycle de 3-12 chaînons peut contenir d'autres substituants tels que F, Cl, Br, I, OH, CF₃, NO₂, CN, OCF₃, oxo, O-(C₁-C₆)-alkyle, O- (C₁-C₄)-alcoxy-(C₁-C₄)-alkyle, S-(C₁-C₆)-alkyle, (C₁- C₆)-alkyle, (C₂-C₆)-alcényle, (C₃-C₈)-cycloalkyle, (C₂-C₆)-alcynyle, O-(C₀-C₈)-alkylène-aryle, (C₀-C₈)- alkylène-aryle, N(R34)(R35), CO(C₁-C₆)-alkyle, COO(R36) et S(O)ᵤ(R37) ;
u vaut 0, 1, 2 ;
R34, R35 signifient, indépendamment l'un de l'autre H, (C₁-C₈)-alkyle ;
R34 et R35 forment éventuellement ensemble avec l'atome d'azote auquel ils sont liés, un cycle de 5-6 chaînons, qui peut encore contenir, outre l'atome d'azote, 0-1 autre hétéroatome du groupe NH, N-(C₁-C₆)-alkyle, oxygène et soufre et qui peut éventuellement être substitué par 1-2 groupes oxo ;
R36, R37 signifient H, (C₁-C₈)-alkyle ;
R13, R14 signifient, indépendamment l'un de l'autre H, (C₁-C₈)-alkyle, hydroxy-(C₁-C₄)alkyle, OH, (C₁-C₄)- alcoxy-(C₁-C₄)-alkyle ;
R29, R30, R31 signifient indépendamment l'un de l'autre H, (C₁-C₈)-alkyle, (C₂-C₆)-alcényle, (C₀- C₆)-alkylène-aryle ;
R32, R33 signifient, indépendamment l'un de l'autre H, (C₁-C₆)-alkyle ;
ou
R32 et R33 forment éventuellement ensemble avec l'atome d'azote auquel ils sont liés, un cycle de 5-6 chaînons, qui peut encore contenir, outre l'atome d'azote, 0-1 autre hétéroatome du groupe NH, N-(C₁-C₆)-alkyle, oxygène et soufre et qui peut éventuellement être substitué par 1-2 groupes oxo ;
R3 signifie H, (C₁-C₆)-alkyle ;
R4, R5 signifient, indépendamment l'un de l'autre, H, (C₁-C₆)-alkyle, OH, O-(C₁-C₆)-alkyle, O-CO(C₁-C₆)- alkyle, S-(C₁-C₆)-alkyle ;
R6, R7, R8, R9 signifient, indépendamment l'un de l'autre H, (C₁-C₈)-alkyle ;
ou
R6 et R7, R8 et R9 indépendamment l'un de l'autre, signifient éventuellement oxo ;
n, m indépendamment l'un de l'autre, valent 0, 1, 2 ;
A, B, D, G signifient, indépendamment l'un de l'autre, N, C(R38) et le nombre total des atomes d'azote dans ce cycle est de 0 ou 1, où, pour le cas où le nombre total d'atomes d'azote serait de 1, A ou B signifie N ;
R38 signifie H, F, Cl, Br, I, OH, CF₃, NO₂, CN, OCF₃, O-(C₁-C₆)-alkyle, O-(C₁-C₄)-alcoxy-(C₁-C₄)-alkyle, S-(C₁-C₆)-alkyle, (C₁-C₆)-alkyle, (C₂-C₆)-alcényle, (C₃-C₈)-cycloalkyle, O-(C₃-C₈)-cycloalkyle, (C₃-C₈)- cycloalcényle, O-(C₃-C₈)-cycloalcényle, (C₂-C₆)- alcynyle, (C₀-C₈)-alkylène-aryle, O-(C₀-C₈)- alkylène-aryle, S-aryle, N(R39)(R40), SO₂-CH₃, COOH, COO-(C₁-C₆)-alkyle, CON(R41)(R42), N(R43)CO(R44), N(R45)SO₂(R46), CO(R47), -(CR48R49)ₓ-O(R50) ;
R39, R40, R41, R42, R43, R45 signifient, indépendamment l'un de l'autre H, (C₁-C₈)-alkyle ;
ou
R39 et R40, R41 et R42 forment, indépendamment l'un de l'autre, éventuellement ensemble avec l'atome d'azote auquel ils sont liés, un cycle de 5-6 chaînons, qui peut encore contenir, outre l'atome d'azote, 0-1 autre hétéroatome du groupe NH, N-(C₁- C₆)-alkyle, oxygène et soufre ;
R44, R46, R47 signifient, indépendamment l'un de l'autre H, (C₁-C₈)-alkyle, aryle ;
R48, R49 signifient, indépendamment l'un de l'autre H, (C₁-C₈)-alkyle ;
R50 signifie H, (C₁-C₆)-alkyle ;
x vaut 1, 2, 3, 4 ;
Het est choisi dans le groupe constitué par les oxadiazoles, les thiadiazoles, les triazoles, les thiophènes, les furannes et les pyrroles ;
X signifie une liaison, un groupe de formule -(CR51R52)_{y}-, où un ou plusieurs groupes -(CR51R52)- peuvent être remplacés par Y, avec formation d'un radical qui a chimiquement un sens, C=C, C≡C ;
Y signifie O, S, N(R53), CO, SO, SO₂ ;
R51, R52 signifient, indépendamment l'un de l'autre H, (C₁-C₄)-alkyle, où R51 et R52 dans les y groupes peuvent à chaque fois présenter des significations identiques ou différentes ;
y vaut 1, 2, 3, 4, 5, 6 ;
R53 signifie H, (C₁-C₈)-alkyle ;
E signifie une structure carbocyclique ou hétérocyclique divalente de 3-14 chaînons comprenant 0-4 hétéroatomes du groupe N, O et S, qui peut éventuellement porter des substituants du groupe formé par H, F, Cl, Br, I, OH, CF₃, NO₂, CN, OCF₃, oxo, O-(C₁-C₆)-alkyle, O-(C₁-C₄)-alcoxy-(C₁- C₄)-alkyle, S- (C₁-C₆)-alkyle, (C₁-C₆)-alkyle, (C₂- C₆)-alcényle, (C₃-C₈)-cycloalkylé, O-(C₃-C₈)- cycloalkyle, (C₂-C₆)-alcynyle, (C₀-C₈)-alkylène- aryle, O-(C₀-C₈)-alkylène-aryle, S-aryle, N(R54)(R55), SO₂-CH₃, COOH, COO-(C₁-C₆)-alkyle, CON(R56)(R57), N(R58)CO(R59), N(R60)SO₂(R61), CO(R62) et qui peut être monocyclique ou bicyclique ;
R54, R55, R56, R57, R58, R60 signifient, indépendamment l'un de l'autre H, (C₁-C₈)-alkyle ;
R54 et R55, R56 et R57 forment, indépendamment l'un de l'autre, éventuellement ensemble avec l'atome d'azote auquel ils sont liés, un cycle de 5-6 chaînons, qui peut encore contenir, outre l'atome d'azote, 0-1 autre hétéroatome du groupe NH, N-(C₁- C₆)-alkyle, oxygène et soufre ;
R59, R61, R62 signifient, indépendamment l'un de l'autre H, (C₁-C₈)-alkyle, aryle ;
K signifie une liaison, un groupe de formule -(CR63R64)_{z}-, où un ou plusieurs groupes -(CR63R64)- peuvent être remplacés par Z, avec formation d'un radical qui a chimiquement un sens, C≡C, C=C ;
Z signifie 0, S, N(R65), CO, SO, SO₂ ;
R63, R64 signifient, indépendamment l'un de l'autre H, (C₁-C₈)-alkyle, hydroxy, (C₁-C₆)-alcoxy, où R63 et R64 dans les z groupes peuvent à chaque fois présenter des significations identiques ou différentes ;
z vaut 1, 2, 3, 4, 5, 6 ;
R65 signifie H, (C₁-C₈)-alkyle ;
R11 signifie H, (C₁-C₈)-alkyle, (C₁-C₄)-alcoxy-(C₁-C₄)- alkyle, (C₃-C₈)-alcényle, (C₃-C₈)-alcynyle, un cycle monocyclique, bicyclique ou spirocyclique de 3 à 10 chaînons, qui peut comporter 0 à 4 hétéroatomes, choisis dans le groupe formé par l'oxygène, l'azote et le soufre, le système cyclique pouvant en outre être substitué par F, Cl, Br, CF₃, NO₂, CN, (C₁-C₆)-alkyle, O-(C₁-C₈)- alkyle, (C₁-C₄)-alcoxy-(C₁-C₄)-alkyle, (C₀-C₈)- alkylène-aryle, oxo, CO(R66), CON(R67)(R68), hydroxy, hydroxy-(C₁-C₄)-alkyle, COO(R69), N(R70)CO(C₁-C₆)-alkyle, N(R71)(R72) ou SO₂CH₃ ;
R66, R67, R68, R69, R70, R71, R72 signifient, indépendamment l'un de l'autre H, (C₁-C₈)-alkyle ;
ou
R67 et R68, R71 et R72 forment, indépendamment l'un de l'autre, éventuellement ensemble avec l'atome d'azote auquel ils sont liés, un cycle de 5-6 chaînons, qui peut encore contenir, outre l'atome d'azote, 0-1 autre hétéroatome du groupe NH, N-(C₁- C₆)-alkyle, oxygène et soufre ; ou
leurs N-oxydes ainsi que leurs sels physiologiquement acceptables.

2. Composés de formule I selon la revendication 1,
dans laquelle :
R1, R2 signifient, indépendamment l'un de l'autre H, (C₁-C₈)-alkyle, -(CR13R14)ₒ-R12, (C₁-C₄)-alcoxy-(C₁- C₄)-alkyle, (C₃-C₈)-alcényle, (C₃-C₈)-alcynyle, CO- (C₁-C₈)-alkyle, -CO-(CH₂)ₒ-R12, CO(C(R15)(R16))_{q}N(R17)(R18), CO(C(R19)(R20))ₛO(R21); ou R1 et R2 forment ensemble avec l'atome d'azote auquel ils sont liés un cycle monocyclique, bicyclique ou spirocyclique de 4 à 10 chaînons, qui peut contenir, outre l'atome d'azote, 0 à 4 hétéroatomes supplémentaires, choisis dans le groupe formé par l'oxygène, l'azote et le soufre, le système hétérocyclique pouvant en outre être substitué par F, Cl, Br, CF₃, NO₂, CN, (C₁-C₆)-alkyle, O-(C₁-C₄)- alkyle, (C₁-C₄)-alcoxy-(C₁-C₄)-alkyle, hydroxy-(C₁- C₄)-alkyle, (C₀-C₂)-alkylène-aryle, oxo, CO(R22), CON(R23)(R24), hydroxy, COO(R25), N(R26)CO(C₁-C₆)- alkyle ou N(R27) (R28) ;
R12 signifie OH, O-(C₁-C₆)-alkyle, CN, COO(R29), CON(R30)(R31), N(R32)(R33), un cycle monocyclique, bicyclique ou spirocyclique de 3-12 chaînons, qui peut contenir un ou plusieurs hétéroatomes du groupe N, O et S et le cycle de 3-12 chaînons peut contenir d'autres substituants tels que F, Cl, Br, I, OH, CF₃, NO₂, CN, OCF₃, oxo, O-(C₁-C₆)-alkyle, (C₁-C₄)-alcoxy- (C₁-C₄)-alkyle, S- (C₁-C₆)-alkyle, (C₁- C₆)-alkyle, (C₂-C₆)-alcényle, (C₃-C₈)-cycloalkyle, (C₂-C₆)-alcynyle, O-(C₀-C₈)-alkylène-aryle, (C₀-C₈)- alkylène-aryle, N(R34)(R35), CO(C₁-C₆)-alkyle et COO(R36) ; et
R6, R7, R8, R9 signifient, indépendamment l'un de l'autre H, (C₁-C₈)-alkyle ;
où les autres radicaux et groupes dans les composés de formule I présentent les significations mentionnées dans la revendication 1.

3. Composés de formule I selon la revendication 1,
dans laquelle :
R1, R2 signifient indépendamment l'un de l'autre H, (C₁-C₈)-alkyle, -(CR13R14)ₒ-R12, (C₁-C₄)-alcoxy-(C₁- C₄)-alkyle, (C₃-C₈)-alcényle, CO-(C₁-C₈)-alkyle, -CO-(CH₂)ₒ-R12, CO(C(R15)(R16))_{q}N(R17)(R18), CO(C(R19)(R20))ₛO(R21) ; ou R1 et R2 forment ensemble avec l'atome d'azote auquel ils sont liés un cycle monocyclique, bicyclique ou spirocyclique de 4 à 10 chaînons, qui peut contenir, outre l'atome d'azote, 0 à 2 hétéroatomes supplémentaires, choisis dans le groupe formé par l'oxygène, l'azote et le soufre, le système cyclique hétérocyclique pouvant en outre être substitué par F, Cl, CF₃, (C₁-C₆)-alkyle, O-(C₁-C₄)- alkyle, (C₁-C₄)-alcoxy-(C₁-C₄)-alkyle, hydroxy-(C₁- C₄)-alkyle, (C₀-C₂)-alkylène-aryle, oxo, CO(R22), CON(R23)(R24), hydroxy, COO(R25), N(R26)CO(C₁-C₆)- alkyle, N(R27) (R28) ou SO₂CH₃ ;
o vaut 0, 1, 2, 3, 4, 5, 6 ;
q vaut 1, 2, 3 ;
s vaut 0, 1, 2, 3, 4 ;
R15, R16, R17, R18, R19, R20, R21, R22, R23, R24, R25, R26, R27, R28 signifient, indépendamment l'un de l'autre H, (C₁-C₆)-alkyle ;
ou
R17 et R18, R23 et R24, R27 et R28 forment, indépendamment l'un de l'autre, éventuellement ensemble avec l'atome d'azote auquel ils sont liés, un cycle de 5-6 chaînons, qui peut encore contenir, outre l'atome d'azote, 0-1 autre hétéroatome du groupe NH, N-(C₁-C₆)-alkyle, oxygène et soufre ;
R12 signifie OH, O-(C₁-C₆)-alkyle, CN, COO(R29), CON(R30)(R31), un cycle monocyclique, bicyclique ou spirocyclique de 3-12 chaînons, qui peut contenir un ou plusieurs hétéroatomes du groupe N, O et S et le cycle de 3-12 chaînons peut contenir d'autres substituants tels que F, Cl, Br, OH, CF₃, CN, oxo, O-(C₁-C₆)-alkyle, (C₁-C₄)-alcoxy-(C₁-C₄)- alkyle, (C₁-C₆)-alkyle, O-(C₀-C₈)-alkylène-aryle, (C₀-C₈)-alkylène-aryle, N(R34)(R35), CO(C₁-C₆)- alkyle, COO(R36), S(O)ᵤ(R37) ;
u vaut 0, 1, 2 ;
R34, R35 signifient, indépendamment l'un de l'autre H, (C₁-C₈)-alkyle ;
ou
R34 et R35 forment éventuellement ensemble avec l'atome d'azote auquel ils sont liés, un cycle de 5-6 chaînons, qui peut encore contenir, outre l'atome d'azote, 0-1 autre hétéroatome du groupe NH, N-(C₁-C₆)-alkyle, oxygène et soufre et qui peut éventuellement être substitué par 1-2 groupes oxo ;
R36, R37 signifient H, (C₁-C₈)-alkyle ;
R13, R14 signifient, indépendamment l'un de l'autre H, (C₁-C₈)-alkyle, hydroxy-(C₁-C₄)alkyle, OH, (C₁- C₄)-alcoxy-(C₁-C₄)-alkyle ;
R29, R30, R31 signifient, indépendamment l'un de l'autre H, (C₁-C₈)-alkyle ;
R3 signifie H, (C₁-C₆)-alkyle ;
R4, R5 signifient, indépendamment l'un de l'autre, H, (C₁-C₆)-alkyle, OH, O-(C₁-C₆)-alkyle, O-CO(C₁-C₆)- alkyle, S-(C₁-C₆)-alkyle ;
R6, R7, R8, R9 signifient H ;
ou
R6 et R7, R8 et R9 indépendamment l'un de l'autre, signifient éventuellement oxo ;
n vaut 1,
m vaut 1 ou 2 ;
A, B, D, G signifient, indépendamment l'un de l'autre, N, C(R38) et le nombre total des atomes d'azote dans ce cycle est de 0 ou 1, où, pour le cas où le nombre total d'atomes d'azote serait de 1, A ou B signifie N ; ou les groupes A et B ou D et G représentent à chaque fois C(R38) et forment ensemble une unité ortho- phénylène, de manière telle qu'on obtient au total un système naphtalène 1,4-disubstitué ;
R38 signifie H, F, Cl, Br, CF₃, CN, O-(C₁-C₆)-alkyle, O-(C₁-C₄)-alcoxy-(C₁-C₄)-alkyle, S-(C₁-C₆)-alkyle, (C₁-C₆)-alkyle, (C₀-C₈)-alkylène-aryle, O-(C₀-C₈)- alkylène-aryle, N(R39)(R40), SO₂-CH₃, CON(R41)(R42), N(R43)CO(R44), CO(R47), -(CR48R49)ₓ- O(R50) ;
R39, R40, R41, R42, R43 signifient, indépendamment l'un de l'autre H, (C₁-C₈)-alkyle ;
ou
R39 et R40, R41 et R42 forment, indépendamment l'un de l'autre, éventuellement ensemble avec l'atome d'azote auquel ils sont liés, un cycle de 5-6 chaînons, qui peut encore contenir, outre l'atome d'azote, 0-1 autre hétéroatome du groupe NH, N-(C₁- C₆)-alkyle, oxygène et soufre ;
R44, R47 signifient, indépendamment l'un de l'autre H, (C₁-C₈)-alkyle, aryle ;
R48, R49 signifient H ;
R50 signifie H, (C₁-C₆)-alkyle ;
x vaut 1, 2 ;
Het est choisi dans le groupe constitué par les oxadiazoles, les thiadiazoles, les triazoles, les thiophènes, les furannes et les pyrroles ;
X signifie une liaison, un groupe de formule -(CR51R52)_{y}-, où un ou plusieurs groupes -(CR51R52)- peuvent être remplacés par Y, avec formation d'un radical qui a chimiquement un sens, C=C, C=C ;
R51, R52 signifient, indépendamment l'un de l'autre H, (C₁-C₄)-alkyle, où R51 et R52 dans les y groupes peuvent à chaque fois présenter des significations identiques ou différentes ;
Y signifie 0, S, N(R53), CO ;
R53 signifie H, (C₁-C₈)-alkyle ;
E signifie une structure carbocyclique ou hétérocyclique divalente de 3-8 chaînons comprenant 0-4 hétéroatomes du groupe N, 0 et S, qui peut éventuellement porter des substituants du groupe formé par H, F, Cl, Br, OH, CF₃, NO₂, CN, OCF₃, O-(C₁-C₆)-alkyle, O-(C₁-C₄)-alcoxy-(C₁-C₄)- alkyle, S-(C₁-C₆)-alkyle, (C₁-C₆)-alkyle, (C₂-C₆)- alcényle, O-(C₃-C₈)-cycloalkyle, (C₂-C₆)-alcynyle, (C₀-C₈)-alkylène-aryle, O-(C₀-C₈)-alkylène-aryle, S- aryle, N(R54)(R55), SO₂-CH₃, N(R58)CO(R59), N(R60)SO₂(R61), CO(R62) et qui peut être monocyclique ou bicyclique ;
R54, R55, R58, R60 signifient, indépendamment l'un de l'autre H, (C₁-C₈)-alkyle ;
R59, R61, R62 signifient, indépendamment l'un de l'autre H, (C₁-C₈)-alkyle, aryle ;
K signifie 0, une liaison, CH₂O, OCH₂, S, SO, SO₂, N(R80), N(R81)CO, CON(R82), (C(R83)(R84))ᵥ, CO, C=C, C≡C, SCH₂, SO₂CH₂ ;
v vaut 1, 2, 3, 4 ;
R80, R81, R82, R83, R84 signifient, indépendamment l'un de l'autre H, (C₁-C₈)-alkyle ;
R11 signifie H, (C₁-C₈)-alkyle, (C₁-C₄)-alcoxy-(C₁-C₄)- alkyle, (C₃-C₈)-alcényle, (C₃-C₈)-alcynyle, un cycle monocyclique, bicyclique ou spirocyclique de 3 à 10 chaînons, qui peut comporter 0 à 4 hétéroatomes, choisis dans le groupe formé par l'oxygène, l'azote et le soufre, le système cyclique pouvant en outre être substitué par F, Cl, Br, CF₃, CN, (C₁-C₆)-alkyle, O-(C₁-C₈)-alkyle, (C₁-C₄)-alcoxy-(C₁-C₄)-alkyle, hydroxy-(C₁-C₄)- alkyle, (C₀-C₈)-alkylène-aryle, oxo, CO(R66), CON(R67)(R68), hydroxy, COO(R69), N(R70)CO(C₁-C₆)- alkyle, N(R71)(R72) ou SO₂CH₃ ;
R66, R67, R68, R69, R70, R71, R72 signifient, indépendamment l'un de l'autre H, (C₁-C₈)-alkyle ;
ou
R67 et R68, R71 et R72 forment, indépendamment l'un de l'autre, éventuellement ensemble avec l'atome d'azote auquel ils sont liés, un cycle de 5-6 chaînons, qui peut encore contenir, outre l'atome d'azote, 0-1 autre hétéroatome du groupe NH, N-(C₁- C₆)-alkyle, oxygène et soufre ; ou
leurs N-oxydes ainsi que leurs sels physiologiquement acceptables.

4. Composés de formule I selon la revendication 1 ou 3, où
R1, R2 signifient, indépendamment l'un de l'autre, H, (C₁-C₈)-alkyle, -(CR13R14)ₒ-R12, (C₁-C₄)-alcoxy-(C₁- C₄)-alkyle, CO-(C₁-C₈)-alkyle, -CO-(CH₂)ₒ-R12, CO(C(R15)(R16))_{q}N(R17)(R18), CO(C(R19)(R20))ₛO(R21) ; ou R1 et R2 forment ensemble avec l'atome d'azote auquel ils sont liés un cycle monocyclique ou bicyclique de 4 à 10 chaînons, qui peut contenir, outre l'atome d'azote, 0 à 2 hétéroatomes supplémentaires, choisis dans le groupe formé par l'oxygène, l'azote et le soufre, le système hétérocyclique pouvant en outre être substitué par F, Cl, CF₃, (C₁-C₆)-alkyle, O-(C₁-C₄)-alkyle, (C₁-C₄)-alcoxy-(C₁- C₄)-alkyle, (C₀-C₂)-alkylène-aryle, oxo, CO(R22), hydroxy, N(R27)(R28) ou SO₂CH₃ ; de préférence, indépendamment l'un de l'autre, H, (C₁-C₈)-alkyle, -(CR13R14)ₒ-R12, (C₁-C₄)-alcoxy-(C₁- C₄)-alkyle, CO-(C₁-C₈)-alkyle, -CO-(CH₂)ₒ-R12, CO(C(R15)(R16))_{q}N(R17)(R18), ou R1 et R2 forment ensemble avec l'atome d'azote auquel ils sont liés un cycle monocyclique ou bicyclique de 4 à 10 chaînons, qui peut contenir, outre l'atome d'azote, 0 à 2 hétéroatomes supplémentaires, choisis dans le groupe formé par l'oxygène et l'azote, le système hétérocyclique pouvant en outre être substitué par F, (C₁-C₆)-alkyle, (C₁-C₄)- alcoxy-(C₁-C₄)-alkyle, oxo, CO(R22), hydroxy, N(R27) (R28) ;
o vaut 0, 1, 2, 3, 4 ; de préférence 0, 1, 2, 3 ;
q vaut 1 ou 2 ;
s vaut 0, 1, 2, 3 ; de préférence 0, 1, 2 ;
R15, R16, R17, R18, R19, R20, R21, R22, R27, R28 signifient, indépendamment l'un de l'autre H, (C₁-C₆)-alkyle ;
ou
R17 et R18, R27 et R28 signifient, indépendamment l'un de l'autre, éventuellement ensemble avec l'atome d'azote auquel ils sont liés, un cycle de 5-6 chaînons choisi parmi la pyrrolidine, la pipéridine, la N-méthylpipérazine et la morpholine ;
R12 signifie OH, O-(C₁-C₆)-alkyle, CN, un cycle monocyclique ou bicyclique de 3-10 chaînons, qui peut contenir 1-3 hétéroatomes du groupe N, O et S et le cycle de 3-10 chaînons peut contenir d'autres substituants tels que F, Cl, Br, OH, CF₃, CN, oxo, O-(C₁-C₆)-alkyle, (C₁-C₄)-alcoxy-(C₁-C₄)- alkyle, (C₁-C₆)-alkyle, (C₀-C₂)-alkylène-aryle, N(R34) (R35), CO(C₁-C₆)-alkyle ; de préférence OH, O-(C₁-C₆)-alkyle, un cycle monocyclique ou bicyclique de 3-10 chaînons, qui peut contenir 1-2 hétéroatomes du groupe N, O et S et le cycle de 3-10 chaînons peut contenir d'autres substituants tels que F, OH, oxo, (C₁-C₆)- alkyle, CO(C₁-C₆)-alkyle ;
u vaut 0 ou 2 ; de préférence 2 ;
R34, R35 signifient, indépendamment l'un de l'autre H, (C₁-C₈)-alkyle ;
ou
R34 et R35 forment éventuellement ensemble avec l'atome d'azote auquel ils sont liés, un cycle de 5-6 chaînons, qui peut encore contenir, outre l'atome d'azote, 0-1 autre hétéroatome du groupe NH, N-(C₁-C₆)-alkyle, oxygène et soufre et qui peut éventuellement être substitué par 1-2 groupes oxo ;
R36, R37 signifient H, (C₁-C₈)-alkyle ;
R13, R14 signifient, indépendamment l'un de l'autre H, (C₁-C₈)-alkyle, hydroxy-(C₁-C₄)alkyle, OH, (C₁- C₄)-alcoxy-(C₁-C₄)-alkyle ;
R3 signifie H ;
R4, R5 signifient, indépendamment l'un de l'autre H, (C₁-C₆)-alkyle, OH, O-(C₁-C₆)-alkyle, O-CO(C₁-C₆)- alkyle ; de préférence indépendamment l'un de l'autre H, OH, O-(C₁-C₆)-alkyle ; de manière tout particulièrement préférée H ;
R6, R7, R8, R9 signifient H ;
n vaut 1 ;
m vaut 1 ;
A, B, D, G signifient C(R38) ;
R38 signifie H, F, Cl, Br, CF₃, CN, O-(C₁-C₆)-alkyle, (C₁-C₆)-alkyle, SO₂-CH₃, CON(R41)(R42), N(R43)CO(R44), CO(R47), -(CR48R49)ₓ-O(R50) ; de manière particulièrement préférée H, F, Cl, CF₃, CN, (C₁-C₆)-alkyle, -(CR48R49)ₓ-O(R50) ;
R41, R42, R43 signifient, indépendamment l'un de l'autre H, (C₁-C₈)-alkyle ;
ou
R39 et R40, R41 et R42 forment, indépendamment l'un de l'autre, éventuellement ensemble avec l'atome d'azote auquel ils sont liés, un cycle de 5-6 chaînons, qui peut encore contenir, outre l'atome d'azote, 0-1 autre hétéroatome du groupe NH, N-(C₁- C₆)-alkyle, oxygène et soufre ;
R47 signifie H, (C₁-C₈)-alkyle ;
R48, R49 signifient H ;
R50 signifie H, (C₁-C₆)-alkyle ;
x vaut 1 ;
Het signifie un groupe hétéroaromatique, choisi dans le groupe constitué par les oxadiazoles, les thiadiazoles, les triazoles, les thiophènes, les furannes et les pyrroles ; de manière tout particulièrement préférée, Het est choisi parmi les oxadiazoles et les thiadiazoles ;
X signifie une liaison, CH₂-CH₂, CH₂-Y, YCH₂, (R75)YCH₂, CH₂-NCO(R75), CH₂CON(R75), C(R76)(R77), C(R78)(R79)O, N(R75), C=C, C≡C ; de préférence une liaison, CH₂-CH₂, C(R76)(R77), N(R75), CH₂Y, CH₂Y(R75), CH₂-NCO(R75), CH₂CON(R75), C=C ; de manière particulièrement préférée une liaison, CH₂- CH₂, C(R76)(R77), C=C, (R75)YCH₂, CH₂-NCO(R75) ;
Y signifie O, S, N(R53) ;
R53 signifie H, (C₁-C₈)-alkyle ;
R75, R76, R77, R78, R79 signifient, indépendamment l'un de l'autre H, (C₁-C₈)-alkyle ;
E signifie une structure carbocyclique ou hétérocyclique divalente de 5-7 chaînons comprenant 0-3 hétéroatomes du groupe N, O et S, qui peut éventuellement porter des substituants du groupe formé par H, F, Cl, Br, OH, CF₃, NO₂, CN, OCF₃, O-(C₁-C₆)-alkyle, S-(C₁-C₆)-alkyle, (C₁-C₆)- alkyle, (C₂-C₆)-alcényle, O-(C₀-C₈)-alkylène-aryle, S-aryle, N(R54)(R55), SO₂-CH₃, N(R58)CO(R59), CO(R62) et qui peut être monocyclique ou bicyclique ; de préférence une structure carbocyclique ou hétérocyclique divalente de 5-7 chaînons comprenant 0-2 hétéroatomes du groupe formé par N, O et S, qui peut éventuellement porter des substituants du groupe formé par H, F, Cl, Br, OH, CF₃, NO₂, OCF₃, O-(C₁-C₆)-alkyle, (C₁-C₆)-alkyle, (C₂-C₆)-alcényle, N(R54)(R55), SO₂-CH₃, CO(R62), et qui est de manière particulièrement préférée monocyclique ; par exemple E signifie benzène, pyridine, pyrimidine, pipéridine, pyrrolidine, cyclopentane, cyclohexane, pipérazine, homopipérazine, thiazole, thiophène, furanne, pyrrole, pyrazole, 1,2,3,6- tétrahydropyridine, 4,5-dihydro-isoxazole, oxazole ;
R54, R55, R58 signifient, indépendamment l'un de l'autre H, (C₁-C₈)-alkyle ;
R59, R62 signifient, indépendamment l'un de l'autre H, (C₁-C₈)-alkyle ;
K signifie O, une liaison, CH₂O, OCH₂, N(R80), N(R81)CO, CON(R82), (C(R83)(R84))ᵥ, CO, C≡C, SCH₂ ; de préférence O, une liaison, CH₂O, OCH₂, CON(R82), (C(R83)(R84))ᵥ, CO, C=C ;
v vaut 1, 2, 3 ; de préférence 1, 2 ;
R80, R81, R83, R84 signifient, indépendamment l'un de l'autre H, (C₁-C₈)-alkyle ;
R11 signifie (C₁-C₈)-alkyle, (C₁-C₄)-alcoxy-(C₁-C₄)- alkyle, un cycle monocyclique, bicyclique ou spirocyclique de 3 à 10 chaînons, qui peut comporter 0 à 3 hétéroatomes, choisis dans le groupe formé par l'oxygène, l'azote et le soufre, le système cyclique pouvant en outre être substitué par F, Cl, Br, CF₃, CN, (C₁-C₆)-alkyle, 0- (C₁-C₈)-alkyle, (C₀-C₂)-alkylène-aryle, oxo, CO(R66), CON(R67) (R68), hydroxy, N(R70)CO(C₁-C₆)- alkyle, N(R71) (R72) ou SO₂CH₃ ; de préférence (C₁-C₈)-alkyle, (C₁-C₄)-alcoxy-(C₁- C₄)-alkyle, un cycle monocyclique ou bicyclique de 3 à 10 chaînons, qui peut comporter 0 à 2 hétéroatomes, choisis dans le groupe formé par l'oxygène, l'azote et le soufre, le système cyclique pouvant en outre être substitué par F, Cl, Br, CF₃, CN, (C₁-C₆)-alkyle, O-(C₁-C₈)-alkyle, oxo, CO(R66), CON(R67) (R68), N(R70)CO(C₁-C₆)-alkyle ou SO₂CH₃ ;
R66, R67, R68, R70, R71, R72 signifient, indépendamment l'un de l'autre H, (C₁-C₈)-alkyle ;
ou
R67 et R68, R71 et R72 forment, indépendamment l'un de l'autre, éventuellement ensemble avec l'atome d'azote auquel ils sont liés, un cycle de 5-6 chaînons, qui peut encore contenir, outre l'atome d'azote, 0-1 autre hétéroatome du groupe NH, N-(C₁- C₆)-alkyle, oxygène et soufre ; ou
leurs N-oxydes ainsi que leurs sels physiologiquement acceptables.

5. Composés de formule I selon l'une quelconque des revendications 1 ou 2 à 4 où les radicaux R1, R2, R11, R38 et les groupes X, E et K présentent les significations suivantes :
R1, R2 signifient indépendamment l'un de l'autre H, (C₁-C₈)-alkyle, -(CR13R14)ₒ-R12, (C1-C4)-alcoxy-(C₁- C₄)-alkyle, ou R1 et R2 forment ensemble avec l'atome d'azote auquel ils sont liés un cycle monocyclique, bicyclique ou spirocyclique de 4 à 10 chaînons, qui peut contenir, outre l'atome d'azote, 0 à 2 hétéroatomes supplémentaires, choisis dans le groupe formé par l'oxygène, l'azote et le soufre, le système cyclique hétérocyclique pouvant en outre être substitué par (C₁-C₆)-alkyle, O-(C₁-C₄)-alkyle, (C₁-C₄)-alcoxy-(C₁- C₄)-alkyle, hydroxy-(C₁-C₄)-alkyle, (C₀-C₂)- alkylène-aryle, oxo, CO(R22), CON(R23)(R24), hydroxy, N(R27)(R28) ou SO₂CH₃ ; de préférence, indépendamment l'un de l'autre H, (C₁-C₈)-alkyle, -(CR13R14)ₒ-R12, (C₁-C₄)-alcoxy- (C₁- C₄)-alkyle, ou R1 et R2 forment ensemble avec l'atome d'azote auquel ils sont liés un cycle monocyclique, bicyclique ou spirocyclique de 4 à 10 chaînons, qui peut contenir, outre l'atome d'azote, 0 à 2 hétéroatomes supplémentaires, choisis dans le groupe formé par l'oxygène, l'azote et le soufre, le système hétérocyclique pouvant en outre être substitué par (C₁-C₆)-alkyle, O-(C₁-C₄)-alkyle, (C₁-C₄)-alcoxy-(C₁-C₄)-alkyle, hydroxy-(C₁-C₄)-alkyle, (C₀-C₂)-alkylène-aryle, oxo, CO(R22), CON(R23) (R24), hydroxy ou N(R27)(R28) ; de manière tout particulièrement préférée, indépendamment l'un de l'autre, H, (C₁-C₈)-alkyle, ou R1 et R2 forment ensemble avec l'atome d'azote auquel ils sont liés, un cycle monocyclique de 5 à 6 chaînons, qui peut contenir outre l'atome d'azote 0 à 1 hétéroatome supplémentaire, choisi dans le groupe formé par l'oxygène, l'azote et le soufre, le système hétérocyclique pouvant en outre être substitué par (C₁-C₆)-alkyle ;
o vaut 0, 1, 2, 3, 4 ;
R22, R23, R24, R27, R28 signifient, indépendamment l'un de l'autre H, (C₁-C₆)-alkyle ;
ou
R23 et R24, R27 et R28 forment, indépendamment l'un de l'autre, éventuellement ensemble avec l'atome d'azote auquel ils sont liés, un cycle de 5-6 chaînons, qui peut encore contenir, outre l'atome d'azote, 0-1 autre hétéroatome du groupe NH, N-(C₁- C₆)-alkyle, oxygène et soufre ;
R12 signifie OH, O-(C₁-C₆)-alkyle, CN, un cycle monocyclique, bicyclique ou spirocyclique de 3-12 chaînons, qui peut contenir 1-3 hétéroatomes du groupe N, O et S et le cycle de 3-12 chaînons peut contenir d'autres substituants tels que F, OH, CF₃, CN, oxo, (C₁-C₆)-alkyle, (C₀-C₂)-alkylène-aryle, N(R34) (R35), COO(R36), CO(C₁-C₆)-alkyle ;
R34, R35 signifient, indépendamment l'un de l'autre H, (C₁-C₄)-alkyle ;
R36 signifie H, (C₁-C₆)-alkyle, (C₀-C₂)-alkylène-aryle ;
R13, R14 signifient, indépendamment l'un de l'autre H, (C₁-C₈)-alkyle, hydroxy-(C₁-C₄) alkyle, OH, (C₁- C₄)-alcoxy-(C₁-C₄)-alkyle ;
R38 signifie H, F, C1, Br, CF₃, CN, (C₁-C₆)-alkyle ;
X signifie une liaison, CH₂CH₂, C(R76)(R77), N(R75), C=C, (R75)YCH₂, CH₂NCO(R75), CH₂CON(R75) ;
Y signifie O, S, N(R53), CO ;
R75, R76, R77 signifient, indépendamment l'un de l'autre H, (C₁-C₈)-alkyle ;
R53 signifie H, (C₁-C₈)-alkyle ;
E signifie une structure carbocyclique ou hétérocyclique divalente de 5-7 chaînons comprenant 0-3 hétéroatomes du groupe N, O et S, qui peut éventuellement porter des substituants du groupe formé par H, F, Cl, Br, CF₃, OH, CN, OCF₃, O-(C₁-C₆)-alkyle, (C₁-C₆)-alkyle, SO₂-CH₃, CO(R65) ;
R65 signifie H, (C₁-C₈)-alkyle ;
K signifie 0, une liaison, CH₂O, CH₂, OCH₂, S, SO₂, N(R80), N(R81)CO, CON(R82), (C(R83)(R84))ᵥ, CO, C=C, SCH₂, SO₂CH₂ ; de préférence O, une liaison, CH₂O, CH₂, OCH , N(R80), C=C ;
v vaut 1, 2, 3 ;
R80, R81, R82, R83, R84 signifient, indépendamment l'un de l'autre H, (C₁-C₈)-alkyle ;
R11 signifie (C₁-C₈)-alkyle, (C₁-C₄)-alcoxy-(C₁-C₄)- alkyle, un cycle monocyclique, bicyclique ou spirocyclique de 3 à 10 chaînons, qui peut comporter 0 à 4 hétéroatomes, choisis dans le groupe formé par l'oxygène, l'azote et le soufre, le système cyclique pouvant en outre être substitué par F, Cl, Br, CF₃, CN, (C₁-C₆)-alkyle, O-(C₁-C₈)-alkyle, oxo, CO(R66), hydroxy, N(R70)CO(C₁-C₆)-alkyle ou SO₂CH₃ ;
R66, R70 signifient, indépendamment l'un de l'autre H, (C₁-C₈)-alkyle ;
leurs N-oxydes ainsi que leurs sels physiologiquement acceptables.

6. Composés de formule I selon l'une quelconque des revendications 1 à 5, où
A, B, D, G signifient, indépendamment l'un de l'autre, N ou C(R38) et le nombre total des atomes d'azote dans ce cycle est de 0 ou 1, où, pour le cas où le nombre total d'atomes d'azote serait de 1, B signifie N.

7. Composés de formule I selon l'une quelconque des revendications 1 à 6, où
n vaut 1 et
m vaut 1 ou 2.

8. Médicament contenant un ou plusieurs des composés selon l'une quelconque des revendications 1 à 7.

9. Médicament contenant un ou plusieurs des composés selon l'une quelconque des revendications 1 à 7 et une ou plusieurs substances actives anorexigènes.

10. Composés de formule I selon l'une quelconque des revendications 1 à 7 pour une utilisation comme médicament destiné à la prophylaxie ou au traitement de l'obésité.

11. Composés de formule I selon l'une quelconque des revendications 1 à 7 pour une utilisation comme médicament destiné à la prophylaxie ou au traitement du diabète de type II.

12. Composés de formule I selon l'une quelconque des revendications 1 à 7 en combinaison avec au moins une autre substance active anorexigène pour une utilisation comme médicament destiné à la prophylaxie ou au traitement de l'obésité.

13. Composés de formule I selon l'une quelconque des revendications 1 à 7 en combinaison avec au moins une autre substance active anorexigène pour une utilisation comme médicament destiné à la prophylaxie ou au traitement du diabète de type II.

14. Procédé pour la préparation d'un médicament contenant un ou plusieurs des composés de formule I selon l'une quelconque des revendications 1 à 7, **caractérisé**
**en ce que** la substance active est mélangée avec un support pharmaceutiquement approprié et ce mélange est amené dans une forme appropriée pour l'administration.

15. Utilisation des composés de formule I selon l'une quelconque des revendications 1 à 7 pour la préparation d'un médicament destiné à la réduction pondérale chez les mammifères.

16. Utilisation des composés de formule I selon l'une quelconque des revendications 1 à 7 pour la préparation d'un médicament destiné à la prophylaxie ou au traitement de l'obésité.

17. Utilisation des composés de formule I selon l'une quelconque des revendications 1 à 7 pour la préparation d'un médicament destiné à la prophylaxie ou au traitement du diabète de type II.
